(19) Europäisches Patentamt
European Patent Office
Office européen des brevets

(11) Veröffentlichungsnummer: **0 188 400 B1**

(12) **EUROPÄISCHE PATENTSCHRIFT**

(45) Veröffentlichungstag der Patentschrift: **08.07.92**

(51) Int. Cl.[5]: **C07K 7/00**, A61K 37/02, C12N 15/00, C12P 21/02

(21) Anmeldenummer: **86810007.4**

(22) Anmeldetag: **10.01.86**

Die Akte enthält technische Angaben, die nach dem Eingang der Anmeldung eingereicht wurden und die nicht in dieser Patentschrift enthalten sind.

# (54) Oligopeptide sowie Zwischenprodukte und Verfahren zu ihrer Herstellung.

(30) Priorität: **16.01.85 CH 188/85**

(43) Veröffentlichungstag der Anmeldung:
**23.07.86 Patentblatt 86/30**

(45) Bekanntmachung des Hinweises auf die Patenterteilung:
**08.07.92 Patentblatt 92/28**

(84) Benannte Vertragsstaaten:
**AT BE CH DE FR GB IT LI LU NL SE**

(56) Entgegenhaltungen:

**NEUROPEPTIDES, Band 4, 1984, Seiten 425-434; J.R. TIPPINS et al.: "The myotropic and plasma-calcium modulating effects of calcitonin gene-related peptide (CGRP)"**

**NATURE, Band 313, 3. Januar 1985, Seiten 54-56; S.D. BRAIN et al.: "Calcitonin gene-related peptide is a potent vasodilator"**

**SCIENCE, Band 229, 13. September 1985, Seiten 1094-1097; S.G. AMARA et al.: "Expression in brain of a messenger RNA encoding a novel neuropeptide homologous to calcitonin gene-related peptide"**

**Human Genet. (1985) 70: 259-263**

**Circulation Research 1987; 60: 393-397**

(73) Patentinhaber: **CIBA-GEIGY AG**
**Klybeckstrasse 141**
**CH-4002 Basel(CH)**

(72) Erfinder: **Jansz, Hendrik Simons, Prof. Dr.**
**Kersbergenlaan 37**
**NL-3703 AM Zeist(NL)**
Erfinder: **Lips, Cornelis Josephus Maria, Dr.**
**Wassenaarseweg 109**
**NL-2596 CN 's-Gravenhage(NL)**
Erfinder: **Steenbergh, Paul Herman, Dr.**
**Kon. Wilhelminastraat 4**
**NL-3981 VG Bunnik(NL)**
Erfinder: **Rink, Hans**
**Rebenstrasse 10**
**CH-4125 Riehen(CH)**
Erfinder: **Sieber, Peter**
**Bachmattweg 10**
**CH-4153 Reinach(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 188 400 B1

## Beschreibung

Die Erfindung betrifft neue Peptide, deren Struktur sich von einem bisher unbekannten zweiten humanen Calcitonin-Gen ableitet, d.h. sogenannte "Calcitonin gene related peptides Typ II (CGRP II)", deren Salze, entsprechende DNA-Sequenzen und Mikroorganismen, die diese DNA-Sequenzen enthalten und die zur Produktion der erfindungsgemässen Peptide fähig sind, als Zwischenprodukte zur Herstellung dieser Peptide, Verfahren zur Herstellung der Peptide oder DNA-Sequenzen, pharmazeutische Präparate, die die genannten Peptide oder Salze davon enthalten, sowie die Verwendung dieser Peptide oder ihrer Salze als Arzneimittel.

Aus S.D. Brain et al., Nature 313 (1985) 54-56 sind die Formeln von menschlichem "Calcitonin gene related peptide" Typ I (CGRP I) und von Ratten-CGRP bekannt. Das erfindungsgemässe humane CGRP II unterscheidet sich vom humanen CGRP I hinsichtlich der Aminosäuren in den Positionen 3, 22 und 25 und vom Ratten-CGRP hinsichtlich der Aminosäuren in den Positionen 1, 22, 25 und 35.

Die Erfindung betrifft erstens insbesondere Peptide, die als Teilsequenz eines grösseren Peptids oder ausschliesslich die Aminosäurensequenz der Formel I

-AlaCysAsnThrAlaThrCysValThrHisArgLeuAlaGlyLeuLeuSer-
ArgSerGlyGlyMetValLysSerAsnPheValProThrAsnValGlySer-
LysAlaPhe- (I)

aufweisen, wobei die Cysteinreste intra- oder intermolekulare Disulfidbrücken ausbilden können, und Derivate davon mit amidierter terminaler Carboxylgruppe und/oder acylierter terminaler Aminogruppe und ihre Salze.

Das obengenannte grössere Peptid weist insbesondere bis zu 90, hauptsächlich bis zu 80, z.B. bis zu 72, Aminosäurereste auf. Vorzugsweise hat es die Aminosäurensequenz der Formel II

AspTyrValGlnMetLysAlaSerGluLeuLysGlnGluGlnGluThrGlnSer-
SerSerSerAlaAlaGlnLysArgAlaCysAsnThrAlaThrCysValThrHisArg-
LeuAlaGlyLeuLeuSerArgSerGlyGlyMetValLysSerAsnPheValProThr-
AsnValGlySerLysAlaPheGlyArgArgArgSerAspLeuGluAla- (II)

oder ein Fragment derselben, das neben mindestens einem zusätzlichen Aminosäurerest die Sequenz der Formel I aufweist.

Bevorzugterweise bilden die beiden Cysteinreste eine intramolekulare Disulfidbrücke zueinander aus. Es können aber z.B. auch Dimere gebildet werden, wobei die beides, Peptidketten Kopf-Kopf oder, vorzugsweise, Kopf-Schwanz, d.h. antiparallel, via intermolekulare Disulfidbrücken miteinander verknüpft sein können.

Die terminale Aminogruppe ist vorzugsweise frei, kann aber auch acyliert, wie insbesondere acetyliert sein.

Pharmakologisch wirksam sind diejenigen der obengenannten Peptide, worin die terminale Carboxylgruppe in amidierter Form, d.h. insbesondere als Carbamoylgruppe, vorliegt. Die obengenannten Peptide, worin die terminale Carboxylgruppe in freier Form oder in Salzform vorliegt, gehören als Zwischenprodukte zur Herstellung der pharmakologisch wirksamen Amide ebenfalls zum Gegenstand der Erfindung.

In allererster Linie betrifft die Erfindung das Peptidamid der Formel III

S———————S
Ala-Cy-Asn-Thr-Ala-Thr-Cy-Val-Thr-His-Arg-Leu-Ala-Gly-Leu-
Leu-Ser-Arg-Ser-Gly-Gly-Met-Val-Lys-Ser-As-Phe-Val-Pro-
Thr-Asn-Val-Gly-Ser-Lys-Ala-Phe-$NH_2$ (III)

(CGRP II) und dessen Salze.

Daneben betrifft die Erfindung auch das N-acetylierte Peptidamid der Formel IIIa

```
   O     S———————————————————S
   ‖     |                   |
CH3-C-Ala-Cy-Asn-Thr-Ala-Thr-Cy-Val-Thr-His-Arg-Leu-Ala-
Gly-Leu-Leu-Ser-Arg-Ser-Gly-Gly-Met-Val-Lys-Ser-Asn-Phe-      (IIIa)
Val-Pro-Thr-Asn-Val-Gly-Ser-Lys-Ala-Phe-NH2.
```

Die Erfindung betrifft auch Peptide der Formel IIIb,

```
    S———————————————————S
    |                   |
Ala-Cy-Asn-Thr-Ala-Thr-Cy-Val-Thr-His-Arg-Leu-Ala-Gly-Leu-
Leu-Ser-Arg-Ser-Gly-Gly-Met-Val-Lys-Ser-Asn-Phe-Val-Pro-      (IIIb)
                                         Ω
Thr-Asn-Val-Gly-Ser-Lys-Ala-Phe-AS -OH
```

worin $AS^{\Omega}$ für eine Aminosäure, wie vorzugsweise Glycin oder Tyrosin, steht, die enzymatisch zur $NH_2$-Gruppe der terminalen Amidgruppe des Peptidamids der Formel III abgebaut beziehungsweise durch Ammoniak in Gegenwart eines geeigneten Enzyms substituiert werden kann, sowie Derivate davon, worin die terminale Aminogruppe acetyliert ist.

In Uebereinstimmung mit den international anerkannten Nomenklaturregeln bezeichnen in dieser Anmeldung die Abkürzungen für die Aminosäuren, z.B. die obengenannten Abkürzungen, die freie Säure und, wenn nicht anders angegeben, die L-Konfiguration. Die $\alpha$-Aminogruppe ist an der linken Seite der Abkürzung, die Carboxygruppe an der rechten Seite zu denken. Das Fehlen eines H-Atoms in der $\alpha$-Amino-Gruppe wird durch einen links an der Abkürzung für die Aminosäure stehenden Bindestrich gekennzeichnet. Das Fehlen einer HO-Gruppe in der Carboxygruppe wird durch einen rechts stehenden Bindestrich ausgedrückt. Substituenten in der Seitenkette von Aminosäuren werden unmittelbar hinter das Aminosäuresymbol in Klammern gesetzt oder sind mit dem Aminosäuresymbol durch einen davon senkrecht nach oben oder unten ausgehenden Strich verbunden.

Die Abkürzung

```
S——S
|  |
Cy Cy
```

steht für Cystin.

Bisher ist nicht bekannt, ob die erfindungsgemässen Peptide in der Natur, d.h. z.B. im menschlichen Organismus, als solche vorkommen. Sollte dies aber der Fall sein, so betrifft die Erfindung in erster Linie die obengenannten Peptide und deren Salze in einer höheren Konzentration als sie gegebenenfalls in der Natur oder in gegebenenfalls bekannten Extrakten vorkommen. Insbesondere betrifft die Erfindung die obengenannten Peptide in isolierter oder gereinigter Form, in reiner oder im wesentlichen reiner Form, in einer im wesentlichen anderen Umgebung, d.h. mit anderen Beimengungen, z.B. mit beigemischten pharmazeutischen Trägermaterialien, als sie gegebenenfalls in der Natur vorkommen, in einer zur pharmazeutischen Verwendung geeigneten Form, in charakterisierter Form und/oder in einer Form ausserhalb eines lebenden oder toten Organismus, eines Organs, einer Zelle, eines Gewebes und einer Körperflüssigkeit Insbesondere betrifft die Erfindung die synthetisch oder gentechnisch hergestellten Peptide und deren Salze.

Im obigen Zusammenhang bedeutet der Begriff "isoliert" abgetrennt von anderen Substanzen, insbesondere von anderen chemischen Verbindungen, mit denen die erfindungsgemässen Verbindungen gegebenenfalls in der Natur zusammen vorkommen. Der Begriff "gereinigt" bedeutet chemischen und/oder physikalischen Reinigungsmethoden unterzogen. Der Begriff "im wesentlichen rein" bedeutet eine Reinheit von mehr als 50 %.

Die Erfindung betrifft auch die Salze, insbesondere die pharmazeutisch verwendbaren, ungiftigen Salze der erfindungsgemässen Peptide. Die obengenannten Peptide können Säureadditionssalze bilden, z.B. mit anorganischen Säuren, insbesondere Mineralsäuren, z.B. Salzsäure, Schwefelsäure oder Phosphorsäure,

oder Salze mit organischen Carbon-, Sulfon- oder Sulfosäuren, z.B. Essigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nicotinsäure oder Isonicotinsäure, ferner Aminosäuren, sowie Methansulfonsäure, Ethansulfonsäure, 2-Hydroxyethansulfonsäure, Ethan-1,2-disulfonsäure, Benzolsulfonsäure, 4-Methyl-benzolsulfonsäure oder Naphthalin-2-sulfonsäure, oder mit anderen sauren organischen Verbindungen, wie Ascorbinsäure.

Diejenigen der obengenannten Peptide, die mindestens eine Carboxylgruppe und mindestens eine basische Gruppe, z.B. eine Aminogruppe, aufweisen, können innere Salze bilden.

Diejenigen der obengenannten Peptide, die mindestens eine freie Carboxylgruppe enthalten, können, insbesondere wenn sie mehr Carboxylgruppen als basische Gruppen aufweisen, Metall- oder Ammoniumsalze bilden, wie Alkalimetall- und Erdalkalimetall-, z.B. Natrium-, Kalium-, Magnesium- oder Calciumsalze, sowie Ammoniumsalze mit Ammoniak oder geeigneten organischen Aminen, wobei in erster Linie aliphatische, cycloaliphatische, cycloaliphatisch-aliphatische oder araliphatische primäre, sekundäre oder tertiäre Mono-, Di- oder Polyamine, sowie heterocyclische Basen für die Salzbildung in Frage kommen, wie Niederalkylamine, z.B. Triethylamin, Hydroxyniederalkylamine, z.B. 2-Hydroxyethylamin, Bis-(2-hydroxyethyl)-amin, 2-Hydroxy-ethyl-diethyl-amin oder Tri-(2-hydroxyethyl)-amin, basische aliphatische Ester von Carbonsäuren, z.B. 4-Aminobenzoesäure-2-diethylaminoethylester, Niederalkylenamine, z.B. 1-Ethylpiperidin, Cycloalkylamine, z.B. Dicyclohexylamin, oder Benzylamine, z.B. N,N'-Dibenzylethylendiamin, ferner Basen vom Pyridintyp, z.B. Pyridin, Collidin oder Chinolin.

Zur Isolierung oder Reinigung können auch pharmazeutisch ungeeignete Salze Verwendung finden. Zur therapeutischen Anwendung gelangen jedoch nur die pharmazeutisch verwendbaren, nicht-toxischen Salze, die deshalb bevorzugt werden.

Die erfindungsgemässen Peptidamide sind in Femtomol-Dosierungen vasodilatorisch wirksam und führen zu einer Senkung des Blutdrucks und zu einem Anstieg der Herzfrequenz sowie der Kontraktilität des Myokards. Die Wirkungen werden durch Blocker des sympathischen Nervensystems, z.B. Metoprolol [1-Isopropylamino-3-(4-{2-methoxyethyl}-phenoxy)-propan-2-ol] und Trandate® (Labetalolhydrochlorid) nicht beeinflusst, während die Wirkung von Noradrenalin und von Adrenalin im Serum durch diese Substanzen unterdrückt wird.

Bolus Injektionen von etwa 100 bis 10000 ng der erfindungsgemässen Peptidamide, z.B. von CGRP II, induzieren am isoliert perfundierten Mesenterialbett der Ratte, welches durch Infusion von 4 μg/ml Noradrenalin präkonstringiert worden ist, eine konzentrationsabhängige Gefässerweiterung, die sich in einer Senkung des Perfusionsdruckes um bis zu etwa 5300 Pa (40 mm Hg) äussert.

In der despinalisierten Ratte (Gillesbie und Muir, Brit. J. Pharmacol. 30, 88-98 [1967]) senken die erfindungsgemässen Peptidamide, z.B. CGRP II, in einer Dosierung von 1 μg/kg/Minute den Blutdruck, erhöhen die Herzfrequenz um etwa 40 Schläge pro Minute und hemmen die durch Elektrostimulation des Sympathikus sowie die durch intravenöse Injektion von Angiotensin II oder Adrenalin hervorgerufenen pressorischen Effekte.

Die erfindungsgemässen Peptidamide unterdrücken die Magensaftsekretion (Sekretionsvolumen, Freisetzung von Säure und Pepsin) sowohl basal als auch nach Stimulation mittels Bethanechol oder Histamin. Sie wirken auch auf das zentrale Nervensystem.

Die Peptidamide besitzen auch hypokalzämische Wirkung, d.h. sie erniedrigen den Kalziumgehalt im Blut und schützen den Knochen vor Mineralverlust.

Aufgrund der beschriebenen pharmakologischen Wirkungen können die erfindungsgemässen Peptidamide als Arzneimittel verwendet werden, insbesondere zur Erweiterung der Blutgefässe und Steigerung der Gewebedurchblutung sowie gegen Angina pectoris, Hypertonie, Herzinsuffizienz oder andere Herzkreislauferkrankungen, daneben für die gleichen Indikationen wie das humane Calcitonin, z.B. das sich auf dem Markt befindliche Cibacalcin®, z.B. bei Knochenstoffwechselstörungen, wie Osteoporose oder Morbus Paget.

Die Dosierung beträgt etwa 0,1-50 μg/kg, vorzugsweise etwa 1-10 μg/kg Körpergewicht.

Die einem Warmblütler, insbesondere dem Menschen, von etwa 70 kg Körpergewicht zu verabreichenden Dosen betragen etwa 1 μg bis etwa 1000 μg, vorzugsweise etwa 10 μg bis etwa 100 μg, z.B. etwa 40 μg, pro Tag. Die Verabreichung erfolgt vorzugsweise parenteral, z.B. intravenös an 5 Tagen in der Woche während 3 Monaten.

Die erfindungsgemässen Verbindungen können auf an sich bekannte Weise hergestellt werden.

Das erfindungsgemässe Verfahren zur Herstellung der obengenannten Peptide, Peptidamide oder ihrer Derivate mit acylierter terminaler Aminogruppe ist dadurch gekennzeichnet, dass man

a) eine in einer solchen Verbindung vorhandene Amidbindung durch Umsetzung eines Fragments dieser

Verbindung, das eine freie Carboxylgruppe aufweist, oder eines reaktionsfähigen Carbonsäurederivats davon mit dem komplementären Fragment, das eine freie Aminogruppe aufweist, oder mit einem reaktionsfähigen Derivat davon, wobei in den genannten Fragmenten freie funktionelle Gruppen mit Ausnahme der beiden an der Reaktion teilnehmenden Gruppen erforderlichenfalls in geschützter Form vorliegen, knüpft, und gegebenenfalls vorhandene Schutzgruppen abspaltet, oder

b) zur Herstellung einer der obengenannten Verbindungen, die eine intra-oder intermolekulare Disulfidbrücke aufweist, eine der obengenannten Verbindungen, worin die Mercaptogruppen der Cysteinreste in freier Form vorliegen oder worin die Mercaptogruppen der Cysteinreste durch Schutzgruppen geschützt sind, die unter den Reaktionsbedingungen abgespalten werden, mit einem geeigneten Oxidationsmittel oxidiert, oder

c) in einem solchen Peptid, Peptidamid oder N-acylierten Derivat davon, worin mindestens eine funktionelle Gruppe in geschützter Form vorliegt, die vorhandenen Schutzgruppen abspaltet, oder

d) zur Herstellung der obengenannten Peptide Wirtszellen, die mit einem Expressionsvektor transformiert sind, welches eine von einer Expressionskontrollsequenz regulierte, für die gewünschte Aminosäuresequenz kodierende DNA-Sequenz enthält, in einem flüssigen Nährmedium, welches assimilierbare Kohlenstoff- und Stickstoffquellen enthält, kultiviert, das Produkt erforderlichenfalls aus den Wirtszellen freisetzt und isoliert und, falls erforderlich, ein gegebenenfalls erhaltenes Di- oder Polymeres mit einem zur Aufspaltung von Disulfidbindungen geeigneten Reduktionsmittel umsetzt und nötigenfalls das erhaltene reduzierte Produkt mit einem zur Neuknüpfung von Disulfidbindungen geeigneten Oxidationsmittel behandelt, oder

e) zur Herstellung eines der obengenannten Peptidamide ein Peptid, welches zusätzlich zur Aminosäuresequenz des gewünschten Peptidamids C-terminal eine Aminosäure $AS^{\Omega}$ aufweist, die enzymatisch zur $NH_2$-Gruppe der terminalen Amidgruppe des gewünschten Peptidamids abgebaut werden kann oder die durch Ammoniak in Gegenwart eines geeigneten Enzyms substituiert werden kann, mit Hilfe eines geeigneten Enzyms gegebenenfalls in Anwesenheit von Ammoniak behandelt,

und, wenn erwünscht, nach Durchführung einer der Verfahrensvarianten a-e) ein erhaltenes Salz in die freie Verbindung überführt oder eine erhaltene freie Verbindung in ihr Salz überführt.

Die obengenannten Verfahrensvarianten werden im folgenden näher erläutert:

Verfahren a: Gemäss diesem Verfahren wird als letzte Reaktionsstufe bei der Synthese des Endprodukts eine Amidbindung an beliebiger Stelle des Moleküls geknüpft. Bei dem genannten Fragment, das eine freie Carboxylgruppe aufweist, kann es sich sowohl um eine einzelne Aminosäure oder um ein Di-, Oligo- oder Polypeptid als auch, im Falle der Derivate mit acylierter terminaler Aminogruppe, um die acylierende Carbonsäure, wie insbesondere Essigsäure, handeln. Das obengenannte Fragment, das eine freie Aminogruppe aufweist, stellt eine einzelne Aminosäure, ein Di-, Oligo- oder Polypeptid oder, im Falle der Herstellung von Peptidamiden, auch ein Amin, wie insbesondere Ammoniak dar.

Vorzugsweise wird die Reaktion so durchgeführt, dass man ein reaktionsfähiges Carbonsäurederivat des einen Fragments mit dem komplementären Fagment, das eine freie Aminogruppe aufweist, umsetzt, wobei die Aktivierung der Carboxylgruppe des Carbonsäurederivats auch in situ erfolgen kann.

Reaktionsfähige Carbonsäurederivate sind in erster Linie reaktionsfähige aktivierte Ester oder reaktionsfähige Anhydride, ferner reaktionsfähige cyclische Amide; dabei können reaktionsfähige Säurederivate auch in situ gebildet werden.

Aktivierte Ester von Säuren sind insbesondere am Verknüpfungskohlenstoffatom des veresternden Restes ungesättigte Ester, z.B. vom Vinylester-Typ, wie eigentliche Vinylester (die man z.B. durch Umesterung eines entsprechenden Esters mit Vinylacetat erhalten kann; Methode des aktivierten Vinylesters), Carbamoylvinylester (die man z.B. durch Behandeln der entsprechenden Säure mit einem Isoxazoliumreagens erhalten kann; 1,2-Oxazolium- oder Woodward-Methode), oder 1-Niederalkoxyvinylester (die man z.B. durch Behandeln der entsprechenden Säure mit einem Niederalkoxyacetylen erhalten kann; Ethoxyacetylen-Methode), oder Ester vom Amidinotyp, wie N,N'-disubstituierte Amidinoester (die man z.B. durch Behandeln der entsprechenden Säure mit einem geeigneten N,N'-disubstituierten Carbodiimid, z.B. N,N'-Dicyclohexylcarbodiimid, erhalten kann; Carbodiimid-Methode), oder N,N-disubstituierte Amidinoester (die man z.B. durch Behandeln der entsprechenden Säure mit einem N,N-disubstituierten Cyanamid erhalten kann; Cyanamid-Methode), geeignete Arylester, insbesondere durch Elektronen-anziehende Substituenten geeignet substituierte Phenylester (die man z.B. durch Behandeln der entsprechenden Säure mit einem geeignet substituierten Phenol, z.B. 4-Nitrophenol, 4-Methylsulfonyl-phenol, 2,4,5-Trichlorphenol, 2,3,4,5,6-Pentachlor-phenol oder 4-Phenyldiazophenol, in Gegenwart eines Kondensationsmittels, wie N,N'-Dicyclohexyl-carbodiimid, erhalten kann; Methode der aktivierten Arylester), Cyanmethylester (die man z.B. durch Behandeln der entsprechenden Säure mit Chloracetonitril in Gegenwart einer Base erhalten kann; Cyanmethylester-Methode), Thioester, insbesondere gegebenenfalls, z.B. duch Nitro, substituierte Phenylthioester (die man z.B. durch Behandeln der entsprechenden Säure mit gegebenenfalls, z. B. durch Nitro, substituierten Thiophenolen, u.a. mit Hilfe der Anhydrid-oder Carbodiimid-Methode, erhalten kann; Methode der aktivierten Thiolester), Amino-oder Amidoester (die man z.B. durch Behandeln der entsprechenden Säure mit einer N-Hydroxy-amino- bzw. N-Hydroxy-amido-Verbindung, z.B. N-Hydroxy-succinimid, N-Hydroxy-piperidin, N-Hydroxyphthalimid oder 1-Hydroxy-benztriazol, z.B. nach der Anhydrid- oder Carbodiimid-Methode, erhalten kann; Methode der aktivierten N-Hydroxyester) oder Silylester (die man z.B. durch Behandeln der entsprechenden Säure mit einem Silylierungsmittel, z.B. Hexamethyldisilazan, erhalten kann und die leicht mit Hydroxy-, nicht aber Aminogruppen reagieren).

Säureanhydride können symmetrische oder vorzugsweise gemischte Anhydride dieser Säuren sein, so z.B. Anhydride mit anorganischen Säuren, wie Säurehalogenide, insbesondere Säurechloride (die man z.B. durch Behandeln der entsprechenden Säure mit Thionylchlorid, Phosphorpentachlorid oder Oxalylchlorid erhalten kann; Säurechloridmethode), Azide (die man z.B. aus einem entsprechenden Säureester über das entsprechende Hydrazid und dessen Behandlung mit salpetriger Säure erhalten kann; Azidmethode), Anhydride mit Kohlensäurehalbderivaten, wie mit entsprechenden Estern, z.B. Kohlensäureniederalkylhalbestern (die man z.B. durch Behandeln der entsprechenden Säure mit Halogen-, wie Chlorameisensäureniederalkylestern oder mit einem 1-Niederalkoxycarbonyl-2-niederalkoxy-1,2-dihydro-chinolin, z.B. 1-Niederalkoxycarbonyl-2-ethoxy-1,2-dihydrochinolin, erhalten kann; Methode der gemischten O-Alkyl-kohlensäureanhydride), oder Anhydride mit dihalogenierter, insbesondere dichlorierter Phosphorsäure (die man z.B. durch Behandeln der entsprechenden Säure mit Phosphoroxychlorid erhalten kann; Phosphoroxychloridmethode), oder Anhydride mit organischen Säuren, wie gemischte Anhydride mit organischen Carbonsäuren (die man z.B. durch Behandeln der entsprechenden Säure mit einem gegebenenfalls substituierten Niederalkan- oder Phenylalkancarbonsäurehalogenid, z.B. Phenylessigsäure-, Pivalinsäure- oder Trifluoressigsäurechlorid erhalten kann; Methode der gemischten Carbonsäureanhydride) oder mit organischen Sulfonsäuren (die man z.B. durch Behandeln eines Salzes, wie eines Alkalimetallsalzes, der entsprechenden Säure, mit einem geeigneten organischen Sulfonsäurehalogenid, wie Niederalkan- oder Aryl-, z.B. Methan- oder p-Toluolsulfonsäurechlorid, erhalten kann; Methode der gemischten Sulfonsäureanhydride), sowie symmetrische Anhydride (die man z.B. durch Kondensation der entsprechenden Säure in Gegenwart eines Carbodiimids oder von 1-Diethylaminopropin erhalten kann; Methode der symmetrischen Anhydride).

Geeignete cyclische Amide sind insbesondere Amide mit fünfgliedrigen Diazacyclen aromatischen Charakters, wie Amide mit Imidazolen, z.B. Imidazol (die man z.B. durch Behandeln der entsprechenden Säure mit N,N'-Carbonyldiimidazol erhalten kann; Imidazolid-Methode), oder Pyrazolen, z.B. 3,5-Dimethylpyrazol (die man z.B. über das Säurehydrazid durch Behandeln mit Acetylaceton erhalten kann; Pyrazolid-Methode).

Wie erwähnt, können die Carbonsäurederivate auch in situ gebildet werden. So kann man z.B. N,N'-disubstituierte Amidinoester in situ bilden, indem man das Gemisch des komplementären Fragments mit der freien Aminogruppe und des Peptidfragments mit freier Carboxylgruppe in Gegenwart eines geeigneten N,N-disubstituierten Carbodiimids, z.B. N,N'-Dicyclohexylcarbodiimid, zur Reaktion bringt. Ferner kann man Amino- oder Amidoester der Säure in Gegenwart des zu acylierenden Amins bilden, indem man das Gemisch der entsprechenden Säure- und Amino-Ausgangsstoffe in Gegenwart eines N,N'-disubstituierten Carbodiimids, z.B. N,N'-Dicyclohexyl-carbodiimid, und eines N-Hydroxy-amins oder N-Hydroxy-amids, z.B. N-Hydroxysuccinimid, gegebenenfalls in Anwesenheit einer geeigneten Base, z.B. 4-Dimethylamino-pyridin, umsetzt.

Alternativ kann Verfahrensvariante a) auch so durchgeführt werden, dass man ein Fragment mit freier Carboxylgruppe mit dem komplementären Fragment umsetzt, in dem die Aminosäure in reaktionsfähiger Form vorliegt. Die Aminogruppe kann z.B. durch Umsetzung mit einem Phosphit, z.B. Diethylchlorphosphit, 1,1-Phenylen-chlorphosphit, Ethyl-dichlor-phosphit, Ethylen-chlor-phosphit oder Tetraethylpyrophosphit, aktiviert werden.

Die Aminogruppe kann auch durch Bindung an Halogencarbonyl, z.B. Chlorcarbonyl, oder in Form einer Isocyanatgruppe aktiviert sein.

Freie funktionelle Gruppen in den genannten Fragmenten, die, sofern sie nicht an der Reaktion teilnehmen sollen, vorteilhafterweise in geschützter Form vorliegen, sind insbesondere Carboxyl-, Amino-, Hydroxy- und Mercaptogruppen.

Schutzgruppen, ihre Einführung und Abspaltung sind beispielsweise beschrieben in "Protective Groups in Organic Chemistry", Plenum Press, London, New York 1973, und in "Methoden der organischen Chemie", Houben-Weyl, 4. Auflage, Bd. 15/1, Georg-Thieme-Verlag, Stuttgart 1974 sowie in Theodora W. Greene, "Protective Groups in Organic Synthesis, John Wiley & Sons, New York 1981. Charakteristisch für Schutzgruppen ist, dass sie leicht, d.h. ohne dass unerwünschte Nebenreaktionen stattfinden, z.B. solvolytisch, reduktiv, photolytisch oder auch unter physiologischen Bedingungen abspaltbar sind.

Hydroxyschutzgruppen sind z.B. Acylreste, wie gegebenenfalls, z.B. durch Halogen, substituiertes Niederalkanoyl, wie 2,2-Dichloracetyl, oder Acylreste von Kohlensäurehalbestern, insbesondere tert.-Butyloxycarbonyl, gegebenenfalls substituiertes Benzyloxycarbonyl, z.B. 4-Nitro-benzyloxycarbonyl, oder Diphenylmethoxycarbonyl, oder 2-Halogen-niederalkoxycarbonyl, wie 2,2,2-Trichlorethoxycarbonyl, ferner Trityl oder Formyl, oder organische Silyl- oder Stannylreste, ferner leicht abspaltbare verethernde Gruppen, wie tert.-Niederalkyl, z.B. tert.-Butyl, 2-oxa-oder 2-thia-aliphatische oder -cycloaliphatische Kohlenwasserstoffreste, in erster Linie 1-Niederalkoxyniederalkyl oder 1-Niederalkylthio-niederalkyl, z.B. Methoxy-methyl, 1-Methoxy-ethyl, 1-Ethoxy-ethyl, Methylthiomethyl, 1-Methylthioethyl oder 1-Ethylthioethyl, oder 2-Oxa- oder 2-Thiacycloalkyl mit 5-6 Ringatomen, z.B. Tetrahydrofuryl oder 2-Tetrahydropyranyl oder entsprechende Thiaanaloge, sowie gegebenenfalls substituiertes 1-Phenylniederalkyl, wie gegebenenfalls substituiertes Benzyl oder Diphenylmethyl, wobei als Substituenten der Phenylreste z.B. Halogen, wie Chlor, Niederalkoxy, wie Methoxy und/oder Nitro in Frage kommen.

Carboxylgruppen sind üblicherweise in veresterter Form geschützt, wobei solche Estergruppierungen unter schonenden Bedingungen leicht spaltbar sind. In dieser Art geschützte Carboxylgruppen enthalten als veresternde Gruppen in erster Linie in 1-Stellung verzweigte oder in 1- oder 2-Stellung geeignet substituierte Niederalkylgruppen. Bevorzugte, in veresterter Form vorliegende Carboxylgruppen sind u.a. tert.-Niederalkoxycarbonyl, z.B. tert.-Butyloxycarbonyl, Arylmethoxycarbonyl mit einem oder zwei Arylresten, wobei diese gegebenenfalls z.B. durch Niederalkyl, wie tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellen, wie gegebenenfalls, z.B. wie oben erwähnt, substituiertes Benzyloxycarbonyl, z.B. 4-Methoxybenzyloxycarbonyl, oder 4-Nitrobenzyloxycarbonyl, oder gegebenenfalls, z.B. wie oben erwähnt, substituiertes Diphenylmethoxycarbonyl, z.B. Diphenylmethoxycarbonyl oder Di-(4-methoxyphenyl)-methoxycarbonyl, 1-Niederalkoxyniederalkoxycarbonyl, wie Methoxymethoxycarbonyl, 1-Methoxyethoxycarbonyl oder 1-Ethoxymethoxycarbonyl, 1-Niederalkylthioniederalkoxycarbonyl, wie 1-Methylthiomethoxycarbonyl oder 1-Ethylthioethoxycarbonyl, Aroylmethoxycarbonyl, worin die Aroylgruppe gegebenenfalls, z.B. durch Halogen, wie Brom, substituiertes Benzoyl darstellt, z.B. Phenacyloxycarbonyl, 2-Halogenniederalkoxycarbonyl, z.B. 2,2,2,-Trichlorethoxycarbonyl, 2-Bromethoxycarbonyl oder 2-Iodethoxycarbonyl, oder 2-(trisubstituiertes Silyl)-ethoxycarbonyl, worin die Substituenten unabhängig voneinander je einen gegebenenfalls substituierten, z.B. durch Niederalkyl, Niederalkoxy, Aryl, Halogen, und/oder Nitro substituierten, aliphatischen, araliphatischen, cycloaliphatischen oder aromatischen Kohlenwasserstoffrest, wie entsprechendes, gegebenenfalls substituiertes Niederalkyl, Phenylniederalkyl, Cycloalkyl oder Phenyl, bedeuten, z.B. 2-Triniederalkylsilylethoxycarbonyl, 2-Trimethylsilylethoxycarbonyl oder 2-(Di-n-butylmethyl-silyl)-ethoxycarbonyl, oder 2-Triarylsilylethoxycarbonyl, wie 2-Triphenylsilylethoxycarbonyl.

Die oben und im folgenden erwähnten organischen Silyl- oder Stannylreste enthalten vorzugsweise Niederalkyl, insbesondere Methyl, als Substituenten der Silizium- oder Zinn-Atome. Entsprechende Silyl- oder Stannylgruppen sind in erster Linie Triniederalkylsilyl, insbesondere Trimethylsilyl, ferner Dimethyl-tert.-butyl-silyl, oder entsprechend substituiertes Stannyl, z.B. Tri-n-butylstannyl.

Bevorzugte geschützte Carboxylgruppen sind tert.-Niederalkoxycarbonyl, wie tert.-Butoxycarbonyl, und in erster Linie gegebenenfalls, z.B. wie oben erwähnt, substituiertes Benzyloxycarbonyl, wie 4-Nitrobenzyloxycarbonyl, oder Diphenylmethoxycarbonyl, vor allem 2-(Trimethylsilyl)-ethoxycarbonyl, sowie 4-Benzyloxycarbonylbenzyloxycarbonyl, worin die Phenylgruppe des erstgenannten Benzyloxycarbonyl-Restes Teil eines polymeren Trägers ist, d.h. z.B. Teil von Polystyrol, das mit Divinylbenzol quervernetzt ist.

Eine geschützte Aminogruppe kann z.B. in Form einer leicht spaltbaren Acylamino-, Arylmethylamino-, verätherten Mercaptoamino-, 2-Acyl-niederalk-1-en-yl-amino-, Silyl- oder Stannylaminogruppe oder als Azidogruppe vorliegen.

In einer entsprechenden Acylaminogruppe ist Acyl beispielsweise der Acylrest einer organischen Carbonsäure mit z.B. bis zu 18 Kohlenstoffatomen, insbesondere einer gegebenenfalls,z.B. durch Halogen oder Aryl, substituierten Alkancarbonsäure oder gegebenenfalls, z.B. durch Halogen, Niederalkoxy oder Nitro, substituierten Benzoesäure, oder eines Kohlensäurehalbesters. Solche Acylgruppen sind beispielsweise Niederalkanoyl, wie Formyl, Acetyl oder Propionyl, Halogenniederalkanoyl, wie 2-Halogenacetyl, insbesondere 2-Chlor-, 2-Brom-, 2-Iod-, 2,2,2-Trifluor- oder 2,2,2-Trichloracetyl, gegebenenfalls, z.B. durch Halogen, Niederalkoxy oder Nitro substituiertes Benzoyl, z.B. Benzoyl, 4-Chlorbenzoyl), 4-Methoxybenzoyl oder 4-Nitrobenzoyl, oder in 1-Stellung des Niederalkylrestes verzweigtes oder in 1- oder 2-Stellung geeignet substituiertes Niederalkoxycarbonyl, insbesondere tert.-Niederalkoxycarbonyl, z.B. tert.-Butyloxycarbonyl, Arylmethoxycarbonyl mit einem oder zwei Arylresten, die vorzugsweise gegebenenfalls, z.B. durch Niederalkyl, insbesondere tert.-Niederalkyl, wie tert.Butyl, Niederalkoxy, wie Methoxy, Hydroxy, Halogen, z.B. Chlor, und/oder Nitro,mono- oder polysubstituiertes Phenyl darstellen, wie gegebenenfalls substituiertes Benzyloxycarbonyl, z.B. 4-Nitro-benzyloxycarbonyl, oder substituiertes Diphenylmethoxycarbonyl, z.B. Benzhydryloxycarbonyl oder Di-(4-methoxyphenyl)-methoxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogen-niederalkoxycarbonyl, z.B. 2,2,2-Trichlorethoxycarbonyl, 2-Bromethoxycarbonyl oder 2-Iodethoxycarbonyl, oder 2-(trisubstituiertes Silyl)-ethoxycarbonyl, worin die Substituenten unabhängig voneinander je einen gegebenenfalls substituierten, z.B. durch Niederalkyl, Niederalkoxy, Aryl, Halogen oder Nitro substituierten, aliphatischen, araliphatischen, cycloaliphatischen oder aromatischen Kohlenwasserstoffrest mit bis zu 15 C-Atomen, wie entsprechendes, gegebenenfalls substituiertes Niederalkyl, Phenylniederalkyl, Cycloalkyl oder Phenyl, bedeuten, z.B. 2-Triniederalkylsilylethoxycarbonyl, wie 2-Trimethylsilylethoxycarbonyl oder 2-(Di-n-butylmethyl-silyl)-ethoxycarbonyl, oder 2-Triarylsilylethoxycarbonyl, wie 2-Triphenylsilylethoxycarbonyl.

Weitere, als Aminoschutzgruppen in Frage kommende Acylreste sind auch entsprechende Reste organischer Phosphor-, Phosphon- oder Phosphinsäuren, wie Diniederalkylphosphoryl, z.B. Dimethylphosphoryl, Diethylphosphoryl, Di-n-propylphosphoryl oder Diisopropylphosphoryl, Dicycloalkylphosphoryl, z.B. Dicyclohexylphosphoryl, gegebenenfalls substituiertes Diphenylphosphoryl, z.B. Diphenylphosphoryl, gegebenenfalls, z.B. durch Nitro substituiertes Di(phenylniederalkyl)-phosphoryl,z.B. Dibenzylphosphoryl oder Di-(4-nitrobenzyl)-phosphoryl, gegebenenfalls substituiertes Phenyloxy-phenyl-phosphonyl, z.B. Phenyloxyphenyl-phosphonyl, Diniederalkylphosphinyl, z.B. Diethylphosphinyl, oder gegebenenfalls substituiertes Diphenylphosphinyl, z.B. Diphenylphosphinyl.

In einer Arylmethylaminogruppe, die eine Mono-, Di- oder insbesondere Triarylmethylaminogruppe darstellt, sind die Arylreste insbesondere gegebenenfalls substituierte Phenylreste. Solche Gruppen sind beispielsweise Benzyl-, Diphenylmethyl- und insbesondere Tritylamino.

Eine verätherte Mercaptogruppe in einer mit einem solchen Rest geschützten Aminogruppe ist in erster Linie Arylthio oder Arylniederalkylthio, worin Aryl insbesondere gegebenenfalls, z.B. durch Niederalkyl, wie Methyl oder tert.-Butyl, Niederalkoxy, wie Methoxy, Halogen, wie Chlor, und/oder Nitro substituiertes Phenyl ist. Eine entsprechende Aminoschutzgruppe ist z.B. 4-Nitrophenylthio.

In einem als Aminoschutzgruppe verwendbaren 2-Acyl-niederalk-1-en-1-yl-rest ist Acyl z.B. der entsprechende Rest einer Niederalkancarbonsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters. Entsprechende Schutzgruppen sind in erster Linie 1-Niederalkanoyl-prop-1-en-2-yl, z.B. 1-Acetyl-prop-1-en-2-yl, oder 1-Niederalkoxycarbonyl-prop-1-en-2-yl, z.B. 1-Ethoxycarbonyl-prop-1-en-2-yl.

Eine Aminogruppe kann auch in protonierter Form geschützt werden; als entsprechende Anionen kommen in erster Linie diejenigen von starken anorganischen Säuren, wie von Halogenwasserstoffsäuren, z.B. das Chlor- oder Bromanion, oder von organischen Sulfonsäuren, wie p-Toluolsulfonsäure, in Frage.

Bevorzugte Aminoschutzgruppen sind Acylreste von Kohlensäurehalbestern, insbesondere tert.-Butyloxycarbonyl, gegebenenfalls, z.B. wie angegeben, substituiertes Benzyloxycarbonyl, z.B. 4-Nitro-benzyloxycarbonyl, oder Diphenylmethoxycarbonyl, oder 2-Halogen-niederalkoxycarbonyl, wie 2,2,2-Trichlorethoxycarbonyl, ferner Trityl oder Formyl.

Eine Mercaptogruppe, wie z.B. in Cystein, kann insbesondere durch S-Alkylierung mit gegebenenfalls substituierten Alkylresten, Thioacetalbildung, S-Acylierung oder durch das Erstellen asymmetrischer Disulfid-Gruppierungen geschützt werden.Bevorzugte Mercaptoschutzgruppen sind z.B. gegebenenfalls im Phenylrest, z.B. durch Methoxy oder Nitro, substituiertes Benzyl, wie 4-Methoxybenzyl, gegebenenfalls im Phenylteil, z.B. durch Methoxy, substituiertes Diphenylmethyl, wie 4,4'-Dimethoxydiphenyl-methyl, Triphenylmethyl, Trimethylsilyl, Benzyl-thiomethyl, Tetrahydropyranyl, Acylaminomethyl,z.B. Acetylaminomethyl, Benzoyl, Benzyloxycarbonyl oder Aminocarbonyl, wie Ethylaminocarbonyl.

Primäre Carbonsäureamidgruppen ($-CONH_2$) sind z.B. in Form von N-(9-Xanthenyl)-Derivaten oder in Form von N-(Mono-, Di- oder Triarylmethyl)-Derivaten geschützt, wobei Aryl insbesondere unsubstituiertes oder mit bis zu 5 gleichen oder verschiedenen Substituenten substituiertes Phenyl bedeutet. Solche Phenylsubstituenten sind vorzugsweise Niederalkyl, wie Methyl, Niederalkoxy, wie Methoxy, oder ausdrücklich auch polymere Träger. Als Beispiele für solche Arylmethyl-Schutzgruppen seien genannt: 4-Methoxybenzyl, 2,4,6-Trimethoxy-benzyl, Diphenyl-methyl, Di-(4-methoxy-phenyl)-methyl,Di-(4-methyl-phenyl)-methyl und (4-methyl-phenyl)-([polymerer Träger]-phenyl)-methyl.

Guanidinogruppen sind z.B. als Toluolsulfonat geschützt.

Als Schutzgruppe, wie insbesondere Carboxylschutzgruppe, im Sinne dieser Anmeldung ist ausdrücklich auch ein in leicht abspaltbarer Weise mit der zu schützenden funktionellen Gruppe, wie insbesondere Carboxylgruppe, verbundener polymerer Träger zu verstehen, wie er z.B. für die Merrifield-Synthese geeignet ist. Ein solcher geeigneter polymerer Träger ist z.B. ein durch Copolymerisation mit Divinylbenzol schwach vernetztes Polystyrolharz, das zur reversiblen Bindung der Peptidreste geeignete Brückenglieder

trägt.

Die Reaktion kann in an sich bekannter Weise durchgeführt werden, wobei die Reaktionsbedingungen in erster Linie davon abhängen, ob und wie die an der Reaktion teilnehmende Carboxylgruppe aktiviert ist, üblicherweise in Gegenwart eines geeigneten Lösungs- oder Verdünnungsmittels oder eines Gemisches von solchen, und, falls notwendig, in Gegenwart eines Kondensationsmittels, das z.B., wenn die an der Reaktion teilnehmende Carboxylgruppe als Anhydrid vorliegt, auch ein Säure-bindendes Mittel sein kann, unter Kühlen oder Erwärmen, z.B. in einem Temperaturbereich von etwa -30 °C bis etwa +150 °C, in einem geschlossenen Reaktionsgefäss und/oder in der Atmosphäre eines Inertgases, z.B. Stickstoff. Uebliche Kondensationsmittel sind z.B. Carbodiimide, beispielsweise N,N'-Diethyl-, N,N'-Dipropyl-, N,N'-Dicyclohexyl- oder N-Ethyl-N'-(3-dimethylaminopropyl)-carbodiimid, geeignete Carbonylverbindungen, beispielsweise Carbonyldiimidazol, oder 1,2-Oxazoliumverbindungen, z.B. 2-Ethyl-5-phenyl-1,2-oxazolium-3'-sulfonat und 2-tert.-Butyl-5-methyl-isoxazolium-perchlorat, oder eine geeignete Acylaminoverbindung, z.B. 2-Ethoxy-1-ethoxycarbonyl-1,2-dihydrochinolin. Uebliche säurebindende Kondensationsmittel sind z.B. Alkalimetallcarbonate oder -hydrogencarbonate, z.B. Natrium- oder Kaliumcarbonat oder -hydrogencarbonat (üblicherweise zusammen mit einem Sulfat), oder organische Basen, wie üblicherweise sterisch gehinderte Triniederalkylamine, z.B. N,N-Diisopropyl-N-ethyl-amin.

Die Abspaltung der Schutzgruppen, z.B. der Carboxyl-, Amino-, Hydroxy-, Carbonsäureamid- oder Mercaptoschutzgruppen, erfolgt in an sich bekannter Weise, z.B. mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig, wobei auch enzymatische Methoden verwendet werden können.

So kann man tert.-Niederalkoxycarbonyl oder in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl z.B. durch Behandeln mit einer geeigneten Säure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, freigesetzt werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zink, oder Metallsalz, wie einem Chrom-II-salz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer Säure, in erster Linie einer geeigneten Carbonsäure, wie einer gegebenenfalls, z.B. durch Hydroxy, substituierten Niederalkancarbonsäure, z.B. Essigsäure, Ameisensäure, Glycolsäure, Diphenylglycolsäure, Milchsäure, Mandelsäure, 4-Chlor-mandelsäure oder Weinsäure, oder eines Alkohols oder Thiols, wobei man vorzugsweise Wasser zugibt, in freies Carboxyl überführen. Durch Behandeln mit einem reduzierenden Metall oder Metallsalz, wie oben beschrieben, kann man auch 2-Halogen-niederalkoxycarbonyl (gegebenenfalls nach Umwandlung einer 2-Brom-niederalkoxycarbonylgruppe in eine entsprechende 2-Iod-niederalkoxycarbonylgruppe) oder Aroylmethoxycarbonyl in freies Carboxyl umwandeln, wobei Aroylmethoxycarbonyl ebenfalls durch Behandeln mit einem nucleophilen, vorzugsweise salzbildenden Reagens, wie Natriumthiophenolat oder Natriumjodid, gespalten werden kann. Substituiertes 2-Silylethoxycarbonyl kann auch durch Behandeln mit einem, das Fluoridanion liefernden Salz der Fluorwaserstoffsäure, wie einem Alkalimetallfluorid, z.B. Natrium-oder Kaliumfluorid, in Anwesenheit eines macrocyclischen Polyethers ("Kronenether"), oder mit einem Fluorid einer organischen quaternären Base, wie Tetra-niederalkylammoniumfluorid oder Triniederalkyl-aryl-ammoniumfluorid, z.B. Tetraethylammoniumfluorid oder Tetrabutylammoniumfluorid, in Gegenwart eines aprotischen polaren Lösungsmittels, wie Dimethylsulfoxid oder N,N-Dimethylacetamid, in freies Carboxyl übergeführt werden.

Eine geschützte Aminogruppe setzt man in an sich bekannter und je nach Art der Schutzgruppen in verschiedenartiger Weise, vorzugsweise mittels Solvolyse oder Reduktion, frei. 2-Halogen-niederalkoxycarbonylamino (gegebenenfals nach Umwandlung einer 2-Brom-niederalkoxycarbonylaminogruppe in eine 2-Iod-niederalkoxycarbonylaminogruppe), Aroylmethoxycarbonylamino oder 4-Nitrobenzyloxycarbonylamino kann z.B. durch Behandeln mit einem geeigneten chemischen Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagens, wie Natriumthiophenolat, und 4-Nitro-benzyloxycarbonylamino auch durch Behandeln mit einem Alkalimetall-, z.B. Natriumdithionit, gespalten werden. Gegebenenfalls substituiertes Diphenylmethoxycarbonylamino, tert.-Niederalkoxycarbonylamino oder 2-trisubstituiertes Silylethoxycarbonylamino kann durch Behandeln mit einer geeigneten Säure, z.B. Ameisen- oder Trifluoressigsäure, gegebenenfalls substituiertes Benzyloxycarbonylamino

z.B. mittels Hydrogenolyse, d.h. durch Behandeln mit Wasserstoff in Gegenwart eines geeigneten Hydrierkatalysators, wie eines Palladiumkatalysators, gegebenenfalls substituiertes Triarylmethylamino oder Formylamino z.B. durch Behandeln mit einer Säure, wie Mineralsäure, z.B. Chlorwasserstoffsäure, oder einer organischen Säure, z.B. Ameisen-, Essig- oder Trifluoressigsäure, gegebenenfalls in Gegenwart von Wasser, gespalten werden, und eine mit einer organischen Silylgruppe geschützte Aminogruppe kann z.B. mittels Hydrolyse oder Alkoholyse freigesetzt werden. Eine durch 2-Halogenacetyl, z.B. 2-Chloracetyl, geschützte Aminogruppe kann durch Behandeln mit Thioharnstoff in Gegenwart einer Base, oder mit einem Thiolatsalz, wie einem Alkalimetallthiolat, des Thioharnstoffs und anschliessende Solvolyse, wie Alkoholyse oder Hydrolyse, des entstandenen Kondensationsprodukts freigesetzt werden. Eine durch 2-substituiertes Silylethoxycarbonyl geschützte Aminogruppe kann auch durch Behandeln mit einem Fluoridanionen liefernden Salz der Fluorwaserstoffsäure, wie oben im Zusammenhang mit der Freisetzung einer entsprechend geschützten Carboxylgruppe angegeben, in die freie Aminogruppe übergeführt werden.

In Form einer Azidogruppe geschütztes Amino wird z.B. durch Reduktion in freies Amino übergeführt, beispielsweise durch katalytische Hydrierung mit Wasserstoff in Gegenwart eines Hydrierkatalysators, wie Platinoxid, Palladium oder Raney-Nickel, oder auch durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z.B. Methylenchlorid, oder auch in Wasser oder einem Gemisch von Wasser und einem organischen Lösungsmittel, wie einem Alkohol oder Dioxan, bei etwa 20° C bis 25° C, oder auch unter Kühlen oder Erwärmen, durchgeführt.

Eine durch eine geeignete Acylgruppe, eine organische Silylgruppe oder durch gegebenenfalls substituiertes 1-Phenylniederalkyl geschützte Hydroxy- oder Mercaptogruppe wird analog einer entsprechend geschützten Aminogruppe freigesetzt. Eine durch 2,2-Dichloracetyl geschützte Hydroxy- bzw. Mercaptogruppe wird z.B. durch basische Hydrolyse, eine durch tert.-Niederalkyl oder durch einen 2-oxa- oder 2-thia-aliphatischen oder -cycloaliphatischen Kohlenwasserstoffrest veretherte Hydroxy- bzw. Mercaptogruppe durch Acidolyse, z.B. durch Behandeln mit einer Mineralsäure oder einer starken Carbonsäure, z.B. Trifluoressigsäure, freigesetzt. Zwei Hydroxygruppen, die zusammen mittels einer vorzugsweise substituierten Methylengruppe, wie durch Niederalkyliden, z.B. Isopropyliden, Cycloalkyliden, z.B. Cyclohexyliden, oder Benzyliden, geschützt sind, können durch saure Solvolyse, besonders in Gegenwart einer Mineralsäure oder einer starken organischen Säure, freigesetzt werden.

Eine durch 9-Xanthenyl geschützte Carbonsäureamidgruppe wird z.B. mit Bromwasserstoff in Eisessig oder mit Fluorwasserstoff in Gegenwart von Anisol freigesetzt.

Eine durch Mono-, Di- oder Triarylmethyl geschützte Carbonsäureamidgruppe kann z.B. mit Fluorwasserstoff in Gegenwart von Anisol freigesetzt werden; darüberhinaus kann z.B. eine Diphenylmethyl-Schutzgruppe hydrogenolytisch in Gegenwart eines Palladium-Kohle-Katalysators und eine Di-(4-methoxy-phenyl)-methyl-Schutzgruppe sowie eine 2,4,6-Trimethoxy-benzyl-Schutzgruppe mittels Trifluoressigsäure abgespalten werden. Auch eine Diarylmethyl-Schutzgruppe, worin ein Arylrest Teil eines polymeren Trägers ist, kann mit Fluorwasserstoff in Gegenwart von Anisol abgespalten werden.

Wenn erwünscht, werden beim Vorhandensein von mehreren geschützten funktionellen Gruppen die Schutzgruppen so gewählt, dass gleichzeitig mehr als eine solche Gruppe abgespalten werden kann, beispielsweise acidolytisch, wie durch Behandeln mit Trifluoressigsäure oder Ameisensäure, oder reduktiv, wie durch Behandeln mit Zink und Essigsäure, oder mit Wasserstoff und einem Hydrierkatalysator, wie einem Palladium-Kohle-Katalysator.

Die Reaktionsvariante a) umfasst ausdrücklich auch die Ausführungsform des Verfahren zur Herstellung von Peptidamiden, wobei die terminale Carboxylgruppe des Peptids von einem polymeren Träger mit Hilfe von Ammoniak abgespalten und dabei, gewissermassen in situ, in das Amid überführt wird. In diesem Fall stellt Ammoniak das komplementäre Fragment mit freier Aminogruppe dar.

Verfahren b: Geeignete Oxidationsmittel zur Oxidation von Mercaptogruppen zwecks Knüpfung von Disulfidbindungen sind z.B. Luftsauerstoff, welcher vorzugsweise durch eine wässrige Lösung des Polypeptids, der gegebenenfalls eine katalytische Menge eines Uebergangsmetallsalzes, z.B. Eisen-(III)-sulfat, Eisen-(III)-chlorid oder Kupfer-(II)-sulfat, beigefügt wurde, geleitet wird; Iod, auch in Form des Kaliumiodid-Adduktes $KI_3$, welches vorzugsweise in alkoholischer, z.B. methanolischer, oder wässrig-alkoholischer, z.B. wässrig-methanolischer, Lösung eingesetzt wird; Kaliumhexacyanoferrat-(III) in wässriger Lösung; 1,2-Diiodethan oder Azodicarbonsäuredimethylester oder -diethylester, welche in Wasser oder in einer Mischung bestehend aus Wasser und einem mit Wasser mischbaren Alkohol, z.B. Methanol, zur Reaktion gebracht werden. Die Oxidation wird vorzugsweise bei Raumtemperatur ausgeführt.

Eine Mercaptoschutzgruppe, die unter Reaktionsbedingungen abgespalten werden kann, die für Verfahren b) geeignet ist, ist z.B. Acetylaminomethyl. Im Falle der Acetylaminomethyl-Schutzgruppe wird Verfahren b) vorteilhafterweise in Essigsäure, der ein wenig Salzsäure zugesetzt ist, und unter Verwendung von

Iod als Oxidationsmittel durchgeführt, z.B. wie im Beispielteil beschrieben.

Verfahren c: Die in Frage kommenden funktionellen Gruppen, deren Schutz und Wiederfreisetzung durch Abspaltung der Schutzgruppen sind bei Verfahren a) beschrieben. Verfahren c) umfasst ausdrücklich auch die Abspaltung von Schutzgruppen, die an einen Träger gebunden sind, welcher z.B. bei der Merrifield-Synthese verwendet werden kann. In diesem Fall umfasst Verfahren c) also die Abspaltung des Peptids oder Peptidamids vom Träger.

Verfahren d: Für die Transformation geeignete Wirtszellen sind beispielsweise Mikroorganismen, wie Hefen, z.B. Saccharomyces cerevisiae, und insbesondere Bakterienstämme, die über kein Restriktions- oder Modifikationsenzym verfügen, vor allem Stämme von Escherichia coli, z.B. E. coli X1776, E. coli HB101, E. coli W3110, E. coli HB101/LM1035, E. coli JA221 (J. Clarke und J. Carbon, J.Mol.Biol. 120, 517 [1978]) oder E. coli K12 Stamm 294, Bacillus subtilis, Bacillus stearothermophilus, Pseudomonas, Haemophilus, Streptococcus und andere, ferner Zellen höherer Organismen, insbesondere etablierte humane oder tierische Zellinien. Bevorzugte Wirtszellen sind die genannten Stämme von E. coli, insbesondere E. coli HB101, und E. coli JA221.

Grundsätzlich sind alle Vektoren geeignet, welche die erfindungsgemässen heterologen, für die erfindungsgemässen Aminosäuresequenzen kodierenden DNA-Sequenzen in dem gewählten Wirt replizieren und exprimieren.

Beispiele für Vektoren, die zur Expression eines CGRP-Gens in einem E. coli-Stamm geeignet sind, sind Bacteriophagen, z.B. Derivate des Bacteriophagen λ, oder vorzugsweise Plasmide, wie insbesondere das Plasmid colE1 und seine Derivate, z.B. pMB9, pSF2124, pBR317 oder pBR322. Die bevorzugten Vektoren der vorliegenden Erfindung leiten sich von Plasmid pBR322 ab. Geeignete Vektoren enthalten ein vollständiges Replicon und ein Markierungsgen, welches die Selektion und Identifizierung der mit den Expressionsplasmiden transformierten Mikroorganismen auf Grund eines phänotypischen Merkmals ermöglicht. Geeignete Markierungsgene verleihen dem Mikrooganismus beispielsweise Resistenz gegenüber Schwermetallen, Antibiotika und dergleichen. Weiterhin enthalten bevorzugte Vektoren der vorliegenden Erfindung ausserhalb der Replicon- und Markierungsgen-Regionen Erkennungssequenzen für Restriktionsendonucleasen, so dass an diesen Stellen die für die erfindungsgemässen Aminosäuresequenzen kodierende DNA-Sequenz und gegebenenfalls die Expressions-Kontrollsequenz eingefügt werden können. Der bevorzugte Vektor, das Plasmid pBR322, enthält ein intaktes Replicon, gegen Tetracyclin und Ampicillin Resistenz verleihende Markierungsgene ($tet^R$ und $amp^R$) und eine Reihe von nur einmal vorkommenden Erkennungssequenzen für Restriktionsendonucleasen, z.B. PstI (schneidet im $amp^R$-Gen, das $tet^R$-Gen bleibt intakt), BamHI, HindIII, SalI (schneiden alle im $tet^R$-Gen, das $amp^R$-Gen bleibt intakt), NruI und EcoRI.

Mehrere Expressionskontrollsequenzen können zur Regulation der CGRP-Expression eingesetzt werden. Insbesondere werden Expressionskontrollsequenzenstark exprimierter Gene der zu transformierenden Wirtszelle verwendet. Im Falle von pBR322 als Hybridvektor und E. coli als Wirtsmikroorganismus sind beispielsweise die Expressionskontrollsequenzen (welche unter anderem den Promotor und die ribosomale Bindungsstelle enthalten) des Lactoseoperons, Tryptophanoperons, Arabinoseoperons und dergleichen, des β-Lactamasegens, die entsprechenden Sequenzen des Phage λN-Gens oder des Phage fd-Schichtproteingens und andere geeignet. Während der Promotor des β-Lactamasegens (β-lac-Gen) bereits im Plasmid pBR322 enthalten ist, müssen die übrigen Expressionskontrollsequenzen in das Plasmid eingeführt werden. Bevorzugt als Expressionskontrollsequenz in der vorliegenden Erfindung ist diejenige des Tryptophanoperons (trp po).

Zur Replikation und Expression in Hefe geeignete Vektoren enthalten einen Hefe-Replikationsstart und einen selektiven genetischen Marker für Hefe. Hybridvektoren, die einen Hefe-Replikationsstart enthalten, z.B. das chromosomale autonom replizierende Segment (ars), bleiben nach der Transformation innerhalb der Hefezelle extrachromosomal erhalten und werden bei der Mitose autonom repliziert. Ferner können Hybridvektoren, die der Hefe-2μ-Plasmid-DNA homologe Sequenzen enthalten, verwendet werden. Solche Hybridvektoren werden durch Rekombination innerhalb der Zelle bereits vorhandenen 2μ-Plasmiden einverleibt oder replizieren autonom. 2μ-Sequenzen sind besonders für Plasmide mit grosser Transformationshäufigkeit geeignet und gestatten eine hohe Kopienzahl. Geeignete Markierungsgene für Hefe sind vor allem solche, die dem Wirt antibiotische Resistenz verleihen oder, im Fall von auxotrophen Hefemutanten, Gene, die Wirtsdefekte komplementieren. Entsprechende Gene verleihen z.B. Resistenz gegen das Antibiotikum Cycloheximid oder sorgen für Prototrophie in einer auxotrophen Hefemutante, z.B. das URA3-, LEU2-, HIS3- oder vor allem das TRP1-Gen. Vorzugsweise enthalten Hefe-Hybridvektoren weiterhin einen Replikationsstart und ein Markierungsgen für einen bakteriellen Wirt, insbesondere E. coli, damit die Konstruktion und die Klonierung der Hybridvektoren und ihrer Vorstufen in einem bakteriellen Wirt erfolgen kann. Für die Expression in Hefe geeignete Expressionskontrollsequenzen sind beispielsweise diejenigen des TRP1-, ADHI-, ADHII-, PHO3- oder PHO5-Gens, ferner im glykolytischen Abbau involvierte Promoter, z.B. der PGK-und der GAPDH-Promoter.

Insbesondere geeignet sind zur Replikation und phänotypischen Selektion befähigte Expressionsvektoren, welche eine Expressionskontrollsequenz und eine für eine der erfindungsgemässen Aninosäuresequenzen kodierende DNA-Sequenz enthalten, wobei besagte DNA-Sequenz mitsamt Transkriptionsstartsignal und -terminationssignal sowie Translationsstartsignal und -stopsignal in besagtem Expressionsplasmid unter Regulation besagter Expressionskontrollsequenz so angeordnet ist, dass in einer mit besagtem Expressionsplasmid transformierten Wirtszelle die betreffende erfindungsgemässe Aminosäuresequenz exprimiert wird.

Um eine effektive Expression zu erreichen, muss das für eine der erfindungsgemässen Aminosäuresequenzen kodierende Gen richtig (in "Phase") mit der Expressionskontrollsequenz angeordnet sein. Es ist vorteilhaft, die Expressionskontrollsequenz in den Bereich zwischen dem Haupt-mRNA-Start und dem ATG der Genkodiersequenz, welche natürlich mit der Expressionskontrollsequenz verknüpft ist (z.B. die $\beta$-lac-Kodiersequenz bei Verwendung des $\beta$-lac-Promotors), mit dem für die betreffende Aminosäuresequenz kodierenden Gen, welches vorzugsweise sein eigenes Translationsstartsignal (ATG) und Translationsstopsignal (z.B. TAG) mitbringt, zu verknüpfen. Dadurch wird eine effektive Transkription und Translation gewährleistet.

Beispielsweise wird ein Vektor, insbesondere pBR322, mit einer Restriktionsendonuclease geschnitten und, gegebenenfalls nach Modifikation des so gebildeten linearisierten Vektors, eine mit entsprechenden Restriktionsenden versehene Expressionskontrollsequenz eingeführt. Die Expressionskontrollsequenz enthält am 3'-Ende (in Translationsrichtung) die Erkennungssequenz einer Restriktionsendonuclease, so dass der die Expressionskontrollsequenz bereits enthaltende Vektor mit besagtem Restriktionsenzym verdaut und das mit passenden Enden versehene, für die betreffende erfindungsgemässe Aminosäuresequenz kodierende Gen eingesetzt werden kann. Dabei entsteht ein Gemisch von zwei Hybridplasmiden, welche das Gen in richtiger bzw. in falscher Orientierung enthalten. Vorteilhaft ist es, den die Expressionskontrollsequenz bereits enthaltenden Vektor noch mit einer zweiten Restriktionsendonuclease innerhalb der Vektor-DNA zu spalten und in das entstandene Vektor-Fragment das mit richtigen Enden versehene, für die betreffende erfindungsgemässe Aminosäuresequenz kodierende Gen einzusetzen. Alle Operationen am Vektor erfolgen vorzugsweise in einer Weise, dass die Funktion des Replicons und zumindest eines Markierungsgens nicht beeinträchtigt wird.

In einer bevorzugten Ausführungsform der vorliegenden Erfindung wird ein von pBR322 abgeleiteter Vektor, welcher eine Expressionskontrollsequenz, insbesondere diejenige vom Tryptophan-Operon (trp po), enthält, die am 3'-Ende (zwischen dem Haupt-mRNA-Start und dem ersten ATG) die Erkennungssequenz für eine, vorzugsweise kohäsive Enden bildende, Restriktionsendonuclease, z.B. EcoRI, trägt, mit der genannten Restriktionsendonuclease und im Vektor-DNA-Teil mit einer zweiten Restriktionsendonuclease, welche flache oder bevorzugt kohäsive Enden bildet, z.B. BamHI, verdaut, wonach der so linearisierte Vektor mit dem entsprechende Enden aufweisenden Gen (z.B. mit einem EcoRI-Ende vor dem ATG-Start und einem BamHI-Ende nach dem Translationsstop-Codon), das für eine der erfindungsgemässen Aminosäuresequenzen kodiert, verknüpft wird. Die Verknüpfung erfolgt in bekannter Weise durch Paarung der komplementären (kohäsiven) Enden und Ligierung, z.B. mit $T^4$-DNA-Ligase.

Das über den mRNA-Weg, aus genomischer DNA oder synthetisch gewonnene, mit entsprechenden kohäsiven (insbesondere EcoRI- und BamHI-) Enden versehene CGRP-Gen kann vor dem Einbringen in ein Expressionsplasmid auch in einen Vektor, z.B. pBR322, kloniert werden, um grössere Mengen an CGRP-Gen, beispielsweise für die Sequenzanalyse, zu gewinnen. Die Isolierung der Klone, welche das Hybridplasmid enthalten, wird beispielsweise mit einer CGRP-spezifischen, radioaktiv-markierten Oligodesoxynucleotid-Probe durchgeführt. Die Charakterisierung des CGRP-Gens erfolgt beispielsweise nach dem Verfahren von Maxam und Gilbert [A.M. Maxam und W. Gilbert, Proc. Natl. Acad. Sci. USA 74, 560 (1977); siehe auch Meth. Enzym. 65, 499 (1980)].

Die Transformation der Wirtszellen mit den erfindungsgemässen Expressionsplasmiden erfolgt beispielsweise wie in der Literatur beschrieben, so für S. cerevisiae [A. Hinnen et al., Proc. Natl. Acad. Sci. USA 75, 1929 (1978)],
B. subtilis [Anagnostopoulus et al., J. Bacteriol. 81, 741 (1961)] und E. coli [M. Handel et al., J. Mol. Biol. 53, 159 (1970)]. Die Isolierung der transformierten Wirtszellen erfolgt vorteilhaft aus einem selektiven Nährmedium, dem das Biocid zugesetzt wird, gegen welches das im Expressionsplasmid enthaltene Markierungs-Gen Resistenz verleiht. Wenn, wie bevorzugt, die Expressionsplasmide das $amp^R$-Gen enthalten, wird dem Nährmedium demgemäss Ampicillin zugesetzt. Zellen, welche das Expressionsplasmid nicht enthalten, werden in einem solchen Medium abgetötet.

Die Erfindung betrifft ebenfalls die auf dem genannten Weg erhältlichen transformierten Wirtszellen, die zur Produktion der erfindungsgemässen Peptide fähig sind.

Die Kultivierung der erfindungsgemässen transformierten Wirtszellen gemäss Verfahren d) erfolgt in an sich bekannter Weise. So können für die Kultivierung der erfindungsgemässen, transformierten Wirtsmikroorganismen verschiedene Kohlenstoffquellen verwendet werden. Beispiele bevorzugter Kohlenstoffquellen sind assimilierbare Kohlenhydrate, wie Glucose, Maltose, Mannit oder Lactose, oder ein Acetat, das entweder allein oder in geeigneten Gemischen verwendet werden kann. Geeignete Stickstoffquellen sind beispielsweise Aminosäuren, wie Casaminosäuren, Peptide und Proteine und ihre Abbauprodukte, wie Trypton, Pepton oder Fleischextrakte; weiterhin Hefeextrakte, Malzextrakt, wie auch Ammoniumsalze, z.B. Ammoniumchlorid, -sulfat oder -nitrat, die entweder allein oder in geeigneten Mischungen verwendet werden können. Anorganische Salze, die ebenfalls verwendet werden können, sind z.B. Sulfate, Chloride, Phosphate und Carbonate von Natrium, Kalium, Magnesium und Calcium.

Weiterhin enthält das Kulturmedium z.B. wachstumsfördernde Substanzen, wie Spurenelemente, z.B. Eisen, Zink und Mangan, und vorzugsweise Substanzen, die einen Selektionsdruck ausüben und das Wachstum von Zellen, die das Expressionsplasmid verloren haben, verhindern. So wird dem Medium beispielsweise Ampicillin zugesetzt, wenn das Expressionsplasmid ein $amp^R$-Gen enthält. Ein solcher Zusatz von antibiotisch wirksamen Substanzen bewirkt auch, dass kontaminierende, Antibiotika-empfindliche Mikroorganismen abgetötet werden.

Die Kultivierung erfolgt nach an sich bekannten Verfahren. Die Kultivierungsbedingungen, wie Temperatur, pH-Wert des Mediums und Fermentationszeit, werden so ausgewählt, dass maximale Titer der erfindungsgemässen Peptide erhalten werden. So wird ein E. coli-oder ein Hefe-Stamm bevorzugt unter aeroben Bedingungen in submerser Kultur unter Schütteln oder Rühren bei einer Temperatur von etwas 20 bis 40 ° C, vorzugsweise etwa 30 ° C, und einem pH-Wert von 4 bis 9, vorzugsweise bei pH 7, während etwa 4 bis 20 h, vorzugsweise 8 bis 12 h, kultiviert. Dabei sammelt sich das Expressionsprodukt (CGRP) intrazellulär an.

Das N-terminale Methionin kann in bestimmten Wirtszellen posttranslational abgespalten werden. Ausserdem kann in bestimmten Wirtszellen die N-terminale Aminosäure acetyliert werden.

Wenn die Zelldichte einen ausreichenden Wert erreicht hat, wird die Kultivierung abgebrochen und das erfindungsgemässe Peptid aus den Zellen des Mikroorganismus freigesetzt. Zu diesem Zweck werden die Zellen zerstört, z.B. durch Behandlung mit einem Detergens, wie SDS oder Triton, oder mit Lysozym oder einem gleichartig wirkenden Enzym lysiert. Alternativ oder zusätzlich kann man mechanische Kräfte, wie Scherkräfte (z.B. X-Presse, French-Presse, Dyno-Mill) oder Schütteln mit Glasperlen oder Aluminiumoxid, oder abwechselndes Einfrieren, z.B. in flüssigem Stickstoff, und Auftauen, z.B. auf 30 ° bis 40 ° C, sowie Ultraschall zum Brechen der Zellen anwenden. Die resultierende Mischung, die Proteine, Nucleinsäuren und andere Zellbestandteile enthält, wird nach der Zentrifugation in an sich bekannter Weise an Proteinen, inklusive den erfindungsgemässen Peptiden, angereichert. So wird z.B. der grösste Teil der Nicht-Protein-Bestandteile durch Polyethylenimin-Behandlung abgetrennt und die Proteine einschliesslich der erfindungsgemässen Peptide z.B. durch Sättigen der Lösung mit Ammoniumsulfat oder mit anderen Salzen ausgefällt. Bakterielle Proteine können auch mittels Ansäuern mit Essigsäure (z.B. 0,1 %, pH 4-5) ausgefällt werden. Eine weitere Anreicherung an den erfindungsgemässen Peptiden kann durch Extraktion des essigsauren Ueberstandes mit n-Butanol erreicht werden. Weitere Reinigungsschritte umfassen beispielsweise Gelelektrophorese, chromatographische Verfahren, wie Ionenaustauschchromatographie, Grössen-Exklusionschromatographie, HPLC, Umkehrphasen-HPLC und dergleichen, Auftrennung der Mischungsbestandteile nach Molekülgrössen mittels einer geeigneten Sephadex®-Säule, Dialyse, Affinitätschromatographie, z.B. Antikörper-, insbesondere monoklonale Antikörper-Affinitätschromatoraphie, und weitere, insbesondere aus der Literatur, bekannte Verfahren.

Beispielsweise kann man zur Isolierung eines exprimierten erfindungsgemässen Peptids folgendermassen vorgehen:
Abtrennung der Zellen aus der Kulturlösung mittels Zentrifugation;
Herstellung eines Rohextrakts durch Zerstören der Zellen, z.B. durch Behandlung mit einem lysierenden Enzym und/oder abwechselndem Einfrieren und Wiederauftauen;
Abzentrifugieren der unlöslichen Bestandteile;
Ausfällen der DNA durch Zugabe von Polyethylenimin;
Fällung der Proteine einschliesslich der erfindungsgemässen Peptide durch Ammoniumsulfat;
Affinitätschromatographie des gelösten Niederschlags an einer monoklonalen Anti-CGRP-Antikörper-Säule;
Entsalzung der so gewonnen Lösung mittels Dialyse oder Chromatographie an Sephadex G25.

Alternativ können nach Abtrennen der DNA die bakteriellen Proteine mittels 0,1%iger Essigsäure ausgefällt werden, aus dem sauren Ueberstand das betreffende erfindungsgemässe Peptid mit n-Butanol extrahiert oder der saure Ueberstand direkt der Ionenaustausch-Chromatographie (z.B. an Carboxymethylcellulose) unterworfen werden. Weitere Reinigungsschritte schliessen Gelfiltration an Sephadex G50 (oder

G75), und Umkehrphasen-HPLC ein. Entsalzung erfolgt wieder an Sephadex G25.

Zum Nachweis der erfindungsgemässen Peptide kann ein Test mit Anti-CGRP-Antikörpern (z.B. aus Kaninchen erhältliche Antikörper oder aus Hybridomazellen erhältliche monoklonale Antikörper) herangezogen werden.

Verfahren e: Eine Aminosäure $AS^{\Omega}$, die enzymatisch zur $NH_2$-Gruppe der terminalen Amidgruppe des gewünschten Peptidamids abgebaut werden kann, ist z.B. Glycin. Ein geeignetes Enzym kann in diesem Falle aus Hypophysen, vorzugsweise Schweinehypophysen gewonnen werden, wie beschrieben in A.F. Bradbury, M.D.A. Finnie und D.G. Smyth, Nature 298, 686-688 (1982).

Eine Aminosäure $AS^{\Omega}$, die durch Ammoniak in Gegenwart eines geeigneten Enzyms substituiert werden kann, ist z.B. Tyrosin. Ein geeignetes Enzym ist in diesem Falle z.B. Carboxypeptidase Y (Carlsberg Biotechnology Ltd., Kopenhagen, Dänemark), vgl. z.B. Klaus Breddam et al., Carlsberg Res. Commun. 46, 121-128 (1981).

Zusatzoperationen: Salze von Verbindungen mit der (Teil-)Sequenz der Formel I mit salzbildenden Gruppen können in an sich bekannter Weise hergestellt werden. So kann man Salze von Verbindungen mit der Teilsequenz der Formel I, die mehr saure als basische Gruppen aufweisen, z.B. durch Behandeln mit Metallverbindungen, wie Alkalimetallsalzen von geeigneten organischen Carbonsäuren, z.B. dem Natriumsalz der $\alpha$-Aethyl-capronsäure, oder mit anorganischen Alkali- oder Erdalkalimetallsalzen, z.B. Natriumhydrogencarbonat, oder mit Ammoniak oder einem geeigneten organischen Amin bilden, wobei man vorzugsweise stöchiometrische Mengen oder nur einen kleinen Ueberschuss des salzbildenden Mittels verwendet. Säureadditionssalze von Verbindungen mit der Sequenz der Formel I erhält man in üblicher Weise, z.B. durch Behandeln mit einer Säure oder einem geeigneten Anionenaustauschreagens. Innere Salze von Verbindungen mit der Teilsequenz der Formel I, welche z.B. eine freie Carboxylgruppe und eine freie Aminogruppe enthalten, können z.B. durch Neutralisieren von Salzen, wie Säureadditionssalzen, auf den isoelektrischen Punkt, z.B. mit schwachen Basen, oder durch Behandeln mit flüssigen Ionenaustauschern gebildet werden.

Salze können in üblicher Weise in die freien Verbindungen übergeführt werden, Metall- und Ammoniumsalze z.B. durch Behandeln mit geeigneten Säuren, und Säureadditonssalze z.B. durch Behandeln mit einem geeigneten basischen Mittel.

Die Erfindung betrifft auch DNA-Sequenzen, die für die Aminosäuresequenzen der erfindungsgemässen Peptide kodieren.

Die erfindungsgemässen DNA-Sequenzen enthalten als Teilsequenz einer grösseren Sequenz eine Sequenz der Formel IV,

```
d(GCETGYAAYACXGCEACXTGYGTXACXCAYNGKYTZGCEGGXYTZYTZQRSNGK
  AlaCysAsnThrAlaThrCysValThrHisArgLeuAlaGlyLeuLeuSerArg
                                                                  (IV)
  QRSGGXGGXATGGTXAAMQRSAAYTTLGTXCCXACXAAYGTXGGXQRSAAMGCETTL)
  SerGlyGlyMetValLysSerAsnPheValProThrAsnValGlySerLysAlaPhe
```

worin nur die Desoxynukleotidsequenz des kodierenden Stranges, beginnend mit dem 5'-Ende, und darunter die durch die jeweiligen Tripletts kodierten Aminosäuren angegeben sind und worin

A = Adenosyl,
T = Thymidyl,
G = Guanosyl,
C = Cytidyl,
E = A, T, C oder G,
K = A, T, C oder G, wenn N = C, oder A oder G, wenn N = A,
L = T oder C,
M = A oder G,
N = A oder C,
Q = T oder A,
R,S: R = C und S = A, T, C oder G, wenn Q = T; oder R = G und S = T oder C, wenn Q = A,
X = A, T, C oder G,

Y = T oder C und
Z = A, T, C oder G, wenn Y = C, oder A oder G, wenn Y = T,
bedeuten, wobei das vor der Klammer stehende "d" bedeutet, dass die in der Klammer genannten Nucleoside 2-Desoxyribo-nucleoside sind.

Die erfindungsgemässen DNA-Sequenzen enthalten vorzugsweise nicht mehr als 290, hauptsächlich nicht mehr als 230, insbesondere nicht mehr als 200 und vorzugsweise nicht mehr als 150, z.B. 136, Desoxynukleotide im kodierenden Strang.

Zweckmässigerweise werden die Enden der erfindunsgemässen DNA-Sequenzen bereits bei ihrer Herstellung mit Resten versehen, die einen Einbau in Vektoren ermöglichen, z.B. mit Erkennungssequenzen für Restriktionsendonucleasen, mit dem Startcodon ATG und einem Stoppcodon, z.B. TAG. Falls die spätere Herstellung von Peptidamiden beabsichtigt ist, kann man die DNA Sequenz z.B. mit einem Codon versehen, das für eine Aminosäure kodiert, die später enzymatisch so abgebaut werden kann, dass daraus die $NH_2$-Gruppe der terminalen Amidgruppe des Peptidamids resultiert. Eine in diesem Sinne abbaubare Aminosäure ist z.B. Glycin. Alternativ kann man zur späteren Herstellung von Peptidamiden die DNA-Sequenz mit einem Codon versehen, das für eine Aminosäure, wie insbesondere Tyrosin, kodiert, die durch Ammoniak in Gegenwart eines geeigneten Enzyms, wie hauptsächlich einer geeigneten Exopeptidase, z.B. Carboxypeptidase Y substituiert werden kann

In erster Linie betrifft die Erfindung die obengenannten DNA-Sequenzen, die von E. coli bevorzugte und für die Aminosäuren der erfindungsgemässen Peptide kodierende Tripletts enthalten.

Solche Tripletts sind insbesondere
für

| | |
|---|---|
| Alanin (Ala) | GCT |
| Arginin (Arg) | CGT |
| Glycin (Gly) | GGT |
| Cystein (Cys) | TGC |
| Valin (Val) | GTT |
| Leucin (Leu) | CTG |
| Serin (Ser) | TCT |
| Threonin (Thr) | ACC |
| Phenylalanin (Phe) | TTC |
| Tyrosin (Tyr) | TAC |
| Methionin (Met) | ATG |
| Asparaginsäure (Asp) | GAC |
| Glutaminsäure (Glu) | GAA |
| Lysin (Lys) | AAA |
| Isoleucin (Ile) | ATC |
| Histidin (His) | CAC |
| Prolin (Pro) | CCG |
| Glutamin (Gln) | CAG |
| Asparagin (Asn) | AAC |

Bevorzugtes Stopsignal (NON) ist das Codon TAG.

Bevorzugt sind DNA-Sequenzen mit bis zu 200, z.B. 136, Desoxynukleotiden im kodierenden Strang, die die folgende DNA-Sequenz enthalten, wobei nur der kodierende Strang, beginnend mit dem 5'-Ende, angegeben ist und zum besseren Verständnis auch die Aminosäuren, für die jedes Triplett kodiert, genannt sind:

```
  AlaCysAsnThrAlaThrCysValThrHisArgLeuAlaGlyLeuLeuSerArg
d(GCCTGCAACACTGCCACCTGTGTGACTCATCGGCTGGCAGGCTTGCTGAGCAGA
                                                                      (IVa)
  SerGlyGlyMetValLysSerAsnPheValProThrAsnValGlySerLysAlaPhe
  TCAGGGGGCATGGTGAAGAGCAACTTCGTGCCCACCAATGTGGGTTCCAAAGCCTTT)
```

Besonders bevorzugt für die Expression in E. coli sind DNA-Sequenzen mit bis zu 200, z.B. 136, Desoxynukleotiden im kodierenden Strang, die die folgende DNA-Sequenz enthalten, wobei nur der

kodierende Strang, beginnend mit dem 5'-Ende, angegeben ist und zum besseren Verständnis auch die Aminosäuren, für die jedes Triplett kodiert, genannt sind:

```
  AlaCysAsnThrAlaThrCysValThrHisArgLeuAlaGlyLeuLeuSerArg
d(GCTTGCAACACCGCTACCTGCGTTACCCACCGTCTGGCTGGTCTGCTGTCTCGT
                                                              (IVb)
  SerGlyGlyMetValLysSerAsnPheValProThrAsnValGlySerLysAlaPhe
  TCTGGTGGTATGGTTAAATCTAACTTCGTTCCGACCAACGTTGGTTCTAAAGCTTTC)
```

Die erfindungsgemässen DNA-Sequenzen werden nach an sich bekannten Verfahren hergestellt.

Methoden der Synthese von DNA sind von S.A. Narang [Tetrahedron 39, 3 (1983)] zusammenfassend dargestellt worden. Die bekannten Synthesetechniken erlauben die Herstellung von Polydesoxy-nukleotiden mit einer Länge von gegen 20 Basen in guter Ausbeute, hoher Reinheit und in verhältnismässig kurzer Zeit. Geeignet geschützte Desoxynukleotide werden durch die Phosphodiestermethode [K.L. Agarwal et al., Angew. Chem. 84, 489 (1972)], die noch effizientere Phosphotriestermethode [C.B. Reese, Tetrahedron 34, 3143 (1972)] oder Phosphit-Triestermethode [R.L. Letsinger and W.B. Lunsford, J. Am. Chem. Soc. 98, 3655 (1976)] miteinander verknüpft. Eine Vereinfachung der Synthese der Oligo- und Polydesoxynukleotide wird mit der Festphasen-Methode ermöglicht, bei der die Desoxynukleotidketten an ein geeignetes Polymer gebunden sind. K. Itakura et al. [J. Am. Chem. Soc. 103, 706 (1981)] verwenden bei der Festphasen-Synthese anstelle einzelner Desoxynukleotide nach der Phosphotriester-Methode geknüpfte Tridesoxynukleotide, die so in kurzer Zeit und guten Ausbeuten beispielsweise zu einem Polydesoxynukleotid mit 31 Basen kondensiert werden können. Die eigentliche doppelsträngige DNA kann aus chemisch hergestellten kurzen Segmenten enzymatisch aufgebaut werden. H.G. Khorana et al. [J. Biol. Chem. 251, 565 (1976)] verwenden dazu überlappende Polydesoxynukleotid-Sequenzen aus beiden DNA-Strängen, die durch Basen-Paarung in richtiger Anordnung zusammengehalten und dann durch das Enzym DNA-Ligase chemisch verknüpft werden. Eine weitere Möglichkeit besteht darin, je eine Polydesoxynukleotid-Sequenz aus den beiden DNA-Strängen mit einem kurzen überlappenden Segment in Gegenwart der vier benötigten Desoxynukleosid-Triphosphate mit einer DNA-Polymerase, z.B. DNA-Polymerase I, Klenow-Fragment der Polymerase I, $T^4$ DNA-Polymerase, oder mit AMV (avian myeloblastosis virus) reverse Transcriptase, zu inkubieren. Dabei werden durch Basenpaarung die beiden Polydesoxynukleotid-Sequenzen in der richtigen Anordnung zusammengehalten und durch das Enzym mit den benötigten Desoxynukleotiden zu einer vollständigen doppelsträngigen DNA ergänzt [S.A. Narang et al., Anal. Biochem. 121, 356 (1982)].

K. Itakura et al. [J. Biol. Chem. 257, 9226 (1982)] beschreiben, wie basierend auf diesem Prinzip in Gegenwart von DNA-Polymerase I (Klenow-Fragment) aus 4 chemisch synthetisierten Fragmenten einer Länge von 39 bis 42 Basen ein 132 Basen-Paare langes Segment des humanen Leukozyten-Interferon $\alpha_2$-Gens aufgebaut werden kann, wobei 40 % Einsparung an chemischer Synthese gegenüber der oben beschriebenen Methode, bei der nur Ligase verwendet wird, erzielt werden.

Das in dieser Anmeldung beschriebene Verfahren zur Synthese der erfindungsgemässen DNA-Sequenzen ist dadurch gekennzeichnet, dass

α) ein geeignet geschütztes Desoxynukleosid an einen festen Träger gebunden wird,

β) geeignet geschützte Di-, Tri- oder Tetra-desoxynukleotide nach der Phosphotriester- oder Phosphit-Methode hergestellt werden,

γ) ein an den Träger gebundenes Desoxynukleosid oder Oligodesoxynukleotid mit geeignet geschützten Mono- oder (gemäss β) hergestellten) Di-, Tri- oder Tetra-desoxynukleotiden nach der Phosphotriester- oder der Phosphit-Methode verknüpft wird,

δ) nach γ) erhältliche, an Träger gebundene Oligodesoxynukleotide mit der gewünschten Basenanzahl vom Träger abgespalten, gegebenenfalls gereinigt, von Schutzgruppen befreit und die freien 5'-terminalen Hydroxygruppen phosphoryliert werden,

ε) 2 gemäss δ) erhaltene Oligodesoxynukleotide aus dem kodierenden und aus dem komplementären Strang mit je mindestens 3, vorzugsweise 8 bis 15, überlappenden Basenpaaren annelliert, mit einer DNA-Polymerase in Gegenwart der vier Desoxynukleosid-Triphosphate zu einem doppelsträngigen DNA-Segment, welches das CGRP-Gen enthält, ergänzt werden, und mit einer Ligase zum gewünschten Strukturgen verknüpft werden.

Die Synthese der DNA-Sequenz der Formel IV kann demgemäss z.B. durchgeführt werden, indem man zwei Oligodesoxynukleotide, nämlich einem negativen und einem positiven Strang mit ungefähr gleich grosser Anzahl Basen, wobei diese Oligodesoxynukleotide in einem Teilbereich die Fähigkeit zur Ausbildung von Wasserstoffbrückenbindungen zueinander besitzen, durch schrittweises Ankondensieren von Trinucleotidblöcken an eine wachsende Nukleotid-Sequenz nach dem Festphasenverfahren aufbaut, die einzelnen Nukleotid-Ketten vom festen Träger ablöst, vorhandene Schutzgruppen abspaltet, reinigt, z.B. mittels Gel-Elektrophorese, und nach Kinasierung und Hybridisierung zur kompletten DNA-Duplex auffüllt, vorzugsweise mittels DNA-Polymerase I (Klenow Fragment).

Im Schritt $\alpha$) können eine Vielzahl von festen Trägermaterialien, wie Polystyrol verschiedener Vernetzung und Quellfähigkeit, Polyacrylamide, Polyacrylamid-Copolymere, auf anorganisches Material wie Kieselgur, Kieselgel oder Alox aufgezogene Polyamide oder funktionalisierte Silane, verwendet werden. In einer bevorzugten Ausführungsform werden als feste Trägermaterialien quervernetzte Polystyrole eingesetzt, die über "Spacer", wie Alkylen-Gruppen mit 2 bis 12 C-Atomen unterbrochen von 1 bis 5 polaren zweiwertigen funktionellen Gruppen, wie Imino, Oxo, Thio, Oxocarbonyl oder Amidocarbonyl, auf an sich bekannte Weise mit der 5'-OH-Gruppe geeignet geschützter Desoxynukleoside verknüpft werden. Besonders bevorzugt ist die Reaktion von in 5'-Stellung und gegebenenfalls im Basenteil geschützten Nukleosiden der Formel V, worin $R_1$ eine durch Säure abspaltbare Schutzgruppe, wie eine Triarylmethylschutzgruppe, beispielsweise eine 4-Methoxytrityl- oder eine 4,4'-Dimethoxytritylgruppe, oder eine Triniederalkylsilylschutzgruppe, beispielsweise eine tert.-Butyldimethylsilylgruppe, und worin Ba eine gegebenenfalls geschützte Base ausgewählt aus Thymyl, Cytosyl, Adenyl oder Guanyl bedeutet, mit Bernsteinsäureanhydrid, gegebenenfalls in Gegenwart von Basen, wie Pyridin, Triethylamin oder Dimethylaminopyridin, gefolgt von der Reaktion mit aminomethyliertem Polystyrol, das durch 0,5 bis 2 % Divinylbenzol quervernetzt ist, mit Hilfe von den Carbonsäure-Rest aktivierenden Reagentien, vorzugsweise N-Hydroxysuccinimid, oder p-Nitrophenol und wasserentziehenden Mitteln, wie Carbodiimiden, beispielsweise Dicyclohexylcarbodimid (Schema 1). In Schema 1 und nachfolgend ist der Desoxyribose-Rest oft in der gebräuchlichen Kurzform dargestellt.

Die Umsetzung erfolgt in einem inerten nicht-protischen Lösungsmittel, z.B. Pyridin, Tetrahydrofuran, Dioxan, Essigsäureethylester, Chloroform, Methylenchlorid, Dimethylformamid oder Diethylacetamid oder in Mischungen davon, bei Raumtemperatur oder leicht erhöhter oder erniedrigter Temperatur, z.B. in einem Temperaturbereich von ca. -10 °C bis ca. 50 °C, bevorzugt bei Raumtemperatur, die Umsetzung in Gegenwart des wasserentziehenden Mittels auch bei niedrigerer Temperatur, z.B. bei ca. 0 °C.

Schema 1

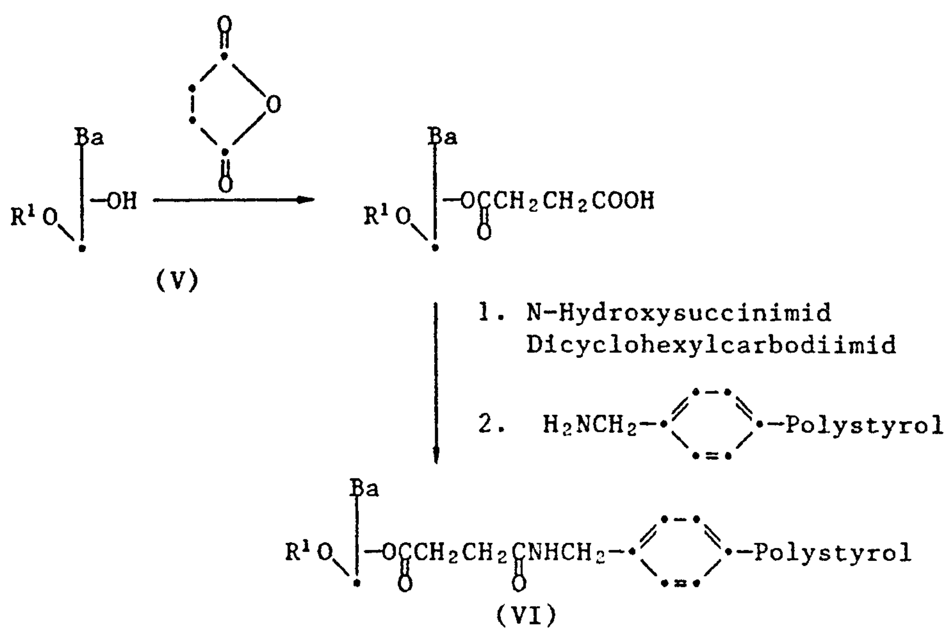


(V)

(VI)

Bei der erfindungsgemässen Herstellung von Di-, Tri- oder Tetranukleotiden gemäss Schritt $\beta$) (Schema 2) werden in 5'-Stellung und gegebenenfalls im Basenteil geschützte Nukleoside der Formel V, worin $R^1$

und Ba die oben angegebenen Bedeutungen haben, mit aktivierten Phosphorestern der Formel VII, worin $X^1$ und $X^2$ unabhängig voneinander Hydroxy oder davon abgeleitete Salze, Halogen, Imidazolyl, 1,2,4-Triazol-1-yl, Tetrazolyl oder 1-Benztriazolyloxy und $X^2$ zusätzlich auch 2-Cyanoethyloxy, 2-Trihalogenethyloxy, 2-Arylsulfonylethyloxy, 2-Niederalkylthioethyloxy, 2-Arylthioethyloxy oder 2-(4-Nitrophenyl)-ethyloxy und $R^2$ eine durch eine Base oder ein Nukleophil, wie Ammoniumhydroxid, Thiophenolat oder ein Arylaldoximat, abspaltbare Schutzgruppe, wie gegebenenfalls durch Halogen, Nitro und/oder Niederalkyl substituiertes Phenyl, Methyl oder gegebenenfalls durch Nitro substituiertes Benzyl, oder eine durch Metallionen abspaltbare Schutzgruppe, wie 8-Chinolyl oder 5-Chloro-8-chinolyl, bedeutet, gegebenenfalls in Gegenwart von wasserentziehenden Mitteln oder in Gegenwart von Basen umgesetzt.

Anschliessend wird eine dermassen gebildete Verbindung der Formel VIII, worin $R^1$, $X^2$ und $R^2$ die oben angegebenen Bedeutungen haben, zuerst gegebenenfalls mit einem 2-substituierten Ethanol umgesetzt, das den Rest $X^2$ in eine Gruppe $OR^3$ umwandelt, worin $R^3$ Cyanoethyl, 2-Trihalogenethyl, 2-Arylsulfonylethyl, 2-Niederalkylthioethyl, 2-Arylthioethyl oder 2-(4-Nitrophenyl)-ethyl bedeutet, und dann die Schutzgruppe $R^1$ abgespalten, und die dermassen hergestellte Verbindung der Formel IX mit einer andern Verbindung der Formel VIII, gegebenenfalls in Gegenwart von wasserentziehenden Mitteln oder in Gegenwart von Basen, zu einem Dinukleotid X umgesetzt (Schema 2). Gegebenenfalls wird eine Verbindung der Formel VIII durch Reaktion mit Basen und Wasser in eine andere Verbindung der Formel VIII, worin $X^2$ Hydroxy oder davon abgeleitete Salze bedeutet, umgewandelt.

Die Umsetzungen erfolgen in einem der oben genannten inerten Lösungsmittel bei Raumtemperatur oder leicht erhöhter oder erniedrigter Temperatur, z.B. bei Raumtemperatur.

Die Abspaltung der Schutzgruppe $R^1$ wird z.B. mit Hilfe von Säuren, wie Mineralsäuren, z.B. Salzsäure oder Schwefelsäure, Carbonsäure, z.B. Esigsäure, Trichloressigsäure oder Ameisensäure, Sulfonsäure, z.B. Methan- oder p-Toluolsulfonsäure, oder insbesondere Lewis-Säure, z.B. Zinkchlorid, Zinkbromid, Aluminiumchlorid, Dialkylaluminiumhalogenid, z.B. Dibutyl- oder Diethylaluminiumchlorid, oder Bortrifluorid, bei 10 °C bis 50 °C, insbesondere bei Raumtemperatur, durchgeführt. Bei Verwendung eines Dialkylaluminiumhalogenids erfolgt die Abspaltung in einem lipophilen Lösungsmittel, insbesondere in Toluol, und bei Verwendung einer anderen der genannten Lewis-Säuren in einem Lösungsmittelgemisch, bestehend aus einem Halogenkohlenwasserstoff, z.B. Methylenchlorid, und einem Niederalkanol, z.B. Ethanol oder Isopropanol.

Schema 2

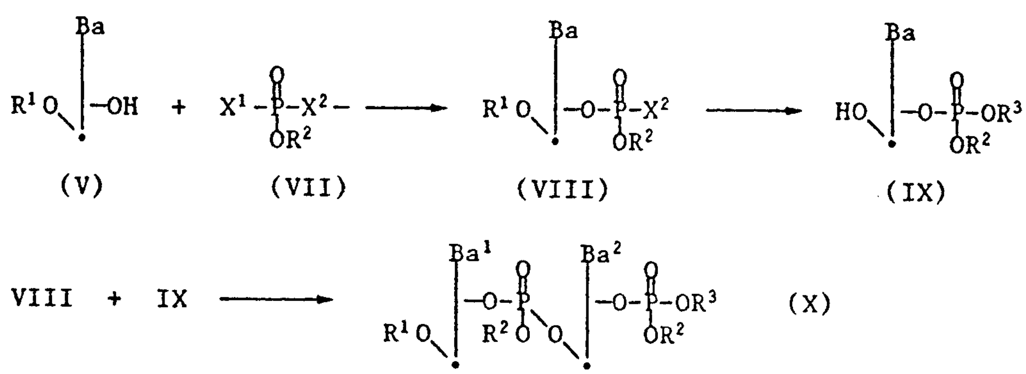

(V) (VII) (VIII) (IX) (X)

Dinukleotide der Formel X können auch durch Reaktion von Nukleosiden der Formel V, worin $R^1$ und Ba die oben angegebenen Bedeutungen haben, mit Phosphiten der Formel VIIA hergestellt werden, worin $X^1$ Halogen, insbesondere Chlor, $X^2$ Halogen, insbesondere Chlor, oder Diniederalkylamino, insbesondere Dimethylamino oder Diisopropylamino, oder Morpholino, Piperidino oder Pyrrolidino, bedeuten und $R^2$ die für VII oben angegebene Bedeutung, insbesondere Methyl, hat, gegebenenfalls in Gegenwart einer geeigneten Base (Schema 3). Die erhältlichen Verbindungen der Formel VIIIA werden einerseits mit einem 2-substituierten Ethanol umgesetzt, das den Rest $X^2$ in eine Gruppe $OR^3$ umwandelt, worin $R^3$ die oben angeführten Bedeutungen hat, dann mit einem Oxidationsmittel, beispielsweise Iod in Gegenwart einer Base, zum Phosphat oxidiert und die Schutzgruppe $R^1$ abgespalten, wobei eine Verbindung der Formel IX entsteht, andererseits mit einer Verbindung der Formel IX umgesetzt, dann mit einem Oxidationsmittel, beispielsweise Iod in Gegenwart einer Base, zu einer Verbindung der Formel X oxidiert.

Schema 3

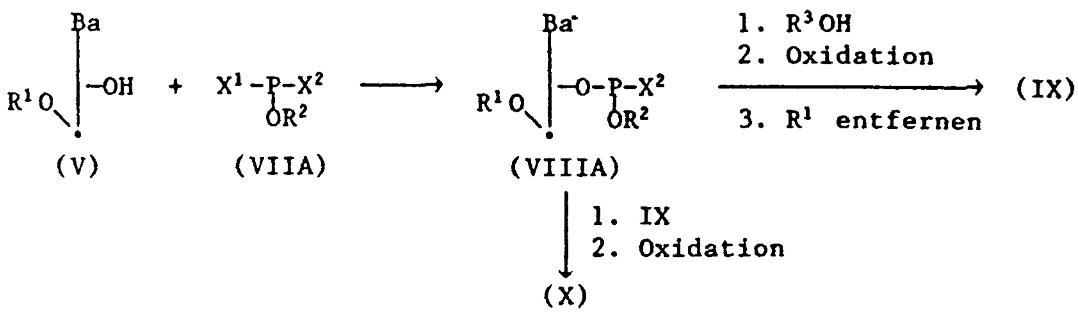

(V) (VIIA) (VIIIA) (IX) (X)

Zur Herstellung von Trinukleotiden wird in Dinukleotiden der Formel X, worin $R^1$, $R^2$ und $R^3$ die oben angegebenen Bedeutungen haben und worin $Ba^1$ und $Ba^2$ unabhängig voneinander Thymyl, Cytosyl, Adenyl oder Guanyl bedeuten, die Schutzgruppe $R^1$ abgespalten und die erhaltene Verbindung mit einer Verbindung der Formel VIII, gegebenenfalls in Gegenwart von wasserentziehenden Mitteln oder in Gegenwart von Basen, oder mit einer Verbindung der Formel VIIIA und nachfolgender Oxidation umgesetzt, wobei eine Verbindung der Formel XI entsteht (Schema 4). Die Abspaltung der Schutzgruppe $R^1$ und die Kondensation zu den Trinukleotiden der Formel XI erfolgt in gleicher Weise, wie bei der Herstellung der Dinukleotide der Formel X beschrieben.

Schema 4

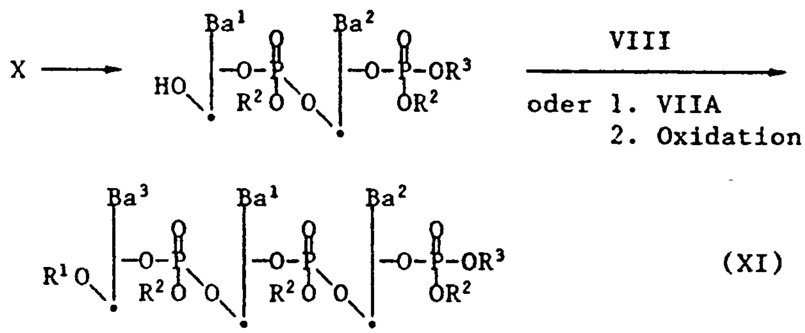

(XI)

Zur Herstellung von Tetranukleotiden werden Trinukleotide der Formel XI so zur Reaktion gebracht, wie oben für Dinukleotide der Formel X beschrieben.

In einer bevorzugten Ausführung wird als Schutzgruppe $R^1$ die 4-Methoxytritylgruppe, als Schutzgruppe $R^2$ eine durch Chlor substituierte Phenylgruppe, insbesondere 2-Chlorphenyl, und als Schutzgruppe $R^3$ die 2-Cyanoethylgruppe verwendet. Bevorzugter Rest $X^1$ und $X^2$ in der Verbindung der Formel VII ist der 1-Benztriazolyloxy-Rest.

Trinukleotide der Formel XI werden vorzugsweise hergestellt, indem in Dinukleotiden der Formel X die Schutzgruppe $R^1$ abgespalten und die erhaltene Verbindung mit Verbindungen der Formel VIII, worin $X^2$ Hydroxyl oder davon abgeleitete Salze bedeutet, in Gegenwart eines wasserentziehenden Mittels umgesetzt wird (Schema 4). Erfindungsgemässe wasserentziehende Mittel sind beispielsweise 2,4,6-Trimethyl- oder Triisopropylbenzolsulfonyl-chlorid, -imidazol, -tetrazol oder -1,2,4-triazol, gegebenenfalls substituiert durch Nitro. Bevorzugtes wasserentziehendes Mittel ist 1-(2,4,6-Trimethylbenzolsulfonyl)-3-nitro-1,2,4-triazol (XII).

Vorzugsweise werden Nukleoside eingesetzt, deren freie Aminogruppe im Basenteil geschützt ist. Bevorzugte Schutzgruppen sind Benzoyl für Adenin, Benzoyl oder 4-Methoxybenzoyl für Cytosin, Isobutyryl oder Diphenylacetyl für Guanin. Thymin wird vorzugsweise ohne Schutzgruppe eingesetzt.

Bei der Herstellung von Oligonukleotiden gemäss Schritt $\gamma$) wird eine an sich bekannte Apparatur mit halbautomatischem oder vollautomatischem, mikroprozessorgesteuertem Zuführungssystem für Lösungsmittel und Reagentien verwendet. In einer gemäss Schritt $\alpha$) hergestellten Verbindung der Formel VI wird

die Schutzgruppe $R^1$, wie oben beschrieben, abgespalten und anschliessend entweder mit einer Verbindung der Formel VIII, oder mit einer Verbindung der Formel VIIIA, oder mit einer Verbindung der Formel X oder XI, in der vorgängig die Schutzgruppe $R^3$ mit Basen abgespalten worden ist (eine Gruppe 2-Cyanoethyl als $R^3$ beispielsweise mit einem Triniederalkylamin, z.B. Triethylamin, in einem der oben genannten inerten Lösungsmittel oder Lösungsmittelgemische bei 10 °C bis 40 °C, insbesondere bei Raumtemperatur), gegebenenfalls in Gegenwart eines wasserentziehenden Mittels oder in Gegenwart einer Base umgesetzt. Anstelle eines Dinukleotids der Formel X oder eines Trinukleotids der Formel XI kann ein gemäss Schritt $\beta$) hergestelltes Tetranukleotid eingesetzt werden. Wird ein Phosphit der Formel VIIIA verwendet, so wird anschliessend mit einem Oxidationsmittel, beispielsweise Iod in Gegenwart einer Base, nachbehandelt. Die dermassen hergestellte Verbindung der Formel XIII, worin $R^1$, $R^2$ und Ba die oben angegebenen Bedeutungen haben und n eine ganze Zahl von 1 bis 4 ist, wird so oft wie erforderlich den für die Verbindung der Formel VI beschriebenen Reaktionsschritten (Abspaltung von $R^1$, Reaktion mit VIII, VIIIA, X, XI oder dem entsprechenden Tetranukleotid, gegebenenfalls mit oxidativer Nachbehandlung) unterworfen, wobei eine Verbindung der Formel XIII entsteht (n = ganze Zahl).

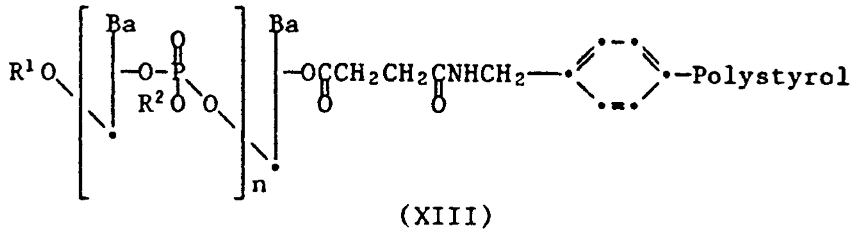

(XIII)

In einer bevorzugten Ausführungsform der Erfindung wird als Schutzgruppe $R^1$ 4-Methoxytrityl verwendet und die Abspaltung mit Zinkbromid in Gegenwart einer CH- oder NH-aciden Verbindung, insbesondere 1,2,4-Triazol oder Tetrazol, durchgeführt. Die Verwendung von z.B. 1,2,4-Triazol bei der Abspaltung der 4-Methoxytrityl-Schutzgruppe ist neu und führt überraschenderweise dazu, dass die Abspaltung schnell, mit hohen Ausbeuten und ohne Nebenreaktionen abläuft. Besonders bevorzugt ist die Verwendung von Zinkbromid und 1,2,4-Triazol in einem molaren Verhältnis zwischen 20:1 und 100:1 in einem Lösungsmittelgemisch bestehend aus einem aprotischen Lösungsmittel und einem Alkohol, beispielsweise Methylenchlorid und 2-Propanol.

In einer bevorzugten Ausführungsform wird eine Verbindung der Formel VI oder der Formel XIII, in der die Schutzgruppe $R^1$ abgespalten worden ist, mit einem Trinukleotid der Formel XI, in dem die Schutzgruppe $R^3$ abgespalten worden ist, in Gegenwart eines wasserentziehenden Mittels umgesetzt, wie beispielsweise 2,4,6-Trimethyl- oder Triisopropylbenzolsulfonyl-chlor, -imidazol, -tetrazol oder -1,2,4-triazol gegebenenfalls substituiert durch Nitro. Besonders bevorzugt ist 1-(2,4,6-Trimethylbenzolsulfonyl)-3-nitro-1,2,4-triazol (XII).

Die besonders bevorzugte Kombination, darin bestehend, dass als Schutzgruppe $R^1$ die 4-Monomethoxytritylgruppe verwendet, zur Abspaltung von $R^1$ Zinkbromid in Gegenwart von 1,2,4-Triazol eingesetzt und als wasserentziehendes Mittel für die Reaktion von entschütztem Oligonukleotid-Polystyrol-Harz der Formel XIII mit einem entschützten Trinukleotid der Formel XI das Triazol der Formel XII verwendet wird, ermöglicht es, dass auch lange Nukleotidketten in kurzer Zeit, in hohen Ausbeuten und mit hoher Reinheit hergestellt werden können.

Zur Abspaltung der Oligodesoxynukleotide vom Träger und zur Entfernung der Schutzgruppen gemäss Schritt $\delta$) werden an sich bekannte Verfahren verwendet. Besonders bevorzugtes Reagens zur Abspaltung vom Träger und zur Entfernung der bevorzugten 2-Chlorphenyl-Schutzgruppe ist ein Arylaldoximat, beispielsweise das 1,1,3,3,-Tetramethylguanidinium-2-nitrobenzaldoximat. Die Reaktion erfolgt in einem der oben genannten inerten Lösungsmittel, dem etwas Wasser beigefügt wird, z.B. in 95%igem Pyridin, bei Raumtemperatur. Anschliessend wird mit wässrigem Ammoniak bei Raumtemperatur oder erhöhter Temperatur, z.B. bei 20 °C bis 70 °C, insbesondere bei 50 °C, umgesetzt.

Zur Ligation der Oligodesoxynukleotide wird gemäss Schritt $\delta$) an der 5'-terminalen Hydroxygruppe ein Phosphatrest eingeführt. Die Einführung des Phosphatrestes (Phosphorylierung) erfolgt in an sich bekannter Weise mit Hilfe von $T^4$ Polynucleotid-Kinase in Gegenwart von ATP.

Wie oben geschildert hergestellte Oligodesoxynukleotide aus dem kodierenden und dem komplementären DNA-Strang enthalten überlappende Sequenzen, bestehend aus mindestens 3, vorzugsweise 8 bis 15

überlappenden Basenpaaren. Solche Oligodesoxynukleotid-Paare werden beim Mischen durch Wasserstoffbrückenbindung zusammengehalten. Die überhängenden, einsträngigen Enden dienen gemäss Schritt $\epsilon$) als Matrix (Templat) für den Aufbau des komplementären Stranges durch eine DNA-Polymerase, z.B. DNA-Polymerase I, Klenow-Fragment der DNA-Polymerase I oder $T^4$ DNA-Polymerase, oder mit AMV reverse Transcriptase, in Gegenwart der vier Desoxynukleosid-Triphosphate (dATP, dCTP, dGTP und TTP). Die bei der Komplementierung entstehenden Duplex-DNAs besitzen flache Enden.

Die gemäss Verfahresschritt $\epsilon$) erhältlichen DNA-Sequenzen enthalten an den Enden Nukleotidsequenzen, die von Restriktionsendonucleasen erkannt und gespalten werden können. Je nach Wahl der Nukleotidsequenzen und dementsprechend der Restriktionsendonucleasen entstehen bei der Spaltung vollständig basengepaarte (flache) Enden ("blunt ends") oder Enden mit einem überhängenden DNA-Strang ("staggered ends").

Die Erfindung betrifft auch pharmazeutische Präparate, die als Wirkstoff eine wirksame Dosis, insbesondere eine zur Behandlung der obengenannten Krankheiten wirksame Dosis, eines der erfindungsgemässen Peptidamide oder ein Salz davon zusammen mit einer signifikanten Menge, d.h. mehr als 50 Gewichtsprozent, vorzugsweise mehr als 95 Gewichtsprozent, insbesondere mehr als 99 Gewichtsprozent, eines pharmazeutischen Trägermaterials enthalten, insbesondere solche Präparate zur intranasalen oder parenteralen, wie intramuskulären oder intravenösen Applikation an Warmblüter, wie in allererster Linie den Menschen.

Die Dosierung des Wirkstoffes hängt von der Warmblüter-Spezies, dem Körpergewicht, Alter und dem individuellen Zustand, der zu behandelnden Krankheit sowie von der Applikationsweise ab.

Die neuen pharmazeutischen Präparate zur parenteralen Applikation enthalten in gebrauchsfertiger Form von etwa 0,001 Gewichtspromille bis etwa 1 Gewichtsprozent, vorzugsweise von etwa 0,005 Gewichtspromille bis etwa 0,1 Gewichtspromille, z.B. 0,01 Gewichtspromille, des Wirkstoffes. Die erfindungsgemässen pharmazeutischen Präparate können z.B. in Dosiseinheitsform, wie Ampullen, vorliegen.

Vorzugsweise verwendet man Lösungen des Wirkstoffes, daneben auch Suspensionen, und zwar insbesondere isotonische wässrige Lösungen oder Suspensionen, wobei diese z.B. bei lyophilisierten Präpraten, welche die Wirksubstanz allein oder zusammen mit einem Trägermaterial, z.B. Mannit, enthalten, vor Gebrauch hergestellt werden können. Insbesondere zur intranasalen Anwendung besonders geeignet sind Suspensionen in Oel. Die pharmazeutischen Präparate können sterilisiert ein und/oder Hilfsstoffe, z.B. Konservier-, Stabilisier-, Netz und/oder Emulgiermittel, Löslichkeitsvermittler, Salze zur Regulierung des osmotischen Druckes und/oder Puffer enthalten und werden in an sich bekannter Weise, z.B. mittels konventioneller Lösungs- oder Lyophilisierungsverfahren, hergestellt. Die genannten Lösungen oder Suspensionen können viskositätserhöhende Stoffe, wie Natrium-Carboxymethylcellulose, Carboxymethylcellulose, Dextran, Polyvinylpyrrolidon oder bevorzugterweise Gelatine, enthalten.

Suspensionen in Oel enthalten als ölige Komponente die für Injektionszwecke gebräuchlichen vegetabilen, synthetischen oder halbsynthetischen Oele. Als solche sind insbesondere flüssige Fettsäureester zu nennen, die als Säurekomponente eine langkettige Fettsäure mit 8-22, besonders 12-22 Kohlenstoffatomen, wie z.B. Laurinsäure, Tridecylsäure, Myristinsäure, Pentadecylsäure, Palmitinsäure, Margarinsäure, Stearinsäure, Arachinsäure, Behensäure oder entsprechende ungesättigte Säuren, wie z.B. Oelsäure, Elaidinsäure, Erucasäure, Brasidinsäure oder Linolsäure, enthalten. Die Alkoholkomponente hat maximal 6 Kohlenstoffatome und ist ein ein- oder mehrwertiger, z.B. ein-, zwei- oder dreiwertiger Alkohol, z.B. Methanol, Ethanol, Propanol, Butanol oder Pentanol oder deren Isomere, vor allem aber Glycol oder Glyzerin. Als Fettsäureester sind daher beispielsweise zu nennen: Ethyloleat, Isopropylmyristat, Isopropylpalmitat, "Labrafil M 2735" (Polyoxyethylenglyzerintrioleat der Firma Gattefossé, Paris), "Miglyol 812" (Triglyzerid gesättiger Fettsäuren der Kettenlänge $C_8$ bis $C_{12}$ der Firma Chemische Werke, Witten/Ruhr, Deutschland), besonders aber vegetabile Oele wie Baumwollsaatöl, Mandelöl, Olivenöl, Ricinusöl, Sesamöl, Sojabohnenöl, vor allem Erdnussöl.

Die Herstellung der Injektionspräparate erfolgt in üblicher Weise unter antimikrobiellen Bedingungen, ebenso das Abfüllen in Ampullen oder Vials sowie das Verschliessen der Behälter.

Die nachfolgenden Beispiele illustrieren die Erfindung. Temperaturen sind in Celsiusgraden angegeben.

Die $R_f$-Werte werden, wenn nicht anders angegeben oder aus dem Zusammenhang ersichtlich, auf Kieselgeldünnschichtplatten in folgendem Lösungsmittelsystem ermittelt:

I: Dichlormethan-Methanol (9:1).

Demgemäss bedeutet z.B. $R_f$ (I) einen im Lösungsmittelsystem I ermittelten $R_f$-Wert.

Abkürzungen

| | |
|---|---|
| abs. = | absolut |
| Acm = | Acetamidomethyl |
| Boc = | tert.Butyloxycarbonyl |
| BSA = | Rinder-Serumalbumin |
| BZL = | Benzyl |
| 2CZ = | 2-Chlor-benzyloxycarbonyl |
| d = | desoxy |
| dest. = | destilliert |
| DMF = | Dimethylformamid |
| DTT = | 1,4-Dithiothreitol (1,4-Dimercapto-2,3-butandiol) |
| EDTA = | Ethylendiamintetraessigsäure |
| EtBr = | Ethidiumbromid |
| FAB = | Fast atom bombardment |
| Fmoc = | 9-Fluorenyl-methyloxycarbonyl |
| h = | Stunde |
| HPLC = | Hochdruckflüssigkeitschromatographie |
| HV = | Hochvakuum |
| iBu = | Isobutyryl |
| M = | molar |
| MBHA = | Mono-(4-methyl)-benzhydrylamin |
| Me = | Methyl |
| Min. = | Minuten |
| mmt = | (Mono-4-methoxy)-trityl |
| MOB = | 4-Methoxy-benzyl |
| MS = | Massenspektroskopie |
| Mtr = | 4-Methoxy-2,3,6-trimethyl-benzolsulfonyl |
| N = | normal |
| OD = | Optische Dichte |
| RG = | Reaktionsgemisch |
| RT = | Raumtemperatur |
| SDS = | Natriumdodecylsulfat |
| tBu = | tertiäres Butyl |
| TNE = | Lösung, die 100 mM NaCl, 50 mM Tris•HCl (pH 7,5) und 5 mM EDTA enthält |
| TOS = | 4-Toluolsulfonyl |
| Tris = | tris-(Hydroxymethyl)-aminomethan |
| Tris•HCl = | Monohydrochlorid von Tris |
| upm = | Umdrehungen pro Minute |
| V = | Volumen |
| XANT = | 9-Xanthenyl |

Beispiel 1: Zu einer Mischung von 0,26 ml 0,1 M Iod in Eisessig, 0,33 ml 50%iger Essigsäure und 16 µl 0,1 N HCl wird eine Lösung von 15 mg (3,8 µMol) Ala-Cys(Acm)-Asn-Thr-Ala-Thr-Cys(Acm)-Val-Thr-His-Arg-Leu-Ala-Gly-Leu-Leu-Ser-Arg-Ser-Gly-Gly-Met-Val-Lys Ser-Asn-Phe-Val-Pro-Thr-Asn-Val-Gly-Ser-Lys-Ala-Phe-$NH_2$ in 2,0 ml 50%iger Essigsäure innerhalb von 5 Min. bei RT unter Rühren zugetropft. Nach 10 Min. bei RT wird durch Zugabe von 40 µl 1 M wässeriger Ascorbinsäurelösung entfärbt und durch Sephadex® G-25 in 50%iger Essigsäure filtriert. Das Eluat wird im Vakuum auf ca. 1 ml eingeengt und einer HPLC-Reinigung wie unten beschrieben unterworfen.

Man erhält CGRP II der Formel III.

```
 S————————————————————S
 |                    |
Ala-Cy-Asn-Thr-Ala-Thr-Cy-Val-Thr-His-Arg-Leu-Ala-Gly-Leu-Leu-
Ser-Arg-Ser-Gly-Gly-Met-Val-Lys-Ser-Asn-Phe-Val-Pro-Thr-Asn-
Val-Gly-Ser-Lys-Ala-Phe-NH2                    (III)
```

Das Ausgangsmaterial erhält man folgendermassen:

Stufe 1.1: 30,0 g Chlormethylpolystyrol (20,1 mMol Cl) werden mit 6,1 g (40,2 mMol) p-Hydroxymethylbenzoesäure in 150 ml entgastem Dimethylformamid vorgelegt und während 3 Minuten werden 6,0 ml (40,2 mMol) 1,8-Diazabicyclo(5,4,0)undec-7-en(1,5,5) zugefügt. Das RG wird während 20 h bei 50° gerührt. Das erhaltene Harz wird abgenutscht und je 3mal mit ca. 150 ml Dimethylformamid, Dimethylformamid/Wasser (9:1), Methanol, Methylenchlorid und Methanol gewaschen. Das Harz wird am HV bis zur Gewichtskonstanz getrocknet, worauf man Hydroxybenzyl-p-carbonyloxymethyl-polystyrol-Syntheseharz erhält.

Stufe 1.2: 1,1 g (4 mMol) Boc-Phe und 3,0 g (ca. 2 mMol) des gemäss Stufe 1.1 erhaltenen Harzes werden unter schwachem Rühren während ca. 5 Min. in 20 ml Methylenchlorid und 1,5 ml Dimethylformamid bei RT suspendiert. Das Gemisch wird auf 0-5° abgekühlt und 865 mg (4,2 mMol) Dicyclohexylcarbodiimid in 1,8 ml Methylenchlorid werden in drei Portionen innerhalb von 5 Min. zugefügt. Nach weiteren 5 Min. werden 24 mg (0,2 mMol) 4-Dimethylaminopyridin und nach 10 Min. 220 µl N-Methyl-morpholin (2 mMol) in 1 ml Methylenchlorid zugefügt. Das Gemisch wird während 1 h bei 0° und anschliessend während 20 h bei RT gehalten. Das Harz wird abfiltriert und je 5mal mit je ca. 50 ml Methylenchlorid, Dimethylformamid, Methanol, Methylenchlorid und Methanol gewaschen. Nach dem Trocknen am HV wies es ein Gewicht von 3,34 g auf. Zur Blockierung nicht umgesetzter Hydroxygruppen wird das Harz in 20 ml Methylenchlorid suspendiert, 1 ml Pyridin zugefügt und nach dem Kühlen im Eisbad mit 1 ml Benzoylchlorid versetzt. Nach 15 Min. bei 0° und 1 h bei RT wird das Harz abgenutscht und je 2mal nacheinander mit je 50 ml Methylenchlorid, Dimethylformamid, Methanol, Methylenchlorid und Methanol gewaschen. Das Harz wird am HV bis zur Gewichtskonstanz getrocknet. Die Aminosäurebeladung errechnet sich aus dem N-Gehalt zu 0,35 mMol/g.

Stufe 1.3: 1,0 g Boc-Phe-Harz (0,35 mMol) werden in einer halbautomatischen Peptidsynthesemaschine zur Abspaltung der Boc-Gruppe, zur Ankupplung der zweiten Aminosäure Boc-Ala und zur Abspaltung dieser Boc-Gruppe folgenden Operationen unterworfen (alle Waschoperationen mit je ca. 20 ml):

| | |
|---|---|
| 1mal 1,0 Min. | Isopropanol, |
| 3mal 0,5 Min. | Ethylendichlorid, |
| 3mal 5,0 Min., | 1mal 15 Min. Trifluoressigsäure/Ethylenchlorid zur Abspaltung der Boc-Gruppe, |
| 3mal 0,5 Min. | Ethylendichlorid, |
| 1mal 2,0 Min. | Isopropanol, |
| 2mal 0,5 Min. | Ethylendichlorid, |
| 2mal 0,5 Min. | Dimethylacetamid, entgast, |
| 3mal 2,0 Min. | 2 % Diisopropylethylamin in Dimethylacetamid, |
| 2mal 0,5 Min. | Ethylendichlorid, |
| 3mal 0,5 Min. | Dimethylacetamid, dest, |
| 1mal 120 Min. | Kupplung: 1,5 mMol Boc-Ala, 221 mg (1,5 mMol) Hydroxy-benzotriazol, 320 mg (1,6 mMol) Dicyclohexylcarbodiimid in 0,32 ml Ethylendichlorid und 3,5 ml Dimethylacetamid: 20 Min. bei RT, 100 Min. bei 45°, |
| 1mal 2,0 Min. | 10 ml Acetanhydrid/Pyridin/Dimethylacetamid (10:10:80, v/v) zur Blockierung nicht umgesetzter Aminogruppen, |
| 1mal 0,5 Min. | Dimethylacetamid, entgast, |
| 1mal 1,0 Min. | Isopropanol, |
| 2mal 0,5 Min. | Ethylendichlorid und |
| 2mal 0,5 Min. | Dimethylacetamid, entgast, |
| 1mal 1,0 Min. | Isopropanol, |
| 3mal 0,5 Min. | Ethylendichlorid, |
| 3mal 5,0 Min., | 1mal 15 Min. Trifluoressigsäure/Ethylenchlorid zur Abspaltung der Boc-Gruppe, |
| 3mal 0,5 Min. | Ethylendichlorid, |
| 1mal 2,0 Min. | Isopropanol, |
| 2mal 0,5 Min. | Ethylendichlorid, |
| 2mal 0,5 Min. | Dimethylacetamid, entgast, |
| 3mal 2,0 Min. | 2 % Diisopropylethylamin in Dimethylacetamid, |
| 2mal 0,5 Min. | Ethylendichlorid, |
| 3mal 0,5 Min. | Dimethylacetamid, dest. |

Stufe 1.4: Nacheinander werden die folgenden Aminosäuren in der obengenannten Peptidsynthesemaschine an das gemäss Stufe 1.3 erhaltene Produkt ankondensiert:
Fmoc-Lys(Boc), Fmoc-Ser(tBu), Fmoc-Gly, Fmoc-Val, Fmoc-Asn, Fmoc-Thr(tBu), Fmoc-Pro, Fmoc-Val, Fmoc-Phe, Fmoc-Asn, Fmoc-Ser(tBu), Fmoc-Lys(Boc), Fmoc-Val, Fmoc-Met, Fmoc-Gly, Fmoc-Gly, Fmoc-Ser(tBu), Fmoc-Arg(Mtr), Fmoc-Ser(tBu), Fmoc-Leu, Fmoc-Leu, Fmoc-Gly, Fmoc-Ala, Fmoc-Leu, Fmoc-Arg(Mtr), Fmoc-His(Fmoc), Fmoc-Thr(tBu), Fmoc-Val, Fmoc-Cys(Acm), Fmoc-Thr(tBu), Fmoc-Ala, Fmoc-Thr(tBu), Fmoc-Asn, Fmoc-Cys(Acm) und Boc-Ala.

Dabei wird jeweils der folgende Operationszyklus durchlaufen, wobei jeweils die Fmoc-Schutzgruppe der vorangehenden Aminosäure abgespalten wird:

| | |
|---|---|
| 1mal 1,0 Min. | Isopropanol, |
| 2mal 0,5 Min. | Ethylendichlorid, |
| 1mal 0,5 Min. | Dimethylacetamid, entgast, |
| 1mal 1,0 Min. | Isopropanol, |
| 3mal 0,5 Min. | Dimethylacetamid, entgast, |
| 4mal 2,0 Min. | 20 % (Vol.) Piperidin in Dimethylacetamid, |
| 2mal 0,5 Min. | Dimethylacetamid, entgast, |
| 2mal 1,0 Min. | Wasser/peroxidfreies Dioxan (1:2 v/v), |
| 2mal 0,5 Min. | Dimethylacetamid, entgast, |
| 2mal 0,5 Min. | Ethylendichlorid, |
| 3mal 0,5 Min. | Dimethylacetamid, dest. |
| 1mal 120 Min. | Kupplung: 1,5 mMol Fmoc-Aminosäure bzw. zum Schluss Boc-Ala, 221 mg (1,5 mMol) Hydroxy-benzotriazol, 320 mg (1,6 mMol) Dicyclohexylcarbodiimid in 0,32 ml Ethylendichlorid und 3.5 ml Dimethylacetamid: 20 Min. bei RT, 100 Min. bei 45°, |
| 1mal 2,0 Min. | 10 ml Acetanhydrid/Pyridin/Dimethylacetamid (10:10:80, v/v) |
| 1mal 0,5 Min. | Dimethylacetamid, entgast, |
| 1mal 1,0 Min. | Isopropanol, |
| 3mal 0,5 Min. | Ethylendichlorid und |
| 2mal 0,5 Min. | Dimethylacetamid, entgast. |

Die Vollständigkeit der Kupplungsreaktion wird durch Nachweis nicht umgesetzter Aminogruppen mit Ninhydrin (Kaiser-Test) qualitativ geprüft. Bei positivem Test wird das Harz mit Ethylenchlorid gewaschen und eine Nachkupplung mit 1,5 mMol Aminosäurederivat und 1,6 mMol Dicyclohexylcarbodiimid in 3,8 ml Ethylendichlorid/Trifluorethanol (75:25) während 120 Min. bei RT durchgeführt. Das Harz wird nach der letzten Kupplung mit Dimethylacetamid, Ethylendichlorid und Isopropanol erschöpfend gewaschen und im HV getrocknet.

Stufe 1.5: Das gemäss Stufe 1.4 erhaltene Produkt wird zur Abspaltung der tBu- und Boc-Schutzgruppen folgendermassen bei RT behandelt (je 30 ml):

| | |
|---|---|
| 2mal 2 Min. | Methylenchlorid, |
| 1mal 5 Min. | Trifluoressigsäure/Methylenchlorid/1,2 Ethandithiol/m-Kresol (50:43:2:5), |
| 1mal 30 Min. | Trifluoressigsäure/Methylenchlorid/1,2 Ethandithiol/m-Kresol (50:43:2:5), |
| 1mal 60 Min. | Trifluoressigsäure/Methylenchlorid/1,2 Ethandithiol/m-Kresol (50:43:2:5), |
| 2mal 2 Min. | Methylenchlorid und |
| 2mal 2 Min. | Methanol. |

Stufe 1.6: Das gemäss Stufe 1.5 erhaltene Produkt wird zwecks Ablösung des Peptids vom Harz und zwecks Amidierung der terminalen Carboxylgruppe nach dem Trocknen am HV in 30 ml Dimethylformamid suspendiert. Bei -70° werden ca. 10 ml Ammoniak einkondensiert. Ueberschüssiges Ammoniak wird bei 0° abgedampft und die Mischung wird in einem Druckgefäss während 20 Stunden bei RT gerührt. Das Harz wird abfiltriert, 2mal mit Wasser (je 20 ml) 3mal mit Methanol/Wasser (1:1) und 10mal mit Trifluorethanol/Wasser (1:1) gewaschen und das Filtrat eingedampft. Der Rückstand wird in 30 ml Essigsäure/Wasser (9:1) gelöst und lyophilisiert.

Stufe 1.7: Das gemäss Stufe 1.6 erhaltene Lyophilisat wird zur Abspaltung der Mtr-Gruppen in 20 ml Trifluoressigsäure/1,2-Ethandithiol (99:1) gelöst. 1.5 ml m-Kresol werden zugefügt und die Mischung wird während 2 h bei 50° gehalten. Das rohe Di-Acm-Peptid wird durch Zugabe von 100 ml Diethylether gefällt, abfiltriert und mit Diethylether gewaschen. Das Produkt wird in 25 ml Essigsäure/Wasser (1:1) gelöst, von einer Trübung abfiltriert und das Filtrat wird zur analytischen Auftrennung im Hplc eingesetzt.

Die Hplc wird unter folgenden Bedingungen durchgeführt:

Säule: Nucleosil 10 $\mu m$ $C_{18}$ (Macherey Nagel, Düren, Bundesrepublik Deutschland), 200x4,8 mm, linearer Gradient 0 %-90 % B, 60 Min. A: 0,1 % Trifluoressigsäure in Wasser, B: 0,1 % Trifluoressigsäure in Acetonitril.

Das dem Hauptpeak entsprechende Material wird gesammelt und dessen Identität mit dem gewünschten Produkt durch FAB-MS bestätigt (Massenpeak: 3937,6).

Mikropräparative Trennung: 100 mg des Rohproduktes werden an einer 250x21 mm Kolonne aufgetrennt. Die gesammelten Fraktionen werden im Vakuum eingedampft. Der Rückstand wird in 2 ml Wasser gelöst, filtriert und lyophilisiert.

Beispiel 2: Escherichia coli-Klone, die Plasmide mit dem DNA-Insert der Formel XIV enthalten,

d(GAATTCATGGCTTGCAACACCGCTACCTGCGTTACCCACCGTCTGGCT
d(CTTAAGTACCGAACGTTGTGGCGATGGACGCAATGGGTGGCAGACCGA

GGTCTGCTGTCTCGTTCTGGTGGTATGGTTAAATCTAACTTCGTTCCGACC (XIV)
CCAGACGACAGAGCAAGACCACCATACCAATTTAGATTGAAGCAAGGCTGG

AACGTTGGTTCTAAAGCTTTCTACTAGGATCC)
TTGCAACCAAGATTTCGAAAGATGATCCTAGG)

werden in 5 ml L-Medium über Nacht (16 Stunden) bei 37° C und 250 upm kultiviert. L-Medium ist wie folgt zusammengesetzt:

| | |
|---|---|
| Bacto Tryptone | 10 g |
| Bacto Hefe-Extrakt | 5 g |
| NaCl | 5 g |
| Glucose | 5 g |
| Ampicillin | 0,1 g |

1 ml dieser Uebernachtkultur wird am nächsten Tag in 25 ml M9-Medium übertragen. M9-Medium ist wie folgt zusammengesetzt:

| | |
|---|---|
| $Na_2HPO_4 \cdot 7H_2O$ | 13,25 g |
| $KH_2PO_4$ | 3,0 g |
| NaCl | 0,5 g |
| $NH_4Cl$ | 1,0 g |
| $CaCl_2 \cdot 2H_2O$ | 0,015 g |
| $MgSO_4 \cdot 7H_2O$ | 0,25 g |
| Casaminosäuren | 2,5 g |
| Vitamin $B_1$ | 0,0099 g |
| Glucose | 5,0 g |
| Ampicillin | 0,1 g |

Es wird bei 37° C und 250 upm solange kultiviert, bis die Bakteriensuspension eine optische Dichte ($OD^{623}$) von ca. 0,9-1,0 erreicht hat. Anschliessend erntet man die Zellen (5 ml der wachsenden Kultur) und resuspendiert die Bakterien in 0,5 ml einer Lösung von 50 mM Tris•HCl (pH 8) und 30 mM NaCl. Danach wird die Suspension auf 1 mg/ml Lysozym (Boehringer) eingestellt und 30 Min. in Eis gestellt. Durch abwechselndes Einfrieren der Suspension in flüssigem Stickstoff und Auftauen bei 37° C werden die Bakterien zerstört. Dieser Vorgang wird 5mal wiederholt, anschliessend wird die Mischung 30 Min. bei 16000 upm bei 4° C zentrifugiert. Die Ueberstände werden mittels HPLC oder mittels Antikörpern auf Gehalt an CGRP IIa untersucht.

Alternativ wird die obenerwähnte Bakteriensuspension folgendermassen aufgearbeitet:

Die Zellen werden aus der Kulturlösung mittels Zentrifugation bei mehr als 8000 upm abgetrennt und anschliessend mechanisch in einer Dyno-Mill Mühle oder enzymatisch in einem Lysepuffer (pH 8) mittels Lysozym aufgeschlossen.

Anschliessend werden nacheinander die folgenden Schritte durchgeführt:

1. pH-Ausfällung der bakteriellen Proteine mittels Essigsäure (Endkonzentration 1 %, pH 4,0), wobei CGRP II im Ueberstand bleibt und das Sediment abgetrennt wird.
2. Das rohe Peptid wird im batch Verfahren an einer Ionenaustauscher Säule (CH 52, Whatmann) adsorbiert und mittels eines Salzgradienten von 10 mM Ammoniumacetat (pH 4,5) bis 300 mM (pH 6,5) eluiert. Die Hauptkomponente wird durch mehrfache Lyophilisation aus destilliertem Wasser oder durch Diafiltration entsalzt.
3. Das Produkt wird mittels multiplikativer Verteilung nach Craig im Zweiphasen System 0,1 % Essigsäure-n-Butanol (1:1) gereinigt (200 Transfers). Das Lösungsmittel entfernt man am Rotationsverdampfer und lyophilisiert die gesammelten Fraktionen.
4. Die Abtrennung von allfälligen Restproteinen und niedermolekularen Anteilen erfolgt durch Gelfiltrationschromatographie an Sephadex® G 50 (Pharmacia), wobei mit 2%iger Essigsäure, 1 % $\beta$-Mercaptoethanol eluiert wird oder mittels Umkehrphasen-HPLC auf dem präparativen Gerät von Waters (LC-Prep 500) auf einer Nucleosil C-18 Säule (Dydac 300 Å, 15-20 $\mu$m) mittels eines Lösungsmittelgradienten.

Man erhält CGRP IIa der Formel XV.

```
  S——————————————S
  |              |
Ala-Cy-Asn-Thr-Ala-Thr-Cy-Val-Thr-His-Arg-Leu-Ala-Gly-Leu-Leu-
Ser-Arg-Ser-Gly-Gly-Met-Val-Lys-Ser-Asn-Phe-Val-Pro-Thr-Asn-Val-
Gly-Ser-Lys-Ala-Phe-Tyr-OH                    (XV)
```

Die als Ausgangsprodukt verwendeten E.coli-Klone erhält man folgendermassen:

Stufe 2.1:

3,05 g (5 mMol) 5'-(4-Monomethoxytrityl)-N-isobutyryl-desoxyguanosin in 20 ml abs. Pyridin Verden mit 750 mg Bernsteinsäureanhydrid und 910 mg 4-Dimethylaminopyridin versetzt und 16 Stunden bei Raumtemperatur belassen. Nach Einengen der Pyridin-Lösung wird in 200 ml Essigsäureethylester aufgenommen, zweimal mit je 200 ml 0,1 M Phosphatpuffer unter Zusatz von 10 ml gesättigter Kochsalz-Lösung ausgeschüttelt, nochmals mit gesättigter Kochsalzlösung gewaschen, getrocknet, eingeengt und Hexan zugetropft. Das ausgefallene Produkt wird abgetrennt und zweimal mit Diethylether zerrieben, dann in 300 ml Essigsäureethylester gelöst und bei 0 °C mit 180 ml 0,1 M Kaliumhydrogensulfat von pH 2,5 ausgeschüttelt. Nach zweimaligem Waschen mit Wasser wird die Essigesterlösung mit Natriumsulfat getrocknet, filtriert, mit 0,5 ml Pyridin versetzt, eingeengt und tropfenweise mit Hexan verdünnt. Das ausgefallene Bernsteinsäurederivat wird abfiltriert.

1,15 g dieser Verbindung werden zusammen mit 190 mg N-Hydroxysuccinimid in 4 ml Essigsäureethylester und 2 ml Dimethylformamid gelöst und bei 0 °C mit 370 mg N,N'-Dicyclohexylcarbodiimid versetzt. Nach Stehen über Nacht im Kühlschrank wird der ausgefallene N,N'-Dicyclohexylharnstoffabfiltriert, das Filtrat mit Essigsäureethylester verdünnt, mit kaltem 0,1 M Natriumhydrogencarbonat und Wasser extrahiert, getrocknet und im Vakuum zur Trockne eingedampft. Der Rückstand wird mit Essigsäureethylester an Kieselgel chromatographiert. DC: $R_f$ 0,55 in Dichlormethan-Methanol (9:1).

100 mg dieses N-Succinimidoyl-bernsteinsäureesters werden mit 1 g Aminomethyl-polystyrol (Amingehalt 110 $\mu$Mol/g) in 2 ml Dichlormethan und 4 ml Dimethylformamid 20 Stunden gerührt. Das Polymer-Harz wird abfiltriert und mit Dimethylformamid, Methanol, Dichlormethan und Methanol ausgewaschen. Nach dem Trocknen werden die nicht umgesetzten Aminogruppen acetyliert, indem das Harz in 6 ml Pyridin mit 1 ml Essigsäureanhydrid und 100 mg 4-Dimethylaminopyridin 30 Min. gerührt wird. Das erhaltene Polymer-Harz, worin 5'-(4-Monomethoxytrityl)-N-isobutyryl-desoxyguanosyl-3'-O-succinyl-Reste an die Aminogruppen des Aminomethyl-polystyrols geknüpft sind, wird mit Dichlormethan, Dimethylformamid, Methanol und Dichlormethan ausgewaschen und bis zur Gewichtskonstanz getrocknet. Die spektroskopische Methoxytrityl-Bestimmung ergibt eine Beladung von 32 $\mu$Mol/g.

Stufe 2.2.:

7,73 g (15 mMol) 5'-(4-Monomethoxytrityl)-thymidin werden zweimal mit abs. Pyridin eingedampft. Der Rückstand wird in 20 ml abs. Tetrahydrofuran gelöst und zu 80 ml einer 0,2 M Lösung von 2-Chlorphenyl-di-(1-benzotriazolyl)-phosphat in Tetrahydrofuran unter Rühren und Feuchtigkeitsausschluss getropft und das Reaktionsgemisch 1 Stunde bei Raumtemperatur gerührt. Die erhaltene Lösung des 2-Chlorphenyl-1-benzotriazolyl-5'-(4-monomethoxytrityl)-thymidin-3'-phosphats wird in drei Teile geteilt.

$\alpha$) Hydrolyse zu Triethylammonium-2-chlorphenyl-5'-(4-monomethoxytrityl)-thymidin-3'-phosphat:

Zu einem Drittel der obigen Lösung von 2-Chlorphenyl-1-benzotriazolyl-5'-(4-methoxytrityl)-thymidin-3'-phosphat werden unter Kühlung 100 ml 0,5 M Triethylammoniumbicarbonat gegeben. Nach 15 Min. wird mit Dichlormethan extrahiert. Die Dichlormethan-Lösung wird mit Wasser gewaschen, eingeengt und tropfenweise mit Petrolether versetzt. Die erhaltene Fällung wird abgenutscht, mit Diethylether-Petrolether (1:1) ausgewaschen und im Vakuum getrocknet. DC: $R_f$ 0,35 in Dichlormethan-Methanol-Wasser (75:22:3).

β) Veresterung zu 2-Cyanoethyl-2-chlorphenyl-5'-(4-monomethoxytrityl)-thymidin-3'-phosphat und Abspaltung der 4-Monomethoxytrityl Schutzgruppe:

Zu einem Drittel der Lösung von 2-Chlorphenyl-1-benzotriazolyl-5'-(4-monomethoxytrityl)-thymidin-phosphat werden 1,3 ml 2-Cyanoethanol und 2 ml Pyridin gegeben. Das Gemisch wird über Nacht bei Raumtemperatur belassen. Die Lösungsmittel werden im Vakuum abdestilliert und der Rückstand in Essigsäureethylester gelöst und mehrmals mit 0,1 M Phosphatpuffer pH 7 und Wasser ausgeschüttelt. Die organische Phase wird getrocknet, eingeengt und in Hexan eingetropft. Der Niederschlag wird abfiltriert, in 50 ml Dichlormethan-Methanol 7:3 gelöst und bei 0 ° C mit einer Lösung von 3,8 g p-Toluolsulfonsäuremonohydrat in 75 ml Dichlormethan-Methanol 7:3 versetzt. Nach 2 Stunden wird die Reaktionslösung mit Dichlormethan verdünnt und mit einer kalten Natriumhydrogencarbonat-Lösung ausgeschüttelt. Die organische Phase wird eingeengt und mit Hexan versetzt. Das ausgefallene 2-Cyanoethyl-2-chlorphenyl-thymidin-3'-phosphat wird an Kieselgel mit Dichlormethan-Methanol (96:4) chromatographiert. DC: $R_f$ von 0,45 in Dichlormethan-Methanol (9:1).

γ) Kondensation zum 5'-(4-Methoxytrityl)-3'-cyanoethyl)-bis-thymidin-dinukleotid:

2,2 g 2-Cyanoethyl-2-chlorphenyl-thymidin-3'-phosphat werden zweimal durch Eindampfen mit abs. Pyridin entwässert, in 20 ml abs. Tetrahydrofuran gelöst und zum restlichen Drittel der Lösung von 2-Chlorphenyl-1-benzotriazolyl-5'-(4-monomethoxytrityl)-thymidin-3'-phosphat gegeben. Nach 18 Stunden bei Raumtemperatur werden zur Reaktions lösung unter Eiskühlung 10 ml Wasser und 200 ml Essigsäureethylester zugegeben. Die organische Phase wird mehrmals mit Natriumhydrogencarbonat und Wasser gewaschen, über Natriumsulfat getrocknet und auf ein kleines Volumen eingeengt. Das im Phosphat-Teil und am 5'- bzw. 3'-Ende geschützte Dinukleotid wird durch Eintropfen in Diethylether-Hexan 1:1 gefällt; $R_f$ = 0,48 in Dichlormethan-Methanol (9:1).

Stufe 2.3: 9,20 g (15 mMol) 5'-(4-Monomethoxytrityl)-N-isobutyryldesoxyguanosin werden zweimal mit abs. Pyridin eingedampft. Der Rückstand wird in 20 ml abs. Tetrahydrofuran gelöst und zu 75 ml einer 0,2 M Lösung von 2-Chlorphenyl-di-(1-benzotriazolyl)-phosphat in Tetrahydrofuran unter Rühren und Feuchtigkeitsausschluss getropft und das Reaktionsgemisch 1 Stunde bei Raummtemperatur gerührt Die erhaltene Lösung des 2-Chlorphenyl-1-benzotriazolyl-5'-(4-monomethoxytrityl)-N-isobutyryl-guanosyl-3'-phosphats wird in Stufe 2.4 weiterverarbeitet.

Stufe 2.4: 1,17 g (1 mMol) des oben beschriebenen vollgeschützten Dinukleotids werden in 30 ml Dichlormethan-Methanol (7:3) gelöst und unter Eiskühlung mit einer Lösung von 1,9 g p-Toluolsulfonsäuremonohydrat in 20 ml Dichlormethan-Methanol (7:3) versetzt. Nach 2 Stunden wird eiskalte Natriumhydrogencarbonat-Lösung zugegeben und mit Dichlormethan extrahiert. Die organische Phase wird getrocknet, eingeengt und in Hexan eingetropft. Das ausgefallene rohe Dinukleotid mit freier 5'-Hydroxygruppe wird an Kieselgel mit einem Gradienten von 2-8 % Methanol in Dichlormethan chromatographiert. $R_f$ = 0,33 in Dichlormethan-Methanol (9:1).

900 mg dieses 5'-Hydroxy-dinukleotids werden zweimal mit Pyridin eingedampft, dann in 5 ml abs. Tetrahydrofuran gelöst und mit 10 ml der in Stufe 2.3 erhaltenen Lösung versetzt. Nach 2 Stunden werden 2 ml eiskaltes Wasser zugegeben und nach einer weiteren Stunde mit Dichlormethan extrahiert. Die organische Phase wird mit gesättigtem Natriumhydrogencarbonat und Wasser gewaschen, getrocknet, eingeengt und mit Aether versetzt. Das ausgefallene Trinukleotid wird durch Chromatographie an Kieselgel gereinigt; $R_f$ = 0,45 in Dichlormethan-Methanol (9:1).

Stufe 2.5: Analog den Stufen 2.2, 2.3 und 2.4 werden folgende geschützte Trinukleotide der allgemeinen Formel XVI hergestellt,

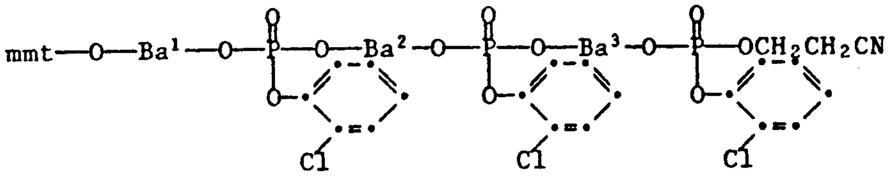

(XVI)

worin $Ba^1$, $Ba^2$ und $Ba^3$ unabhängig voneinander je den bivalenten Rest eines der folgenden Nukleoside bedeuten:

N-Benzoyl-desoxyadenosin (A')
N-Benzoyl-desoxycytidin (C'),
N-Isobutyryl-desoxyguanosin (G') oder
Thymidin (T).

| Trinukleotid | | | $R_f$ (I) |
|---|---|---|---|
| $Ba^1$ | $Ba^2$ | $Ba^3$ | |
| T | T | C' | 0,55 |
| T | C' | T | 0,46 |
| T | C' | G' | 0,45 |
| T | A' | C' | 0,56 |
| T | A' | A' | 0,53 |
| T | A' | G' | 0,60 |
| T | G' | C' | 0,44 |
| T | G' | G' | 0,43 |
| C' | T | T | 0,53 |
| C' | T | G' | 0,46 |
| C' | C' | T | 0,45 |
| C' | C' | A' | 0,51 |
| C' | A' | C' | 0,51 |
| C' | A' | A' | 0,52 |
| C' | A' | G' | 0,44 |
| C' | G' | T | 0,49 |
| C' | G' | A' | 0,38 |
| A' | T | T | 0,55 |
| A' | T | G' | 0,48 |
| A' | C' | C' | 0,48 |
| A' | A' | C' | 0,46 |
| A' | A' | G' | 0,51 |
| A' | G' | T | 0,45 |
| A' | G' | A' | 0,49 |
| G' | C' | T | 0,55 |
| G' | A' | T | 0,44 |
| G' | A' | A' | 0,50 |
| G' | G' | T | 0,46 |

Stufe 2.6: Jedes der in den Stufen 2.4 und 2.5 beschriebenen Trinukleotide wird zur Abspaltung der 2-Cyanethylschutzgruppe folgendermassen behandelt: 10 µMol Trinukleotid werden unter Feuchtigkeitsausschluss in 60 µl Pyridin-Acetonitril-Triethylamin (1:1:1) gelöst. Nach 1 Stunde bei Raumtemperatur werden 0,7 ml peroxidfreier Diethylether zugetropft und der Niederschlag abzentrifugiert. Das rohe Triethylammoniumsalz wird in 50 µl Pyridin gelöst und nochmals mit 0,5 ml Diethylether ausgefällt, abzentrifugiert und 15 Stunden am Hochvakuum getrocknet.

Stufe 2.7: Die Kupplung der gemäss Stufe 2.6 erhaltenen teilgeschützten Trinukleotide an das gemäss Stufe 2.1 erhaltene geschützte Guanosin-Polystyrol-Harz wird folgendermassen durchgeführt:

Alle Operationen werden unter Feuchtigkeitsausschluss in einem Reaktionsgefäss mit 150 µl Inhalt und mikroprozessorgesteuerter Lösungsmittel- und Reagens-Zugabe durchgeführt. 15 mg (0,48 µMol) des Guanosin-Polystyrol-Harzes (Stufe 2.1) werden im Reaktionsgefäss vorgelegt und folgenden Operationen unterworfen:

1. Methylenchlorid, 2 ml/Min., 4 Min.
2. Methylenchlorid-Isopropanol (85:15), 2 ml/Min., 2 Min.
3. Zinkbromid 1 M und 1,2,4-Triazol 0,02 M in Methylenchlorid-Isopropanol (85:15), 2 ml/Min., 2-3,5 Min.
4. Methylenchlorid-Isopropanol (85:15), 2 ml/Min., 4 Min.
5. Triethylammoniumacetat, 0,5 M in DMF, 2 ml/Min., 5 Min.
6. Molekularsieb-getrocknetes Pyridin, 2 ml/Min., 3 Min.
7. Tetrahydrofuran (Peroxid-frei, Molekularsieb-getrocknet), 2 ml/Min., 3 Min.
8. Stickstoffstrom, 10 Min.

9. Injektion 10 µMol Trinukleotid (siehe unten) und 8,9 mg (30 µMol) 1-Mesitylensulfonyl-3-nitro-1,2,4-triazol (MSNT) gelöst in 150 µl Pyridin
10. 45 ° C, 20 Min.
11. Pyridin, 2 ml/Min., 4 Min.
12. Acetanhydrid 5 % und 4-Dimethylaminopyridin 2,5 % in Pyridin, 2 ml/Min., 4 Min.
13. Pyridin, 2 ml/Min., 4 Min.
14. Pyridin-Isopropanol (1:1), 2 ml/Min., 3 Min.

Alle 14 Operationen werden 24mal wiederholt, wobei bei der 9. Operation nacheinander die folgenden Desoxytrinukleotide in Form ihrer Triethylammoniumsalze (s. Stufe 2.6) eingesetzt werden: d(A'A'C', C'A'G', C'A'C', TA'C', C'C'A', TA'A', A'TT, TA'G', A'G'T, C'G'A', G'A'A', TC'G', TG'G', C'G'T, C'A'A', A'A'C', TA'G', C'TT, A'A'G', A'G'A', A'G'T, C'C'T, C'A'T und G'TC').

Die mittlere Kupplungsausbeute beträgt 97 %. Das Endprodukt hat folgende Struktur, wobei darin zusätzlich noch die 2-Chlor-phenyl-Schutzgruppen vorhanden sind: d(mmt-G'TC'C'A'TC'C'TA'G'T'A'G'A'A'A'G'C'TTTA'G'A'A'C'C'A'A'C'G'TTG' G'TC'G'G'A'A'C'G'A'A'G'TTA'G'A'TTTA'A'C'C'A'TA'C'C'A'C'C'A'G'A'A' C'C'G')-Polystyrolsyntheseharz.

Stufe 2.8: Das gemäss Stufe 2.7 erhaltene Polydesoxynukleotid-Polystyrolsyntheseharz wird zwecks Abspaltung des Polynukleotids vom Träger und zwecks Abspaltung der Schutzgruppen folgendermassen behandelt:

35,0 mg (ca. 0,35 µmol) Polydesoxynukleotid-Synthese 64/73 werden mit 66 mg (0,40 mMol) o-Nitrobenzaldoxim und 50 µl (0,40 mMol) 1,1,3,3-Tetramethylguanidin in 400 µl 95%igem Pyridin während 3 Stunden bei 50 ° C und während 12 Stunden bei Raumtemperatur gehalten. Nach dem Abblasen des Pyridins mit Stickstoff wird der Rückstand mit 1,6 ml wässerigem Ammoniak (33 %) versetzt und im geschlossenen Gefäss während 24 Stunden bei 50 ° C gehalten.

Die abgetrennte flüssige Phase wird im Vakuum vom Ammoniak befreit und 3mal mit je 3 ml peroxidfreiem Diethylether gewaschen. Nach dem Abtrennen der niedermolekularen Bestandteile an einer Biogel P6 Säule (100-200 mesh, 3x66 cm, 0,01 M Trimethylammoniumhydrogencarbonat pH 7,5, 1,5 ml/Min.) werden 250 OD (260 nm) Polydesoxynukleotid isoliert.

Insgesamt 60 ODs werden an einer HPLC-Säule aufgetrennt (PRP-1/Hamilton, 250 x 4,6 mm). Gradient (Lösung A: 0,05 M Triethylammoniumacetat pH 7,0; Lösung B: Lösung A:Acetonitril 1:1): 30 % B in A → 60 % B in A in 20 Min. bei 50 ° C und 2 ml/Min. Der lipophile Hauptpeak (Retentionszeit ca. 14 Min.) wird gesammelt, an einer DE52-Cellulose (Whatman) Säule aufkonzentriert, eluiert und mit Ethanol gefällt. Zur Abspaltung der 4-Methoxytrityl-Schutzgruppe wird der Niederschlag in 50 µl Essigsäure/$H_2O$ (4:1) gelöst und 45 Min. bei Raumtemperatur gehalten. Das Reaktionsprodukt wird lyophilisiert, mit Ethanol gefällt und zur Reinigung auf einem 8 % Polyacrylamidgel (7 M Harnstoff) elektrophoretisch aufgetrennt. Die der erwarteten Polydesoxynukleotid-Grösse entsprechende Bande wird ausgeschnitten und das Produkt elektroeluiert, an DE52-Cellulose aufkonzentriert und das Polydesoxynukleotid 64/73 der Struktur d-(GTCCATCCTAGTAGAAAGCTTTAGAACCAACGTTGGTCG GAACGAAGTTAGATTTAACCATACCACCA-GAACG) mit Ethanol gefällt.

Stufe 2.9: Analog den Stufen 2.7 und 2.8 erhält man das Polydesoxynukleotid (1/73) d(CTGGAATTCATGGCTTGCAACACCGCTACCTGCGTTACCCACCGTCTGGCTGGTCTGCTGTCTCG TTCTGGTG).

Stufe 2.10: Die gemäss den Stufen 2.8 und 2.9 erhaltenen Polynukleotide (64/73 und 1/73 komplementär) werden an den 5'-Enden radioaktiv phosphoryliert mit [$\gamma$-$^{32}$P]ATP und $T_4$ Polynukleotid-Kinase (Boehringer, Bundesrepublik Deutschland), wie beschrieben in Molecular Cloning, A Laboratory Manual (Herausgeber T. Maniatis et al.), Cold Spring Harbor Lab. 1982, Seite 125.

Stufe 2.11: Zwecks Polymerisation der gemäss Stufe 2.10 erhaltenen kinasierten Polynukleotide zur Duplex werden je 50 pMol kinasiertes Fragment 1/73 und kinasiertes Fragment 64/73 in 24 µl Wasser gelöst, die Lösung während 3 Min. auf 90 ° C erwärmt und innerhalb von 5 Min. auf 12 ° C abgekühlt. Nach Zugabe von 4 µl Endo-R Puffer (0,1 Molar Tris•HCl pH 7,5, 66 mM $MgCl_2$, 66 mM $\beta$-Mercaptoethanol, 0,6 M NaCl), 10 µl Desoxynucleosidtriphosphat-Gemisch (dATP, dCTP, dGTP, TTP, je $2 \cdot 10^{-3}$ Molar, mit $NH_3$ auf pH 7.0 eingestellt) und 2 µl (10 Einheiten) DNA-Polymerase I, Klenow-Fragment (Boehringer) wird während 30 Min. bei 12 ° C inkubiert. Die Reaktion wird durch 3minü-tiges Erhitzen auf 90 ° C gestoppt und das Gemisch bis zur Weiterverarbeitung bei -80 ° C aufbewahrt.

Die erhaltene DNA-Duplex hat die folgende Struktur

d( CTGGAATTCATGGCTTGCAACACCGCTACCTGCGTTACCCACCGTCTGGCT
d( GACCTTAAGTACCGAACGTTGTGGCGATGGACGCAATGGGTGGCAGACCGA

GGTCTGCTGTCTCGTTCTGGTGGTATGGTTAAATCTAACTTCGTTCCGACC
CCAGACGACAGAGCAAGACCACCATACCAATTTAGATTGAAGCAAGGCTGG

AACGTTGGTTCTAAAGCTTTCTACTAGGATCCTG)
TTGCAACCAAGATTTCGAAAGATGATCCTAGGAC)

Diese Duplex wird im folgenden mit "$F_o$" bezeichnet.

Stufe 2.12: 10 µg Plasmid $pBRH_{trp}$ [Deutsche Offenlegungsschrift 3 111 405 (Genentech)] werden mit 50 Einheiten EcoRI (Biolabs) gespalten während 60 Min. bei 37° C, und das Verdauungsgemisch wird nach Phenolextraktion durch einen Saccharose-Dichtegradienten (5-23 %) in 50 mM Tris•HCl (pH 8,0), 1 mM EDTA in einem TST41 (Kontron AG) Rotor fraktioniert. Die Zentrifugation dauert 14 Stunden bei 40 000 upm und 15° C. 0,3 ml Fraktionen werden mit einem ISCO-Gradientkollektor bei 1 ml/Min. gesammelt. Die Fraktionen, welche das kleinere Fragment enthalten, werden vereinigt, die Lösung wird auf TNE eingestellt und mit 2 Volumen Ethanol bei -20° C gefällt. Die DNA wird nach Zentrifugation in einer Eppendorf-Zentrifuge in 100 µl 10 mM Tris•HCl pH 7,5, 0,5 mM EDTA gelöst. 5 µg dieses DNA-Fragmentes werden mit 5 Einheiten BglII (Biolabs) 60 Min. bei 37° C gespalten. Das Reaktionsgemisch wird mit Phenol und Chloroform extrahiert und die DNA wird mit 2 Volumen Ethanol bei -80° C während 10 Min. inkubiert und die DNA durch Zentrifugation gesammelt und erneut in 50 µl 50 mM Tris•HCl (pH 8,0) gelöst. 2 µl von dieser Lösung werden entnommen (0,2 µg DNA) und bei einer DNA-Konzentration von 10 ng/µl in 50 mH Tris•HCl (pH 8,0) mit 1 Einheit intestinaler alkalischer Kälber-Phosphatase (Boehringer) wahrend 30 Min. bei 37° C inkubiert. Das Enzym wird durch Erhitzen der Lösung während 60 Min. bei 65° C inaktiviert. 0,04 µg DNA wird entnommen und 5'-terminal durch Inkubation mit 10 µCi [$\gamma$-$^{32}$P]-ATP (>5000 Ci/mmol, Amersham) und 5 Einheiten $T_4$ Polynucleotid-Kinase (P-L Biochemicals) in 20 µl Reaktionsvolumen in 50 mM Tris•HCl (pH 9,5), 10 mM $MgCl_2$, und 5 mM DTT, während 30 Min. bei 37° C radioaktiv phosphoryliert. Die radioaktive Probe wird mit der nicht-markierten Probe (siehe oben) gemischt und die DNA-Fragmente werden durch einen 5-23 % Saccharose-Dichtegradienten in 50 mM Tris•HCl (pH 8,0), 1 mM EDTA in einem TST60 Rotor fraktioniert. Die Zentrifugation erfolgt während 5 Stunden bei 60 000 upm und 15° C. Es werden 0,2 ml Fraktionen gesammelt. Die Radioaktivität jeder Fraktion wird durch Messung der Cerencov-Strahlung bestimmt und die Fragmente damit identifiziert. Die gewünschten Fraktionen, welche das kleine DNA-Fragment enthalten, werden vereinigt, die DNA mit 2 Volumina Ethanol gefällt und nach Zentrifugation wieder in 20 µl 10 mM Tris•HCl pH 7,5, 0,5 mM EDTA gelöst.

Das $^{32}$P-markierte EcoRI-BglII DNA-Fragment wird mit 0,2 Einheiten TaqI (Biolabs) in 50 µl Volumen während 10 Min. bei 37° C partiell gespalten. Das Reaktionsgemisch wird auf 0,2 % SDS, 10 % Glycerin, 10 mM EDTA, 0,05 % Bromphenol-Blau eingestellt und die DNA-Fragmente auf einem 6 % Polyacrylamidgel in Tris-Borat-EDTA [A.C. Peacock et al., Biochemistry 6, 1818 (1967)] aufgetrennt. Auf dem Autoradiogramm wird die das gewünschte EcoRI-TaqI enthaltende Bande (das grösste Teilfragment) identifiziert. Dieses Fragment (L) wird aus dem Gel extrahiert und gereinigt [W. Müller et al., J. Mol. Biol. 124, 343 (1978)] und in 10 µl 10 mM Tris•HCl pH 7,5, 1 mM EDTA gelöst.

Als Akzeptor-Plasmid wird pBR322, welches mit ClaI und EcoRI gespalten ist, verwendet: 2 µg pBR322 werden mit 4 Einheiten ClaI (Biolabs) in 20 µl Reaktionsvolumen während 60 Min. bei 37° C verdaut. Das Protein wird mit Phenol extrahiert und die DNA anschliessend mit 2 Volumina Ethanol bei -80° C während 10 Min. gefällt. Die DNA wird durch Zentrifugation gesammelt und dann mit 10 Einheiten EcoRI (Biolabs) während 30 Min. bei 37° C in 20 µl Reaktionsvolumen verdaut. Anschliessend wird die Lösung mit 2 Volumina 0,1 M Tris•HCl (pH 8,7 versetzt und mit 1 Einheit alkalischer Kälber-Phosphatase (Boehringer) während 30 Min. bei 37° C inkubiert. Die Phosphatase wird anschliessend durch Inkubation bei 65° C während 60 Min. inaktiviert.

100 ng des Akzeptor-Plasmids werden mit 5 µl Fragment L-DNA in 15 µl Reaktionsvolumen in 10 mM $MgCl_2$, 20 mM Tris•HCl (pH 7,8), 10 mM DTT, 0,5 mM ATP mit 30 Einheiten pro µl Reaktionsvolumen $T_4$ DNA-Ligase (Biolabs) während 2 Stunden inkubiert.

5 µl dieser Lösung werden zu einem Gemisch gegeben, das 150 µl mit Calciumchlorid behandelte E. coli HB101-Zellen (14) in 10 mM $MgCl_2$, 10 mM $CaCl_2$ und 10 mM Tris•HCl (pH 7,5) in einem

Gesamtvolumen von 200 µl enthält. Das Gemisch wird 20 Min. in Eis gekühlt, 1 Min. auf 42°C erhitzt und 10 Min. bei 20°C inkubiert. 1 ml Trypton-Medium [Trypton-Medium enthält 10 g Bacto-Trypton (Difco); 1 g Hefeextrakt (Difco); 1 g Glucose; 8 g NaCl und 294 mg $CaCl_2 \cdot 2H_2O$ in 1 l destilliertem Wasser] wird zugesetzt und das Gemisch 30 Min. bei 37°C unter Schütteln bei 300 upm inkubiert. Die Mischung wird auf zwei Agarplatten (McConkey Agar, Difco; 0,6 ml/Platte), supplementiert mit 50 µg/ml Ampicillin (Sigma), plattiert. Die Platten werden 12 bis 17 Stunden bei 37°C inkubiert.

Die Plasmid-DNA von 10 verschiedenen Kolonien wird wie folgt isoliert:
Die Kolonien werden zur Inokulierung von 10 ml Trypton-Medium, supplementiert mit 50 µg/ml Ampicillin, wie oben, in einem 25 ml Erlenmeyerkolben verwendet. Die Kulturen werden 15 bis 18 Stunden bei 37°C und 300 upm geschüttelt. Die Zellen werden durch Zentrifugieren (Sorval, HS-4 Rotor, 10 Min. bei 4000 upm, 4°C) geerntet.

Man erhält etwa 0,1 g Zellen, und diese werden in 1 ml 50 mM Tris•HCl (pH 8,0) resuspendiert. 0,25 ml Lysozymlösung [10 mg/ml in 50 mM Tris•HCl (pH 8,0); Lysozym wird von Sigma vertrieben] werden zugesetzt und nach 10minütiger Inkubation bei 0°C werden 0,15 ml 0,5 m EDTA (pH 7,5) zugegeben. Nach weiteren 10 Min. bei 0°C werden 60 µl 2%iges Triton X-100 (Merck) zugegeben. Nach 30 Min. bei 0°C wird die Probe 30 Min. bei 15'000 upm und 4°C in einem Sorval SA-600 Rotor zentrifugiert. Der Ueberstand wird mit 1 Vol. Phenol (gesättigt in TNE) deproteinisiert. Die Phasen werden durch Zentrifugieren (Sorval HB-4 Rotor) während 10 Min. bei 5000 upm und 4°C getrennt. Die obere Phase wird zweimal mit 1 Vol. Chloroform extrahiert. Pankreatische RNAse A (Sigma; 10 mg/ml in TNE 10 Min. bei 85°C vorerhitzt) wird bis zu einer Endkonzentration von 25 µg/ml zugegeben und das Gemisch 40 Min. bei 37°C inkubiert. Die Lösung wird dann auf 1M NaCl und 10 % Polyethylenglykol 6000 (Fluka, 20 Min. bei 120°C im Autoklaven behandelt) eingestellt und 2 Stunden bei -10°C inkubiert. Das Präzipitat wird in einem Sorval HB-4 Rotor (20 Min. bei 10 000 upm, 0°C) gesammelt und erneut in 100 µl TNE gelöst. Die DNA-Lösung wird mit 1 Vol. Phenol extrahiert und die DNA wird mit 2 Vol. Ethanol 10 Min. bei -80°C präzipitiert. Das Präzipitat wird durch Zentrifugieren in einer Eppendorf-Zentrifuge gesammelt und die DNA erneut in 20 µl 10 mM Tris•HCl (pH 7,5) und 0,5 mM EDTA gelöst. Aus einer 10 ml Kultur gewinnt man 8 bis 10 µg Plasmid-DNA.

Die Plasmid-DNAs werden nach Verdauung mit den folgenden Restriktionsenzymen analysiert:
Je 0,5 µg Plasmid-DNA wird mit HpaI (Biolabs) sowie mit HpaI (Biolabs) und EcoRI (Biolabs) mit ClaI (Biolabs) nach Standard-Vorschriften, gemäss Angaben des Enzym-Herstellers, gespalten. Die DNAs werden auf einem 1 % Agarose-Gel in 40 mM Tris•Acetat (pH 7,8) 1 mM EDTA und 0,5 µg/ml Ethidiumbromid fraktioniert. Die gewünschten Plasmide enthalten eine HpaI-Stelle und ergeben nach der 3fachen Verdauung neben dem grossen DNA-Fragment 2 kleinere Fragmente, welche grösser sind als das kleine EcoRI-ClaI-Fragment von pBR322. Eines dieser Plasmide wird mit p159 bezeichnet.

Stufe 2.13: 2 µg p159-DNA werden mit 10 Einheiten EcoRI (Biolabs) während 30 Min. bei 37°C verdaut. Die DNA wird mit Phenol extrahiert, mit Ethanol gefällt und nach Zentrifugation in 10 µl 10 mM Tris•HCl (pH 7,5), 0,5 mM EDTA gelöst. Die mit EcoRI verdaute DNA wird weiterhin mit 5 Einheiten DNA-Polymerase (Klenow-Fragment) (Boehringer) in 10 mM $MgCl_2$, 10 mM β-Mercaptoethanol, 50 nM NaCl, 0,1 mM dATP (P&L Biochemicals), 0,1 mM dTTP (P&L Biochemicals) während 15 Min. bei 12°C behandelt. Die Polymerase wird anschliessend durch Inkubation bei 85°C während 5 Min. inaktiviert. Das Reaktionsgemisch wird in 20 mM Tris•HCl (pH 7,8), 10 mM $MgCl_2$, 10 mM DTT, 0,5 mM ATP (Sigma) auf das 10fache verdünnt und mit 30 Einheiten $T_4$ DNA-Ligase pro µl Reaktionsgemisch während 1 Stunde bei 15°C inkubiert.

50 ng der DNA werden in E. coli transformiert (wie oben beschrieben) und auf McConkey-Agarplatten supplementiert mit 50 µg/ml Ampicillin, ausplattiert.

Die Plasmid-DNAs von 10 verschiedenen Kolonien wird, wie oben beschrieben, isoliert. Die Plasmid-DNAs werden analysiert durch Verdauung mit EcoRI. Die gewünschten Plasmide sind EcoRI-resistent. Die Analyse erfolgt wie oben beschrieben. Eines der gewünschten Plasmide wird als HRi145 bezeichnet.

Stufe 2.14: 2 µg pHRi145-DNA werden mit 5 Einheiten ClaI (Boehringer) während 60 min. bei 37°C behandelt, dann mittels Phenol-Extraktion deproteiniert. Die DNA wird mit Ethanol gefällt und dann in 20 µl 10 mM Tris•HCl (pH 7,5), 0,5 mM EDTA gelöst. Die überhängenden Enden werden, wie oben beschrieben, mit DNA Polymerase I (Klenow-Fragment) aufgefüllt, mit der Aenderung, dass dATP und dTTP ersetzt werden durch dCTP (P&L Biochemicals) und dGTP (P&L Biochemicals). Die Polymerase wird inaktiviert durch Inkubation bei 85°C während 5 Min. Das Reaktionsgemisch wird mit 2 Volumina 0,1 M Tris•HCl (pH 8,7) versetzt und mit 0,5 Einheiten Kälber-Phosphatase (Boehringer) während 30 Min. bei 37°C inkubiert. Das Reaktionsgemisch wird deproteiniert durch Phenol-Extraktion. Die DNA wird mit Ethanol gefällt und in 8 µl 10 mM Tris•HCl (pH 7,5), 0,5 mM EDTA gelöst.

Ein chemisch synthetisierter DNA-Linker der Formel

5'-GAATTCCATGGTACCATGGAATTC-3'

wird am 5'-Ende phosphoryliert, indem 8 pMol des Linkers mit 5 µCi [$\gamma$-$^{32}$P)-ATP (5500 Ci•mmol$^{-1}$ Amersham) in 8 µl Reaktionsvolumen, welches 0,1 mM rATP (Sigma), 50 mM Tris•HCl (pH 9,5), 10 mM $MgCl_2$, 5 mM DTT und 2 Einheiten $T_4$ Polynucleotid-Kinase (P&L Biochemicals) enthält, während 30 Min. bei 37°C inkubiert werden. Die Reaktion wird gestoppt durch Einfrieren bei -80°C.

Der radioaktiv markierte Linker wird anschliessend mit 1 µg ClaI und Phosphatase behandelt und mit pHRi145-DNA (siehe oben) in einem 20 µl Reaktionsvolumen ligiert, welches 0,5 mM rATP (Sigma), 10 mM DTT (Calbiochem), 20 mM Tris•HCl (pH 7,8), 1 mM $MgCl_2$ und 800 Einheiten $T_4$ DNA-Ligase (Biolabs) enthält. Die Inkubation erfolgt bei 15°C während 2 Stunden. Die Ligase wird inaktiviert durch Inkubation bei 85°C während 10 Min. Anschliessend werden 2 Volumina Wasser hinzugegeben, die Kochsalz-Konzentration auf 10 mM eingestellt und 20 Einheiten KpnI (Biolabs) hinzugegeben während 30 Min. bei 37°C. Nach der Extraktion mit Phenol und Chloroform wird die Mischung durch ein 0,9 % niedrigschmelzendes Agarose-Gel (Biorad) in 40 mM Tris•Acetat (pH 7,8), 1 mM EDTA und 0,5 µg/ml Ethidiumbromid fraktioniert. Die durch UV-Bestrahlung sichtbare Bande, welche die gleiche Mobilität wie eine Marker-DNA gleicher Grösse aufzeigt, wird mit einem Skalpell herausgeschnitten. Das Gelstück wird 5 Min. bei 65°C geschmolzen und dann auf 37°C abgekühlt. Man erhält ein Volumen von ca. 20 µl. 5 µl dieser Lösung werden entommen und mit 400 Einheiten $T_4$-Ligase (Biolabs) in 10 µl Reaktionsvolumen, welches auf 0,5 mM ATP, 10 mM DTT, 10 mM $MgCl_2$, 20 mM Tris•HCl (pH 7,8) eingestellt wird, während 12 Stunden bei 15°C inkubiert. 1/10 Volumen einer Lösung mit 100 mM Tris•HCl (pH 7,5), 100 mM $CaCl_2$ und 100 mM $MgCl_2$ werden zum Ligase-Gemisch (solifidiziert bei 15°C) hinzugegeben und bei 65°C während 5 Min. inkubiert. Die Lösung wird dann verwendet, um mit Calcium behandelte E. coli HB101-Zellen, wie oben beschrieben, zu transformieren. Es wird auf McConkey-Agarplatten, supplementiert mit 50 µg/ml Ampicillin, ausplattiert.

Die Plasmid-DNAs von 10 verschiedenen Kolonien werden isoliert, wie oben beschrieben, und die DNA wird der folgenden Restriktionsenzymanalyse unterworfen: Je 0,5 µg Plasmid DNA wird nacheinander mit KpnI (Biolabs), NcoI (Biolabs) und EcoRI (Biolabs) nach den Vorschriften des Enzym-Herstellers gespalten. Die Spaltprodukte werden auf 1 % Agarose-Gelen in 40 mM Tris•Acetat (pH 7,8), 1 mM EDTA, 0,5 µg/ml Ethidiumbromid fraktioniert. Alle Plasmide zeigen je eine dieser Enzymspaltstellen, wie gewünscht. Eines wird als HRi148 bezeichnet.

Das Plasmid HRi148 enthält einen Tryptophan-Promotor-Operator und eine ribosomale Bindungsstelle bis und mit ATG und ist ein breit anwendbares Expressionsplasmid.

Stufe 2.15: 5 µg Plasmid-DNA von pHRi148 werden mit den Restriktionsendonucleasen EcoRI und BamHI, verdaut. Der herausgeschnittene Vektor pHRi148/EcoRI/BamHI wird mittels Dichtegradientenzentrifugation isoliert.

Die Herstellung des erhaltenen linearisierten Vektors ist auch beschrieben in "Hans Rink et al., Nucleic Acids Research 12, 6369-6387 (1984)", worin das in der vorliegenden Anmeldung als pHRi148 bezeichnete Plasmid pHR 148 genannt wird.

Stufe 2.16: 20 µg der gemäss Stufe 2.11 erhaltenen DNA-Sequenz $F_o$ werden in 20 µl einer Lösung von 50 µl 100 mM Tris•HCl (pH 7,5), 50 mM NaCl und 100 µg/ml Gelatine nacheinander mit den Restriktionsenzymen EcoRI und BamHI verdaut. Die Lösung wird auf TNE eingestellt, woraufhin 30 µg (= 50 nMol Enden) der Vektor-DNA pHRi148/EcoRI/BamHI zugegeben wird. Die Lösung wird mit Phenol/Chloroform extrahiert und die DNA mit Alkohol ausgefällt. Der DNA-Niederschlag wird in 20 µl einer Lösung von 50 mMol Tris•HCl (pH 7,8), 10 mMol $MgCl_2$, 10 mMol DTT, 0,5 mMol ATP und 100 µg/ml Gelatine mit 25 Einheiten/µl $T_4$-DNA-Ligase (Biolabs) bei 150° 3 Stunden behandelt. Auf diese Weise entsteht in der Lösung ein rekombiniertes Plasmid (pML1050).

Stufe 2.17: Die Transformation von E. coli HB101 mit dem gemäss Stufe 2.16 erhaltenen Plasmid pML1050 wird folgendermassen durchgeführt:

Die für die Transformation notwendigen mit Calcium vorbehandelten E. coli HB101 Zellen werden, wie von Mandel et al., J. Mol. Biol. 53, 159 (1970) beschrieben, hergestellt.

Die gemäss Stufe 2.16 erhaltene Lösung, welche das rekombinierte Plasmid pML1050 enthält, wird für 10 Min. auf 65° erhitzt, um die $T_4$-DNA Ligase zu inaktivieren und anschliessend auf 37° abgekühlt. 10 µl dieses Reaktionsgemisches werden zu 150 µl Calcium-behandelten E. coli HB101-Zellen in 10 mM $MgCl_2$ und 10 mM Tris•HCl (pH 7,5) in einem Gesamtvolumen von 200 µl gegeben.

Danach wird diese Mischung für 30 Min. in Eis gekühlt, 2 Min. auf 42°C erwärmt und dann 50 Min. in 1 ml L-Medium (vgl.Beginn des Beispiels 2) bei 37°C stehen lassen. Darauf wird die Mischung in Aliquoten à 0,2 ml auf 5 Agarplatten (McConkey-Agar, Difco), welche 60 µg/ml Ampicillin (Serva) enthalten, ausgestrichen. Die Agarplatten werden hernach bei 37°C für 16-18 Stunden gehalten. 109 Ampicillin-resistente Kolonien des transformierten E. coli HB101 werden erhalten.

Stufe 2.18: 30 der gemäss Stufe 2.17 erhaltenen transformierten Kolonien werden folgendermassen auf ihren Gehalt an einem Fragment der DNA-Sequenz $F_O$ gemäss Stufe 2.11 geprüft:

Die transformierten Kolonien werden auf Nitrozellulosefilter B85 (Schleicher und Schüll) abgedrückt. Nach Grunstein und Hogness [Proc. Natl. Acad. Sci. USA 72, 3961 (1979)] werden die Kolonien lysiert und ihre denaturierte DNA auf dem Filter fixiert. Anschliessend erfolgt eine Vorhybridisierung der Filter in 20 ml (pro Filter) 4xSET [= Lösung von 30 mM Tris•HCl (pH 8), 150 mM NaCl, 1 mM EDTA], 0,1 % (g/v) Ficoll 400 (Pharmacia), 0,5 % SDS, 50 µg/ml denaturierte Kalbsthymus-DNA für 4 Stunden bei 64 °C. Danach werden die Nitrozellulosefilter in 20 ml (pro Filter) 5xSET (g/v) Ficoll 400, 0,2 % SDS und 50 µg/ml denaturierter Kalbsthymus-DNA für 16 Stunden bei 64 °C mit der $^{32}P$-radioaktiv markierten Probe (ca. $10^3$-$10^4$ Cerencovcpm pro Filter) behandelt. Als Probe wird das Oligodesoxynucleotid 64/73 (vgl. Stufe 2.10) verwendet.

Anschliessend werden die Filter in 2xSET, 0,2 % SDS bei Raumtemperatur zweimal gewaschen, danach zweimal in 2xSET, 0,5 % SDS bei 60 °C (zuerst 30 Min., dann 60 Min. lang). Dann werden die Filter zwischen 3 MM Papier (Whatman) getrocknet und bei -80 °C auf einen Röntgen-Film (Fuji) mit einer Verstärkerfolie (Intensifying screen, Ilford) für 1-2 Tage gelegt.

Das erhaltene Autoradiogramm zeigt 20 positive Kolonien (Klone), die zur Weiterverarbeitung verwendet werden können, fünf davon erhalten die Bezeichnungen pML1050, pML1051, pML1052, pML1053 und pML1054.

Stufe 2.19: Die Charakterisierung der eingefügten DNA-Sequenz, d.h. des DNA-Inserts, im gemäss Stufe 2.18 erhaltenen Klon pML1050 erfolgt folgendermassen:

Die DNA des rekombinierten Plasmids pML1050 wird nach Ish-Horowitz isoliert (Molecular Cloning, A Laboratory Manual [Herausgeber: T. Maniatis et al.], Gold Spring Harbor Lab. 1982, S. 368). Die Nucleotidsequenz des $F_O$-DNA-Inserts wird nach Maxam und Gilbert bestimmt [Proc. Natl. Acad. Sci. USA 74, 560 (1977); siehe auch Meth. Enzym. 65, 499 (1980)]. Zu diesem Zweck werden 10 µg Plasmid-DNA von pML1050 mit EcoRI- bzw. BamHI-Restriktionsendonuclease gespalten und die linearisierten DNAs durch Gelelution aus Agarosegel isoliert. Zu diesem Zweck werden die linearisierten DNAs durch Gelelektrophorese an 1%iger niedrig schmelzender Agarose(Biorad) in Tris-Acetat-EDTA Puffer pH 8 gereinigt. Nach Anfärben der DNA im Agarosegel mit EtBr wird die Stelle des Gels, welche die DNA-Bande enthält, aus dem Gel ausgeschnitten und bei 65 ° 10 Min. verflüssigt. Zu dieser DNA-Lösung werden 20 Volumina TNE hinzugefügt, die DNA nach Mueller et al. [J. Mol. Biol. 124, 343 (1978)] durch DE-52 Chromatographie gereinigt, mit Phenol/Chloroform extrahiert und die DNA bei -20 °C über Nacht mit Alkohol ausgefällt. Der DNA-Niederschlag wird in 50 µl 0,01 M Tris•HCl (pH 8), 0,1 mM EDTA gelöst und bis zur Verwendung bei -20 °C aufbewahrt. Anschliessend werden die isolierten DNAs mit alkalischer Phosphatase verdaut und über DE-52 chromatographiert. Danach werden die DNAs am 5'-Ende mit [$\gamma$-$^{32}P$]ATP (spezifische Aktivität > 5000 Ci/mmol, Amersham) und $T_4$-Polynucleotid-Kinase (P-L-Biochemicals) radioaktiv markiert.

Die radioaktiv markierten DNAs werden dann mit einer zweiten Restriktionsendonuclease z.B. PvuII bzw. PstI gespalten. Die entstandenen DNA-Fragmente werden durch Gelelution aus Agarose isoliert. Dann wird vom PvuII-EcoRI*-Fragment bzw. PstI-Bam HI*-Fragment die Nucleotidsequenz der $F_O$-DNA, bestimmt. (* gibt das radioaktiv markierte DNA-Ende an.)

Beispiel 3: Test-Kit mit monoklonalen anti-CGRP II-Antikörpern zur Bestimmung von CGRP IIa, kompetitiver Radio-Immunoassay

a. Herstellung der monoklonalen Anti-CGRP II-Antikörper

A) Immunisierung von Mäusen

Reines CGRP II (3 mg) (hergestellt gemäss Beispiel 1) in lyophilisierter Form wird in wenig 0,1%iger Essigsäure gelöst und dann mit phosphatgepufferter Kochsalzlösung auf 3 ml ergänzt. Der pH wird auf 7,2 eingestellt. Portionen dieser Antigen-Lösung werden mit gleichen Mengen komplettem Freund's Adjuvans oder phosphatgepufferter Salzlösung gemischt.

Weibliche Balb/c Mäuse (8 Wochen alt, erhalten von der Tierfarm Sisseln, Schweiz) werden durch Injektion von 100 µg CGRP II-Lösung in gepufferter Salzlösung intravenös injiziert. Vier Tage später wird die Milz für die Fusion entnommen.

B) Herstellung der Hybridoma und Antikörper-Test

Die Herstellung der Hybridomazellen erfolgt durch Verschmelzung der erhaltenen Milzzellen mit der

Myeloma-Zellinie X63-Ag8.6.5.3 [J. F. Kearney et al., J. Immunol. 123, 1548 (1979)]. Dabei werden $10^8$ Milzzellen und $10^7$ Myelomazellen eingesetzt. Die Fusion wird wie beschrieben [S. Alkan et al., Mol. Immunol. 20, 203 (1983)] durchgeführt.

Die Bestimmung der anti-CGRP II-Aktivität in den Hybridoma-Ueberständen erfolgt mit Hilfe eines Radioimmunoassays [RIA, T. Chard, An Introduction to Radioimmunoassay and related Techniques, North-Holland Publ. Comp., Amsterdam 1978].

C) Isolierung und Reinigung der Anti-CGRP II-Antikörper aus Aszites

Balb/c Mäuse werden intraperitoneal mit 0,4 ml Pristan (Carl Roth) vorbehandelt. Nach einer Woche werden 2 bis $5x10^6$ klonierte Hybridoma-Zellen intraperitoneal injiziert. Aszitische Flüssigkeit wird von jeder Maus wiederholt genommen und bei -80° C gefroren. Die gesammelte Flüssigkeit wird aufgetaut und 30 Min. bei 4° mit 16 000 upm zentrifugiert. Das Fett wird abgesaugt und zum verbleibenden Debri-freien Ueberstand langsam unter Rühren bei 0° 0,9 Volumenäquivalente einer gesättigten Ammoniumsulfatlösung zugetropft. Die erhaltene rohe Immunoglobulin-Fraktion wird unter Verwendung von 0,1 m Tris•HCl (pH 8,2) durch Sephacryl G 2000 (Pharmacia), gemäss den Angaben des Herstellers, gegeben. Aktive Fraktionen werden vereinigt und mit Amicon XM50-Filter (Amicon) konzentriert.

b. Test-Kit für kompetitiven Radio-Immunoassay

Eine nach Beispiel 3aC) hergestellte Lösung von anti-CGRP II-Antikörpern wird mit phosphatgepufferter Salzlösung (PBS-Lösung) auf eine Konzentration von 1 µg pro 100 µl verdünnt. 100 µl dieser Lösung werden 2 Stunden bei 37° C in Plastik-Röhrchen oder auf Plastik-Mikrotiterplatt inkubiert, wobei Antikörper unspezifisch auf die Plastikoberfläche adsorbiert werden. Zur Absättigung der noch freien aktiven Stellen auf der Plastikoberfläche wird mit einer Bovinserum-Albumin-Lösung (BSA-Lösung) nachbehandelt.

Verdünnungsreihen einer Probelösung oder der Standardlösung in BSA-Lösung werden mit je 50 µl einer Lösung von nach bekannter Weise (29) mit radioaktivem $^{125}$Iod markiertem CGRP II der Aktivität 10 000 cpm pro 50 µl versetzt und dann auf der Plastikoberfläche 2 Stunden bei 37° C und anschliessend 12 Stunden bei 4° C inkubiert. Die Röhrchen oder Mikrotiterplatten werden mit phosphatgepufferter Salzlösung gewaschen und die Radioaktivität gemessen. Die Konzentration an CGRP II in der Probelösung wird durch eine mit Standardlösung gemessene Eichkurve bestimmt.

Ein Test-Kit für den beschriebenen Radio-Immunoassay enthält:

| | |
|---|---|
| 2 ml | Lösung von anti-CGRP II-Antikörper aus Beispiel 3aC) mit einer Konzentration von 1 bis 10 mg pro ml, |
| 100 ml | phosphatgepufferte Salzlösung (PBS-Lösung) |
| 100 ml | 0,3 % Bovinserum-Albumin und 0,1 % Natriumazid in PBS-Lösung (BSA-Lösung), |
| 2 ml | Lösung von radioaktivem CGRP II der Aktivität 200 000 cpm/ml, |
| 2 ml | Standardlösung enthaltend 100 ng/ml CGRP II, |
| 1 ml | Röhrchen oder Mikrotiterplatten aus Plastik. |

Beispiel 4: Gelatine Lösung

Eine sterilfiltrierte wässrige Lösung von CGRP II wird unter Erwärmen mit einer sterilen Gelatinelösung, welche als Konservierungsmittel Phenol enthält, unter aseptischen Bedingungen vermischt, so dass 1,0 ml Lösung folgende Zusammensetzung hat:

| | |
|---|---|
| CGRP II | 10,0 µg |
| Gelatine | 150,0 mg |
| Phenol | 4,7 mg |
| dest. Wasser bis zu | 1,0 ml. |

Die Mischung wird aseptisch in Vials zu 1,0 ml abgefüllt.

Beispiel 5: Sterile Trockensubstanz zur Injektion

man löst 5 µg CGRP II in 1 ml einer wässrigen Lösung mit 20 mg Mannit. Die Lösung wird sterilfiltriert und unter aseptischen Bedingungen in eine 2 ml Ampulle gefüllt, tiefgekühlt und lyophilisiert. Vor dem Gebrauch wird das Lyophilisat in 1 ml destilliertem Wasser oder 1 ml physiologischer Salzlösung gelöst. Die Lösung wird intramuskulär oder intravenös angewendet. Diese Formulierung kann auch in Doppelkammerspritzampullen abgefüllt werden.

Beispiel 6: Nasenspray

In einer Mischung von 3,5 ml "Miglyol 812" und 0,08 g Benzylalkohol werden 200 µg fein gemahlenes (<5,0 µ) CGRP II suspendiert. Diese Suspension wird in einen Behälter mit Dosierventil eingefüllt. Es werden nun 5,0 ml "Freon 12" unter Druck durch das Ventil in den Behälter gefüllt. Durch Schütteln wird das "Freon" in der Miglyol-Benzylalkoholmischung gelöst.

Beispiel 7: Eine Lösung von 2 ml 0,6 M Ammoniumacetat/0,1 M KCl/1 mM EDTA wird mit konz. Ammoniaklösung auf pH = 8 gestellt und mit 100 µl wässriger 1 M CGRP IIa-Lösung (vgl. Beispiel 2) gemischt. Dann werden 0,7 mg Carboxypeptidase Y (Carlsberg Biotechnology Ltd., Kopenhagen, Dänemark) zugegeben. Nach 30 Min. bei 25° C wird mit 6 M HCl bis pH = 1 angesäuert. Das Reaktionsprodukt wird mittels HPLC unter den in Stufe 1.7 angegebenen Bedingungen gereinigt und isoliert. Man erhält CGRP II der Formel III, identisch mit dem gemäss Beispiel 1 erhaltenen Produkt.

Beispiel 8: Analog Beispiel 2 erhält man durch Kultivierung von Escherichia coli-Klonen, die Plasmide mit der DNA-Sequenz gemäss Formel XVII

```
          MetAlaCysAsnThrAlaThrCysValThrHisArgLeuAlaGlyLeuLeuSer
d(GAATTCATGGCTTGCAACACCGCTACCTGCGTTACCCACCGTCTGGCTGGTCTGCTGTCT
d(CTTAAGTACCGAACGTTGTGGCGATGGACGCAATGGGTGGCAGACCGACCAGACGACAGA

  ArgSerGlyGlyMetValLysSerAsnPheValProThrAsnValGlySerLysAlaPhe
  CGTTCTGGTGGTATGGTTAAATCTAACTTCGTTCCGACCAACGTTGGTTCTAAAGCTTTC
  GCAAGACCACCATACCAATTTAGATTGAAGCAAGGCTGGTTGCAACCAAGATTTCGAAAG

  GlyNON
  GGTTAGGATCC)                                    (XVII)
  CCAATCCTAGG)
```

enthalten, und analoge Aufarbeitung CGRP IIb der Formel XIX.

```
    S--------------------S
    |                    |
Ala-Cy-Asn-Thr-Ala-Thr-Cy-Val-Thr-His-Arg-Leu-Ala-Gly-Leu-Leu-Ser-Arg-Ser-Gly-Gly-Met-Val-Lys-Ser-Asn-Phe-Val-Pro-        (XIX)

Thr-Asn-Val-Gly-Ser-Lys-Ala-Phe-Gly-OH
```

Die Ausgangsprodukte erhält man analog Beispiel 2.

Beispiel 9: 4 mg CGRP IIb (siehe Beispiel 8) werden bei 37° C in 1 ml 20 mM NaCl und 5 mM Natriumphosphat bei pH = 7 20 Stunden lang mit einem Amidierungsenzym, isoliert aus 100 g Schweinehypophysen, wie beschrieben in A.F. Bradbury, M.D.A. Finnie und D.G. Smyth, Nature 298, 686-688 (1982), inkubiert. Anschliessend wird mit 100 µl 1 M HCl angesäuert. Das Reaktionsprodukt wird mittels HPLC unter den in Stufe 1.7 angegebenen Bedingungen gereinigt und isoliert. Man erhält CGRP II der Formel III, identisch mit dem gemäss Beispiel 1 erhaltenen Produkt.

Beispiel 10: Das in Stufe 10.2 erhaltene Peptidamid der Formel

S——————————————S

Boc-Ala-Cy-Asn-Thr(tBu)-Ala-Thr(tBu)-Cy-Val-Thr(tBu)-
His-Arg(Mtr)-Leu-Ala-Gly-Leu-Leu-Ser(tBu)-Arg(Mtr)-Ser(tBu)-
Gly-Gly-Met-Val-Lys(Boc)-Ser(tBu)-Asn-Phe-Val-Pro-Thr(tBu)-
Asn-Val-Gly-Ser(tBu)-Lys(Boc)-Ala-Phe-$NH_2$

wird in 2,8 ml Trifluoressigsäure-Wasser (9:1) gelöst, nach 1 1/2 Stunden bei Raumtemperatur mit 30 ml kaltem Diethylether ausgefällt und abgenutscht. Das Nutschgut wird in 5,2 ml Trifluoressigsäure-Wasser (95:5) gelöst, 50 Minuten bei 50° belassen, auf Raumtemperatur gekühlt und nach Zugabe von 60 µl 1 M Ammoniumiodid 10 Minuten bei Raumtemperatur belassen. Durch Eintropfen in 30 ml kalten Diethylether wird das Peptidamid ausgefällt. Das abgenutschte und getrocknete Peptidamid wird in 3 ml 0,1 M Essigsäure gelöst und Spuren noch vorhandenen Iods werden durch Zugabe von 15 µl 1 M Natriumthiosulfat reduziert. Die erhaltene Lösung wird durch eine Ionenaustauscher-Säule (Amberlite® IRA 93, Acetatform 1,2x8 cm) filtriert und mit 0,1 M Essigsäure eluiert. Nach dem Eindampfen zur Trockne wird in 3 ml bidestilliertem Wasser gelöst, über Nacht unter Argon bei Raumtemperatur belassen und diese Lösung zur präparativen HPLC-Reinigung eingesetzt; Bedingungen: Säule: Vydac 5 µm C18, 250x10 mm (The Separations Group, Hesperia, California, USA), Gradient, bestehend aus den Lösungen A (0,1 Vol.% Trifluoressigsäure in Wasser) und 25-30 Vol.% B (0,1 Vol.% Trifluoressigsäure in Acetonitril) in 30 Minuten sowie A und 30-40 Vol.% B in 5 Minuten; Durchfluss: 4 ml/Minute; Detektion: 210 nm. Der Hauptpeak wird in Fraktionen aufgefangen und deren Reinheit durch analytisches HPLC bestimmt;
Bedingungen: Säule: Vydac 5 µm C18, 250x4,6 mm; Gradient aus A und B: 20-45 % B in 45 Minuten; A und B wie oben. Die reinen Fraktionen werden eingedampft. Die Trifluoressigsäure wird durch Ionenaustausch wie oben entfernt und CGRP II der Formel III durch Lyophilisieren erhalten; Retentionszeit im obigen analytischen HPLC: 24 Minuten, FAB/MS : $MH^+$ 3794 (berechnetes Molekulargewicht: 3793,4), $R_f$ (Kieselgel) = 0,57 (n-Butanol:Pyridin:Eisesssig:Wasser = 35:35:7:23), $R_f$ (Cellulose) = 0,64 (n-Butanol:Pyridin:Eisessig: Wasser = 38:24:8:30), $R_f$ (Cellulose) = 0,55 (n-Butanol:Pyridin: konz. Ammoniak:Wasser = 42:24:4:30).

Das Ausgangsmaterial erhält man folgendermassen:
Stufe 10.1: Das gemäss Beispiel 1, Stufe 1.4, erhaltene Peptid-Harz wird zwecks Abspaltung des geschützten Peptids vom Harz und zwecks Amidierung der terminalen Carboxylgruppe wie folgt behandelt: In einem Autoklaven werden 325 mg Peptid-Harz und 10 ml Dimethylformamid vorgelegt, bei -70° 6 g Ammoniak einkondensiert und 20 Stunden bei Raumtemperatur reagieren gelassen. Nach Abdestillieren des Ammoniaks wird die verbleibende Suspension mit 50 ml Diethylether versetzt. Die Fällung wird nach 2 Stunden bei 0° abgenutscht. Das geschützte Peptidamid wird mit Eisessig aus dem Harz extrahiert (fünfmal mit 5 ml), die Essigsäure durch Lyophilisieren entfernt und der Rückstand in 20 ml Methanol-Wasser (9:1) suspendiert. Nach Abzentrifugieren, Waschen und Trocknen erhält man 77 mg des geschützten Peptidamids.

Stufe 10.2: Eine Lösung von 69 mg des gemäss Stufe 10.1 erhaltenen Produkts in 14 ml Eisessig-Wasser (4:1) wird zwecks Ueberführung ins Disulfid in 4 Minuten unter Rühren bei Raumtemperatur zu einer Lösung von 164 mg Iod in 34 ml Eisessig und 16 ml Wasser getropft. Nach weiteren 10 Minuten wird die Reaktion durch Zugabe von 1,4 ml 1 M Natriumacetat und 0,68 ml 1 M Ascorbinsäure beendet. Die Salze werden auf einer Biogel P2 Säule (2,5x34 cm, in Eisessig-Wasser [6:4]) abgetrennt. Die peptidhaltige Fraktion wird nach Einengen lyophilisiert, der Rückstand in 5 ml Methanol suspendiert und das unlösliche, geschützte Peptidamid durch Zentrifugieren und Trocknen gewonnen.

Beispiel 11: 1 g H-AlA-Cys(MOB)-Asn(XANT)-Thr(BZL)-Ala-Thr(BZL)-Cys(MOB)-Val-Thr(BZL)-His(TOS)-Arg(TOS)-Leu-Ala-Gly-Leu-Leu-Ser(BZL)-Arg(TOS)-Ser(BZL)-Gly-Gly-Met-Val-Lys(2CZ))-Ser(BZL)-Asn-(XANT)-Phe-Val-Pro-Thr(BZL)-Asn(XANT)-Val-Gly-Ser(BZL)-Lys(2CZ)-Ala-Phe-MBHA-Harz und 1 ml Anisol wird in einer Teflon-Apparatur während 1 Stunde bei 0° mit 10 ml trockenem HF gerührt. Nach Abdestillieren des HF und Trocknen im Hochvakuum wird der Rückstand fünfmal mit 10 ml Diethylether extrahiert. Das Peptid wird mit entgaster 0,1 N Essigsäure aus dem Harz extrahiert, mit entgastem Wasser auf 500 ml verdünnt und der pH-Wert mit Ammoniak auf pH 8,4 gestellt. Nach Rühren über Nacht bei Raumtemperatur sind keine freien Mercaptogruppen mehr nachweisbar. Die Lösung wird stark eingeengt, lyophilisiert und der Rückstand durch präparative HPLC gereinigt, wie in Beispiel 10 beschrieben. Die erhaltenen Fraktionen werden im Dünnschichtchromatogramm im System n-Butanol-Pyridin-Eisessig-Wasser (35:35:7:23) auf Kieselgel auf ihre Reinheit geprüft ($R_f$ = 0,57) und nötigenfalls nochmals mit HPLC gereinigt. Die vereinigten reinen Fraktionen werden im Vakuum eingedampft und der Rückstand wird aus

Wasser lyophilisiert. Man erhält CGRP II der Formel III; $R_f$ (Cellulose) = 0,64 (n-Butanol:Pyridin:Eisessig:Wasser = 38:24:8:30).

Das Ausgangsmaterial wird wie folgt erhalten:

Stufe 11.1: 1 g MBHA-Polystyrol-Hydrochlorid (mit Divinylbenzol quervernetztes Polystyrol-Harz, worin einige Phenylgruppen einen Amino-(4-methyl-phenyl)-methyl-Substituenten tragen; Lieferfirma: Applied Biosystems, 850 Lincoln Centre Dr., Foster City, CA 94404, USA; Beladung 0,57 mMol/g) wird in der im Beispiel 1, Stufe 1.3 beschriebenen Maschine wie folgt behandelt (je ca. 20 ml):

| | |
|---|---|
| 3mal 0,5 Min. | Dimethylacetamid, entgast, |
| 3mal 2 Min. | 10 % Diisopropylethylamin in Dimethylacetamid, |
| 6mal 0,5 Min. | Dimethylacetamid, dest. |
| 1mal 120 Min. | Kupplung: 455 mg (1,7 mMol) Boc-Phe, 230 mg Hydroxybenzotriazol, 385 mg Dicyclohexylcarbodiimid in 0,36 ml Ethylenchlorid und 4 ml Dimethylacetamid: 120 Min. bei Raumtemperatur, |
| 1mal 5 Min. | 10 ml Acetanhydrid-Pyridin-Dimethylacetamid (10:10:80), |
| 1mal 0,5 Min. | Dimethylacetamid, entgast, |
| 1mal 1 Min. | Isopropanol, |
| 2mal 0,5 Min. | Ethylenchlorid, |
| 2mal 0,5 Min. | Dimethylacetamid, |
| 1mal 1 Min. | Isopropanol, |
| 3mal 0,5 Min. | Ethylenchlorid-Ethandithiol (99:1), |
| 2mal 5 Min., | 1mal 15 Minuten Trifluoressigsäure-Ethylenchlorid-Ethandithiol (50:49:1) zur Abspaltung der Boc-Gruppe, |
| 3mal 0,5 Min. | Ethylenchlorid-Ethandithiol (99:1). |

Darauf beginnt der Cyclus wieder von vorne.

Die folgenden Aminosäuren werden nacheinander ankondensiert:

Boc-Ala, Boc-Lys(2CZ), Boc-Ser(BZL), Boc-Gly, Boc-Val, Boc-Asn(XANT), Boc-Thr(BZL), Boc-Pro, Boc-Val, Boc-Phe, Boc-Asn(XANT), Boc-Ser(BZL), Boc-Lys(2CZ), Boc-Val, Boc-Met, Boc-Gly, Boc-Gly, Boc-Ser-(BZL), Boc-Arg(TOS), Boc-Ser(BZL), Boc-Leu, Boc-Leu, Boc-Gly, Boc-Ala, Boc-Leu, Boc-Arg(TOS), Boc-His(TOS), Boc-Thr(BZL), Boc-Val, Boc-Cys(MOB), Boc-Thr(BZL), Boc-Ala, Boc-Thr(BZL), Boc-Asn(XANT), Boc-Lys(MOB) und Boc-Ala. Die Vollständigkeit der Kupplungsreaktion wird mit dem Kaiser-Test qualitativ geprüft. Bei positivem Test wird die Kupplung wiederholt. Nach Abspaltung der Boc-Gruppe vom letzten Alanin wird das Harz im HV getrocknet.

## Patentansprüche

## Patentansprüche für folgende Vertragsstaaten : BE, CH, DE, FR, GB, IT, LI, LU, NL, SE

1. Peptide, die als Teilsequenz eines grösseren Peptids mit bis zu 90 Aminosäureresten oder ausschliesslich die Aminosäurensequenz der Formel I

   -AlaCysAsnThrAlaThrCysValThrHisArgLeuAlaGlyLeuLeuSer-
   ArgSerGlyGlyMetValLysSerAsnPheValProThrAsnValGlySer- (I)
   LysAlaPhe-

   aufweisen, wobei die Cysteinreste intra- oder intermolekulare Disulfidbrücken ausbilden können, und Derivate davon mit amidierter terminaler Carboxylgruppe und/oder acylierter terminaler Aminogruppe und ihre Salze.

2. Peptide nach Anspruch 1 mit bis zu 72 Aminosäureresten und Derivate davon mit C-terminaler Carbamoylgruppe und/oder N-terminaler Acetylaminogruppe und ihre Salze.

3. Peptidamid nach Anspruch 2 der Formel III und dessen Salze.

S——————————S
Ala-Cy-Asn-Thr-Ala-Thr-Cy-Val-Thr-His-Arg-Leu-Ala-Gly-Leu-
Leu-Ser-Arg-Ser-Gly-Gly-Met-Val-Lys-Ser-Asn-Phe-Val-Pro- (III)
Thr-Asn-Val-Gly-Ser-Lys-Ala-Phe-$NH_2$

**4.** Peptid nach Anspruch 2 der Formel XV und dessen Salze.

S——————————S
**Ala-Cy-Asn-Thr-Ala-Thr-Cy-Val-Thr-His-Arg-Leu-Ala-Gly-Leu-**
**Leu-Ser-Arg-Ser-Gly-Gly-Met-Val-Lys-Ser-Asn-Phe-Val-Pro- (XV)**
**Thr-Asn-Val-Gly-Ser-Lys-Ala-Phe-Tyr-OH**

**5.** Peptid nach Anspruch 2 der Formel XIX und dessen Salze.

S——————————S
**Ala-Cy-Asn-Thr-Ala-Thr-Cy-Val-Thr-His-Arg-Leu-Ala-Gly-Leu-**
**Leu-Ser-Arg-Ser-Gly-Gly-Met-Val-Lys-Ser-Asn-Phe-Val-Pro- (XIX)**
**Thr-Asn-Val-Gly-Ser-Lys-Ala-Phe-Gly-OH**

**6.** Eine synthetisch oder gentechnisch hergestellte Verbindung nach einem der Ansprüche 1-5 und ihre Salze.

**7.** Eine Verbindung nach einem der Ansprüche 1-5 in einer Reinheit von mehr als 50 % und ihre Salze.

**8.** Ein pharmazeutisch verwendbares Salz einer Verbindung nach einem der Ansprüche 1-7.

**9.** Ein Peptidamid nach einem der Ansprüche 1-3, 6 und 7 oder ein pharmazeutisch verwendbares Salz davon zur Anwendung in einem Verfahren zur therapeutischen Behandlung des menschlichen oder tierischen Körpers.

**10.** Pharmazeutische Präparate, die ein Peptidamid nach einem der Ansprüche 1-3 und 6 oder ein pharmazeutisch verwendbares Salz davon zusammen mit mehr als 95 Gewichtsprozent pharmazeutischem Trägermaterial enthalten.

**11.** Verwendung eines Peptidamids nach einem der Ansprüche 1-3, 6 und 7 oder eines pharmazeutisch verwendbaren Salzes davon zur Herstellung von pharmazeutischen Präparaten für die Anwendung zur Behandlung von Herzkreislaufstörungen.

**12.** Verfahren zur Herstellung eines Peptids, das als Teilsequenz eines grösseren Peptids oder ausschliesslich die Aminosäurensequenz der Formel I

-AlaCysAsnThrAlaThrCysValThrHisArgLeuAlaGlyLeuLeuSer-
ArgSerGlyGlyMetValLysSerAsnPheValProThrAsnValGlySer- (I)
LysAlaPhe-

aufweist, wobei die Cysteinreste intra- oder intermolekulare Disulfidbrücken ausbilden können, oder eines Derivats davon mit amidierter terminaler Carboxylgruppe und/oder acylierter terminaler Aminogruppe oder eines Salzes einer solchen Verbindung, dadurch gekennzeichnet, dass man

a) eine in einer solchen Verbindung vorhandene Amidbindung durch Umsetzung eines Fragments dieser Verbindung, das eine freie Carboxylgruppe aufweist, oder eines reaktionsfähigen Carbonsäurederivats davon mit dem komplementären Fragment, das eine freie Aminogruppe aufweist, oder mit einem reaktionsfähigen Derivat davon, wobei in den genannten Fragmenten freie funktionelle Gruppen mit Ausnahme der beiden an der Reaktion teilnehmenden Gruppen erforderlichenfalls in geschützter Form vorliegen, knüpft, und gegebenenfalls vorhandene Schutzgruppen abspaltet, oder

b) zur Herstellung einer der obengenannten Verbindungen, die eine intra- oder intermolekulare Disulfidbrücke aufweist, eine der obengenannten Verbindungen, worin die Mercaptogruppen der Cysteinreste in freier Form vorliegen oder worin die Mercaptogruppen der Cysteinreste durch Schutzgruppen geschützt sind, die unter den Reaktionsbedingungen abgespalten werden, mit einem geeigneten Oxidationsmittel oxidiert, oder

c) in einem solchen Peptid, Peptidamid oder N-acylierten Derivat davon, worin mindestens eine funktionelle Gruppe in geschützter Form vorliegt, die vorhandenen Schutzgruppen abspaltet, oder

d) zur Herstellung der obengenannten Peptide Wirtszellen, die mit einem Expressionsvektor transformiert sind, welches eine von einer Expressionskontrollsequenz regulierte, für die gewünschte Aminosäuresequenz kodierende DNA-Sequenz enthält, in einem flüssigen Nährmedium, welches assimilierbare Kohlenstoff- und Stickstoffquellen enthält, kultiviert, das Produkt erforderlichenfalls aus den Wirtszellen freisetzt und isoliert und, falls erforderlich, ein gegebenenfalls erhaltenes Di- oder Polymeres mit einem zur Aufspaltung von Disulfidbindungen geeigneten Reduktionsmittel umsetzt und nötigenfalls das erhaltene reduzierte Produkt mit einem zur Neuknüpfung von Disulfidbindungen geeigneten Oxidationsmittel behandelt, oder

e) zur Herstellung eines der obengenannten Peptidamide ein Peptid, welches zusätzlich zur Aminosäuresequenz des gewünschten Peptidamids C-terminal eine Aminosäure $AS^{\Omega}$ aufweist, die enzymatisch zur $NH_2$-Gruppe der terminalen Amidgruppe des gewünschten Peptidamids abgebaut werden kann oder die durch Ammoniak in Gegenwart eines geeigneten Enzyms substituiert werden kann, mit Hilfe eines geeigneten Enzyms gegebenenfalls in Anwesenheit von Ammoniak behandelt,

und, wenn erwünscht, nach Durchführung einer der Verfahrensvarianten a-e) ein erhaltenes Salz in die freie Verbindung überführt oder eine erhaltene freie Verbindung in ihr Salz überführt.

**13.** Eine DNA-Sequenz, die als Teilsequenz einer grösseren Sequenz mit bis zu 290 Desoxynukleotiden im einfachen Strang eine Sequenz der Formel IV enthält,

d(GCETGYAAYACXGCEACXTGYGTXACXCAYNGKYTZGCEGGXYTZYTZQRSNGK
AlaCysAsnThrAlaThrCysValThrHisArgLeuAlaGlyLeuLeuSerArg

(IV)

QRSGGXGGXATGGTXAAMQRSAAYTTLGTXCCXACXAAYGTXGGXQRSAAMGCETTL)
SerGlyGlyMetValLysSerAsnPheValProThrAsnValGlySerLysAlaPhe

worin nur die Desoxynukleotidsequenz des kodierenden Stranges, beginnend mit dem 5'-Ende, und darunter die durch die jeweiligen Tripletts kodierten Aminosäuren angegeben sind und worin

A = Adenosyl,
T = Thymidyl,

G = Guanosyl,
C = Cytidyl,
E = A, T, C oder G,
K = A, T, C oder G, wenn N = C, oder K = A oder G, wenn N = A,
L = T oder C,
M = A oder G,
N = A oder C,
Q = T oder A,
R,S: R = C und S = A, T, C oder G, wenn Q = T; oder R = G und S = T oder C, wenn Q = A,
X = A, T, C oder G,
Y = T oder C und
Z = A, T, C oder G, wenn Y = C, oder Z = A oder G, wenn Y = T,
bedeuten, wobei das vor der Klammer stehende "d" bedeutet, dass die in der Klammer genannten Nukleoside 2-Desoxyribo-nukleoside sind.

**14.** Eine DNA-Sequenz nach Anspruch 13 mit bis zu 150 Desoxynukleotiden im kodierenden Strang, die die DNA-Sequenz der Formel IVb enthält, wobei nur der kodierende Strang, beginnend mit dem 5'-Ende, angegeben ist und zum besseren Verständnis auch die Aminosäuren, für die jedes Triplett kodiert, genannt sind:

```
   AlaCysAsnThrAlaThrCysValThrHisArgLeuAlaGlyLeuLeuSerArg
 d(GCTTGCAACACCGCTACCTGCGTTACCCACCGTCTGGCTGGTCTGCTGTCTCGT
                                                                  (IVb)
   SerGlyGlyMetValLysSerAsnPheVaiProThrAsnValGlySerLysAlaPhe
   TCTGGTGGTATGGTTAAATCTAACTTCGTTCCGACCAACGTTGGTTCTAAAGCTTTC)
```

**15.** Mikroorganismen, die eine DNA Sequenz gemäss Anspruch 13 oder 14 enthalten und die zur Produktion eines Peptids nach einem der Ansprüche 1-5 befähigt sind.

**Patentansprüche für folgenden Vertragsstaat : AT**

**1.** Verfahren zur Herstellung eines Peptids, das als Teilsequenz eines grösseren Peptids oder ausschliesslich die Aminosäurensequenz der Formel I

```
-AlaCysAsnThrAlaThrCysValThrHisArgLeuAlaGlyLeuLeuSer-
ArgSerGlyGlyMetValLysSerAsnPheValProThrAsnValGlySer-        (I)
LysAlaPhe-
```

aufweist, wobei die Cysteinreste intra- oder intermolekulare Disulfidbrücken ausbilden können, oder eines Derivats davon mit amidierter terminaler Carboxylgruppe und/oder acylierter terminaler Aminogruppe oder eines Salzes einer solchen Verbindung, dadurch gekennzeichnet, dass man

a) eine in einer solchen Verbindung vorhandene Amidbindung durch Umsetzung eines Fragments dieser Verbindung, das eine freie Carboxy] gruppe aufweist, oder eines reaktionsfähigen Carbonsäurederivats davon mit dem komplementären Fragment, das eine freie Aminogruppe aufweist, oder mit einem reaktionsfähigen Derivat davon, wobei in den genannten Fragmenten freie funktionelle Gruppen mit Ausnahme dei beiden an der Reaktion teilnehmenden Gruppen erforderlichenfalls in geschützter Form vorliegen, knüpft, und gegebenenfalls vorhandene Schutzgruppen abspaltet, oder

b) zur Herstellung einer der obengenannten Verbindungen, die eine intra- oder intermolekulare Disulfidbrücke aufweist, eine der obengenannten Verbindungen, worin die Mercaptogruppen der Cysteinreste in freier Form vorliegen oder worin die Mercaptogruppen der Cysteinreste durch

Schutzgruppen geschützt sind, die unter den Reaktionsbedingungen abgespalten werden, mit einem geeigneten Oxidationsmittel oxidiert, oder

c) in einem solchen Peptid, Peptidamid oder N-acylierten Derivat davon, worin mindestens eine funktionelle Gruppe in geschützter Form vorliegt, die vorhandenen Schutzgruppen abspaltet, oder

d) zur Herstellung der obengenannten Peptide Wirtszellen, die mit einem Expressionsvektor transformiert sind, welches eine von einer Expressionskontrollsequenz regulierte, für die gewünschte Aminosäuresequenz kodierende DNA-Sequenz enthält, in einem flüssigen Nährmedium, welches assimilierbare Kohlenstoff- und Stickstoffquellen enthält, kultiviert, das Produkt erforderlichenfalls aus den Wirtszellen freisetzt und isoliert und, falls erforderlich, ein gegebenenfalls erhaltenes Di- oder Polymeres mit einem zur Aufspaltung von Disulfidbindungen geeigneten Reduktionsmittel umsetzt und nötigenfalls das erhaltene reduzierte Produkt mit einem zur Neuknüpfung von Disulfidbindungen geeigneten Oxidationsmittel behandelt, oder

e) zur Herstellung eines der obengenannten Peptidamide ein Peptid, welches zusätzlich zur Aminosäuresequenz des gewünschten Peptidamids C-terminal eine Aminosäure $AS^{\Omega}$ aufweist, die enzymatisch zur $NH_2$-Gruppe der terminalen Amidgruppe des gewünschten Peptidamids abgebaut werden kann oder die durch Ammoniak in Gegenwart eines geeigneten Enzyms substituiert werden kann, mit Hilfe eines geeigneten Enzyms gegebenenfalls in Anwesenheit von Ammoniak behandelt,

und, wenn erwünscht, nach Durchführung einer der Verfahrensvarianten a-e) ein erhaltenes Salz in die freie Verbindung überführt oder eine erhaltene freie Verbindung in ihr Salz überführt.

2. Verfahren nach Anspruch 1, dadurch gekennzeichnet, dass man die Ausgangsstoffe so wählt, dass man ein Peptid mit bis zu 72 Aminosäureresten oder ein Derivat davon mit C-terminaler Carbamoylgruppe und/oder N-terminaler Acetylaminogruppe oder ein Salz davon herstellt.

3. Verfahren nach Anspruch 2, dadurch gekennzeichnet, dass man die Ausgangsstoffe so wählt, dass man das Peptidamid der Formel III oder ein Salz davon herstellt.

S————————S

Ala-Cy-Asn-Thr-Ala-Thr-Cy-Val-Thr-His-Arg-Leu-Ala-Gly-Leu-Leu-Ser-Arg-Ser-Gly-Gly-Met-Val-Lys-Ser-Asn-Phe-Val-Pro- (III)

Thr-Asn-Val-Gly-Ser-Lys-Ala-Phe-$NH_2$

4. Verfahren nach Anspruch 2, dadurch gekennzeichnet, dass man die Ausgangsstoffe so wählt, dass man das Peptid der Formel XV oder ein Salz davon herstellt.

S————————S

Ala-Cy-Asn-Thr-Ala-Thr-Cy-Val-Thr-His-Arg-Leu-Ala-Gly-Leu-Leu-Ser-Arg-Ser-Gly-Gly-Met-Val-Lys-Ser-Asn-Phe-Val-Pro- (XV)

Thr-Asn-Val-Gly-Ser-Lys-Ala-Phe-Tyr-OH

5. Verfahren nach Anspruch 2, dadurch gekennzeichnet, dass man die Ausgangsstoffe so wählt, dass man das Peptid der Formel XIX oder ein Salz davon herstellt.

S——————————S

Ala-Cy-Asn-Thr-Ala-Thr-Cy-Val-Thr-His-Arg-Leu-Ala-Gly-Leu-
Leu-Ser-Arg-Ser-Gly-Gly-Met-Val-Lys-Ser-Asn-Phe-Val-Pro- (XIX)
Thr-Asn-Val-Gly-Ser-Lys-Ala-Phe-Gly-OH

6. Verfahren nach einem der Ansprüche 1-5, dadurch gekennzeichnet, dass man die Ausgangsstoffe so wählt, dass man ein pharmazeutisch verwendbares Salz herstellt.

7. Verfahren nach einem der Ansprüche 1-6, dadurch gekennzeichnet, dass die in Verfahrensvariante d) genannte DNA-Sequenz eine Sequenz der Formel IV enthält,

GCETGYAAYACXGCEACXTGYGTXACXCAYNGKYTZGCEGGXYTZYTZQRSNGK
AlaCysAsnThrAlaThrCysValThrHisArgLeuAlaGlyLeuLeuSerArg

(IV)

QRSGGXGGXATGGTXAAMQRSAAYTTLGTXCCXACXAAYGTXGGXQRSAAMGCETTL)
SerGlyGlyMetValLysSerAsnPheValProThrAsnValGlySerLysAlaPhe

worin nur die Desoxynukleotidsequenz des kodierenden Stranges, beginnend mit dem 5'-Ende, und darunter die durch die jeweiligen Tripletts kodierten Aminosäuren angegeben sind und worin

A = Adenosyl,
T = Thymidyl,
G = Guanosyl,
C = Cytidyl,
E = A, T, C oder G,
K = A, T, C oder G, wenn N = C, oder K = A oder G, wenn N = A,
L = T oder C,
M = A oder G,
N = A oder C,
Q = T oder A,
R,S: R = C und S = A, T, C oder G, wenn Q = T; oder R = G und S = T oder C, wenn Q = A,
X = A, T, C oder G,
Y = T oder C und
Z = A, T, C oder G, wenn Y = C, oder Z = A oder G, wenn Y = T,
bedeuten, wobei das vor der Klammer stehende "d" bedeutet, dass die in der Klammer genannten Nukleoside 2-Desoxyribo-nukleoside sind.

8. Verfahren nach Anspruch 7, dadurch gekennzeichnet, dass die in Verfahrensvariante d) genannte DNA-Sequenz die DNA-Sequenz der Formel IVb enthält, wobei nur der kodierende Strang, beginnend mit dem 5'-Ende, angegeben ist und zum besseren Verständnis auch die Aminosäuren, für die jedes Triplett kodiert, genannt sind:

AlaCysAsnThrAlaThrCysValThrHisArgLeuAlaGlyLeuLeuSerArg
d(GCTTGCAACACCGCTACCTGCGTTACCCACCGTCTGGCTGGTCTGCTGTCTCGT

(IVb)

SerGlyGlyMetValLysSerAsnPheValProThrAsnValGlySerLysAlaPhe
TCTGGTGGTATGGTTAAATCTAACTTCGTTCCGACCAACGTTGGTTCTAAAGCTTTC)

## Claims

### Claims for the following Contracting States : BE, CH, DE, FR, GB, IT, LI, LU, NL, SE

**1.** A peptide that has, as a partial sequence of a larger peptide having up to 90 amino acid residues, or exclusively, the amino acid sequence of the formula I

-AlaCysAsnThrAlaThrCysValThrHisArgLeuAlaGlyLeuLeuSer-
ArgSerGlyGlyMetValLysSerAsnPheValProThrAsnValGlySer-
LysAlaPhe- (I)

in which the cysteine residues can form intra- or inter-molecular disulphide bridges, or a derivative thereof having an amidated terminal carboxy group and/or an acylated terminal amino group, or a salt thereof.

**2.** A peptide according to claim 1 having up to 72 amino acid residues, or a derivative thereof having a C-terminal carbamoyl group and/or an N-terminal acetylamino group, or a salt thereof.

**3.** A peptide-amide according to claim 2 of the formula III or a salt thereof

S————————S
Ala-Cy-Asn-Thr-Ala-Thr-Cy-Val-Thr-His-Arg-Leu-Ala-Gly-Leu-
Leu-Ser-Arg-Ser-Gly-Gly-Met-Val-Lys-Ser-Asn-Phe-Val-Pro-
Thr-Asn-Val-Gly-Ser-Lys-Ala-Phe-$NH_2$ (III).

**4.** A peptide according to claim 2 of the formula XV or a salt thereof

S————————S
Ala-Cy-Asn-Thr-Ala-Thr-Cy-Val-Thr-His-Arg-Leu-Ala-Gly-Leu-
Leu-Ser-Arg-Ser-Gly-Gly-Met-Val-Lys-Ser-Asn-Phe-Val-Pro-
Thr-Asn-Val-Gly-Ser-Lys-Ala-Phe-Tyr-OH (XV).

**5.** A peptide according to claim 2 of the formula XIX or a salt thereof

S——————————S

Ala-Cy-Asn-Thr-Ala-Thr-Cy-Val-Thr-His-Arg-Leu-Ala-Gly-Leu-Leu-Ser-Arg-Ser-Gly-Gly-Met-Val-Lys-Ser-Asn-Phe-Val-Pro-Thr-Asn-Val-Gly-Ser-Lys-Ala-Phe-Gly-OH (XIX).

**6.** A synthetically or genetically produced compound according to any one of claims 1 to 5 or a salt thereof.

**7.** A compound according to any one of claims 1 to 5 in a purity of more than 50 % or a salt thereof.

**8.** A pharmaceutically acceptable salt of a compound according to any one of claims 1 to 7.

**9.** A peptide-amide according to any one of claims 1 to 3, 6 and 7 or a pharmaceutically acceptable salt thereof for use in a method for the therapeutic treatment of the human or animal body.

**10.** A pharmaceutical composition that comprises a peptide-amide according to any one of claims 1 to 3 and 6 or a pharmaceutically acceptable salt thereof together with more than 95 % by weight of a pharmaceutical carrier.

**11.** The use of a peptide-amide according to any one of claims 1 to 3, 6 and 7 or a pharmaceutically acceptable salt thereof for the preparation of a pharmaceutical composition for use in the treatment of coronary circulation disorders.

**12.** A process for the preparation of a peptide that has, as a partial sequence of a larger peptide, or exclusively, the amino acid sequence of the formula I

-AlaCysAsnThrAlaThrCysValThrHisArgLeuAlaGlyLeuLeuSer-

ArgSerGlyGlyMetValLysSerAsnPheValProThrAsnValGlySer-

LysAlaPhe- (I)

in which the cysteine residues can form intra- or inter-molecular disulphide bridges, or of a derivative thereof having an amidated terminal carboxy group and/or an acylated terminal amino group, or of a salt of such a compound, characterised in that

a) an amide bond present in such a compound is linked by reaction of a fragment of that compound that has a free carboxy group, or of a reactive carboxylic acid derivative thereof, with the complementary fragment that has a free amino group, or with a reactive derivative thereof, wherein free functional groups in the mentioned fragments, with the exception of the two groups participating in the reaction, are, if necessary, in protected form, and protecting groups which may be present are removed, or

b) for the preparation of one of the above-mentioned compounds that has an intra- or inter-molecular disulphide bridge, one of the above-mentioned compounds in which the mercapto groups of the cysteine residues are in free form, or in which the mercapto groups of the cysteine residues are protected by protecting groups that are removed under the reaction conditions, is oxidised with a suitable oxidising agent, or

c) in such a peptide, peptide-amide or N-acylated derivative thereof in which at least one functional group is in protected form, the protecting groups present are removed, or

d) for the preparation of the above-mentioned peptides, host cells transformed with an expression vector comprising a DNA sequence that codes for the desired amino acid sequence and that is regulated by an expression control sequence are cultivated in a liquid nutrient medium comprising assimilable carbon and nitrogen sources, the product is, if necessary, freed from the host cells and isolated and, if necessary, a dimer or polymer which may be obtained is reacted with a reducing

agent suitable for splitting disulphide bonds and, if necessary, the resulting reduced product is treated with an oxidising agent suitable for the new linking of disulphide bonds, or

e) for the preparation of one of the above-mentioned peptide-amides, a peptide that in addition to the amino acid sequence of the desired peptide-amide has C-terminally an amino acid $AS^{\Omega}$ that can be degraded enzymatically to the $NH_2$ group of the terminal amide group of the desired peptide-amide or that can be substituted by ammonia in the presence of a suitable enzyme, is treated by means of a suitable enzyme optionally in the presence of ammonia,

and, if desired, after carrying out one of the process variants a-e), a resulting salt is converted into the free compound or a resulting free compound is converted into its salt.

**13.** A DNA sequence that comprises, as a partial sequence of a larger sequence having up to 290 deoxynucleotides in a single strand, a sequence of the formula IV

```
d(GCETGYAAYACXGCEACXTGYGTXACXCAYNGKYTZGCEGGXYTZYTZQRSNGK
  AlaCysAsnThrAlaThrCysValThrHisArgLeuAlaGlyLeuLeuSerArg

                                                                  (IV)
  QRSGGXGGXATGGTXAAMQRSAAYTTLGTXCCXACXAAYGTXGGXQRSAAMGCETTL)
  SerGlyGlyMetValLysSerAsnPheValProThrAsnValGlySerLysAlaPhe
```

in which only the deoxynucleotide sequence of the coding strand, commencing with the 5'-end and, beneath that, the amino acids for which each triplet codes, are indicated, and in which

A = adenosyl,
T = thymidyl,
G = guanosyl,
C = cytidyl,
E = A, T, C or G,
K = A, T, C or G when N = C, or
K = A or G when N = A,
L = T or C,
M = A or G,
N = A or C,
Q = T or A,
R,S: R = C and S = A, T, C or G when Q = T, or R = G and S = T or C when Q = A,
X = A, T, C or G,
Y = T or C and
Z = A, T, C or G when Y = C, or
Z = A or G when Y = T,

wherein the "d" standing before the bracket means that the nucleosides mentioned in the brackets are 2-deoxyribonucleosides.

**14.** A DNA sequence according to claim 13 having up to 150 deoxynucleotides in the coding strand and comprising the DNA sequence of the formula IVb, wherein only the coding strand, commencing with the 5'-end, is indicated and, for the purpose of better understanding, the amino acids for which each triplet codes are also mentioned:

AlaCysAsnThrAlaThrCysValThrHisArgLeuAlaGlyLeuLeuSerArg
d(GCTTGCAACACCGCTACCTGCGTTACCCACCGTCTGGCTGGTCTGCTGTCTCGT

(IVb).

SerGlyGlyMetValLysSerAsnPheVaiProThrAsnValGlySerLysAlaPhe
TCTGGTGGTATGGTTAAATCTAACTTCGTTCCGACCAACGTTGGTTCTAAAGCTTTC)

**15.** A microorganism that comprises a DNA sequence according to claim 13 or 14 and that is capable of producing a peptide according to any one of claims 1 to 5.

**Claims for the following Contracting State : AT**

**1.** A process for the preparation of a peptide that has, as a partial sequence of a larger peptide, or exclusively, the amino acid sequence of the formula I

-AlaCysAsnThrAlaThrCysValThrHisArgLeuAlaGlyLeuLeuSer-
ArgSerGlyGlyMetValLysSerAsnPheValProThrAsnValGlySer-
LysAlaPhe- (I)

in which the cysteine residues can form intra- or inter-molecular disulphide bridges, or of a derivative thereof having an amidated terminal carboxy group and/or an acylated terminal amino group, or of a salt of such a compound, characterised in that

a) an amide bond present in such a compound is linked by reaction of a fragment of that compound that has a free carboxy group, or of a reactive carboxylic acid derivative thereof, with the complementary fragment that has a free amino group, or with a reactive derivative thereof, wherein free functional groups in the mentioned fragments, with the exception of the two groups participating in the reaction, are, if necessary, in protected form, and protecting groups which may be present are removed, or

b) for the preparation of one of the above-mentioned compounds that has an intra- or inter-molecular disulphide bridge, one of the above-mentioned compounds in which the mercapto groups of the cysteine residues are in free form, or in which the mercapto groups of the cysteine residues are protected by protecting groups that are removed under the reaction conditions, is oxidised with a suitable oxidising agent, or

c) in such a peptide, peptide-amide or N-acylated derivative thereof in which at least one functional group is in protected form, the protecting groups present are removed, or

d) for the preparation of the above-mentioned peptides, host cells transformed with an expression vector comprising a DNA sequence that codes for the desired amino acid sequence and that is regulated by an expression control sequence are cultivated in a liquid nutrient medium comprising assimilable carbon and nitrogen sources, the product is, if necessary, freed from the host cells and isolated and, if necessary, a dimer or polymer which may be obtained is reacted with a reducing agent suitable for splitting disulphide bonds and, if necessary, the resulting reduced product is treated with an oxidising agent suitable for the new linking of disulphide bonds, or

e) for the preparation of one of the above-mentioned peptide-amides, a peptide that in addition to the amino acid sequence of the desired peptide-amide has C-terminally an amino acid $AS^{\Omega}$ that can be degraded enzymatically to the $NH_2$ group of the terminal amide group of the desired peptide-amide or that can be substituted by ammonia in the presence of a suitable enzyme, is treated by means of a suitable enzyme optionally in the presence of ammonia,

and, if desired, after carrying out one of the process variants a-e), a resulting salt is converted into the free compound or a resulting free compound is converted into its salt.

**2.** A process according to claim 1, characterised in that the starting materials are so selected that a peptide having up to 72 amino acid residues or a derivative thereof having a C-terminal carbamoyl group and/or an N-terminal acetylamino group, or a salt thereof, is prepared.

**3.** A process according to claim 2, characterised in that the starting materials are so selected that the peptide-amide of the formula III or a salt thereof is prepared

```
     S--------------------S
     |                    |
Ala-Cy-Asn-Thr-Ala-Thr-Cy-Val-Thr-His-Arg-Leu-Ala-Gly-Leu-
Leu-Ser-Arg-Ser-Gly-Gly-Met-Val-Lys-Ser-Asn-Phe-Val-Pro-
Thr-Asn-Val-Gly-Ser-Lys-Ala-Phe-NH2                    (III).
```

**4.** A process according to claim 2, characterised in that the starting materials are so selected that the peptide of the formula XV or a salt thereof is prepared

```
     S--------------------S
     |                    |
Ala-Cy-Asn-Thr-Ala-Thr-Cy-Val-Thr-His-Arg-Leu-Ala-Gly-Leu-
Leu-Ser-Arg-Ser-Gly-Gly-Met-Val-Lys-Ser-Asn-Phe-Val-Pro-
Thr-Asn-Val-Gly-Ser-Lys-Ala-Phe-Tyr-OH                  (XV).
```

**5.** A process according to claim 2, characterised in that the starting materials are so selected that the peptide of the formula XIX or a salt thereof is prepared

```
     S--------------------S
     |                    |
Ala-Cy-Asn-Thr-Ala-Thr-Cy-Val-Thr-His-Arg-Leu-Ala-Gly-Leu-
Leu-Ser-Arg-Ser-Gly-Gly-Met-Val-Lys-Ser-Asn-Phe-Val-Pro-
Thr-Asn-Val-Gly-Ser-Lys-Ala-Phe-Gly-OH                  (XIX).
```

**6.** A proces according to any one of claims 1 to 5, characterized in that the starting materials are so selected that a pharmaceutically acceptable salt is prepared.

**7.** A process according to any one of claims 1 to 6, characterised in that the DNA sequence mentioned in process variant d) comprises a sequence of the formula IV

```
d(GCETGYAAYACXGCEACXTGYGTXACXCAYNGKYTZGCEGGXYTZYTZQRSNGK
  AlaCysAsnThrAlaThrCysValThrHisArgLeuAlaGlyLeuLeuSerArg

  QRSGGXGGXATGGTXAAMQRSAAYTTLGTXCCXACXAAYGTXGGXQRSAAMGCETTL)   (IV)
  SerGlyGlyMetValLysSerAsnPheValProThrAsnValGlySerLysAlaPhe
```

in which only the deoxynucleotide sequence of the coding strand, commencing with the 5'-end and,

beneath that, the amino acids for which each triplet codes, are indicated, and in which
A = adenosyl,
T = thymidyl,
G = guanosyl,
C = cytidyl,
E = A, T, C or G,
K = A, T, C or G when N = C, or
K = A or G when N = A,
L = T or C,
M = A or G,
N = A or C,
Q = T or A,
R,S: R = C and S = A, T, C or G when Q = T, or R = G and S = T or C when Q = A,
X = A, T, C or G,
Y = T or C and
Z = A, T, C or G when Y = C, or
Z = A or G when Y = T,
wherein the "d" standing before the bracket means that the nucleosides mentioned in the brackets are 2-deoxyribonucleosides.

**8.** A process according to claim 7, characterised in that the DNA sequence mentioned in process variant d) comprises the DNA sequence of the formula IVb, wherein only the coding strand, commencing with the 5'-end, is indicated and, for the purpose of better understanding, the amino acids for which each triplet codes are also mentioned:

```
 AlaCysAsnThrAlaThrCysValThrHisArgLeuAlaGlyLeuLeuSerArg
d(GCTTGCAACACCGCTACCTGCGTTACCCACCGTCTGGCTGGTCTGCTGTCTCGT
                                                            (IVb).
 SerGlyGlyMetValLysSerAsnPheValProThrAsnValGlySerLysAlaPhe
 TCTGGTGGTATGGTTAAATCTAACTTCGTTCCGACCAACGTTGGTTCTAAAGCTTTC)
```

## Revendications

## Revendications pour lest Etats contractants suivants : BE, CH, DE, FR, GB, IT, LI, LU, NL, SE

**1.** Peptides qui contiennent comme séquence partielle d'un plus grand peptide ayant jusqu'à 90 esters d'acides aminés ou exclusivement la séquence d'acides aminés de formule I

```
-AlaCysAsnThrAlaThrCysValThrHisArgLeuAlaGlyLeuLeuSer-
ArgSerGlyGlyMetValLysSerAsnPheValProThrAsnValGlySer-        (I)
LysAlaPhe-
```

où les radicaux cystéine peuvent former des ponts disulfure de façon intra- ou intermoléculaire et leurs dérivés à acide carboxyle terminal amidé et/ou à groupe amino terminal acylé et leurs sels.

**2.** Peptides selon la revendication 1, avec jusqu'à 72 radicaux acides aminés et leurs dérivés à groupe carbamoyle C-terminal et/ou groupe acétylamino N-terminal et leurs sels.

**3.** Peptidamide selon la revendication 2 de formule III et ses sels.

S—————————S

Ala-Cy-Asn-Thr-Ala-Thr-Cy-Val-Thr-His-Arg-Leu-Ala-Gly-Leu-
Leu-Ser-Arg-Ser-Gly-Gly-Met-Val-Lys-Ser-Asn-Phe-Val-Pro- (III)
Thr-Asn-Val-Gly-Ser-Lys-Ala-Phe-$NH_2$

**4.** Peptide selon la revendication 2 de formule XV et ses sels.

S—————————S

Ala-Cy-Asn-Thr-Ala-Thr-Cy-Val-Thr-His-Arg-Leu-Ala-Gly-Leu-
Leu-Ser-Arg-Ser-Gly-Gly-Met-Val-Lys-Ser-Asn-Phe-Val-Pro- (XV)
Thr-Asn-Val-Gly-Ser-Lys-Ala-Phe-Tyr-OH

**5.** Peptide de la revendication 2 de formule XIX et ses sels.

S—————————S

Ala-Cy-Asn-Thr-Ala-Thr-Cy-Val-Thr-His-Arg-Leu-Ala-Gly-Leu-
Leu-Ser-Arg-Ser-Gly-Gly-Met-Val-Lys-Ser-Asn-Phe-Val-Pro- (XIX)
Thr-Asn-Val-Gly-Ser-Lys-Ala-Phe-Gly-OH

**6.** Composé préparé par synthèse ou ingéniérie génétique selon l'une des revendications 1-5 et ses sels.

**7.** Composé selon l'une des revendications 1-5 à une pureté de plus de 50% et ses sels.

**8.** Sel pharmaceutiquement acceptable d'un composé selon l'une des revendications 1-7.

**9.** Peptidamide selon l'une des revendications 1-3, 6 et 7 ou un de ses sels pharmaceutiquement acceptables aux fins d'application dans un procédé de traitement thérapeutique du corps humain ou animal.

**10.** Préparations pharmaceutiques qui contiennent un peptidamide selon l'une des revendications 1-3 et 6 ou un de leurs sels pharmaceutiquement acceptables avec plus de 95% en poids de support pharmaceutique.

**11.** Application d'un peptidamide selon l'une des revendications 1-3, 6 et 7 ou d'un de ses sels pharmaceutiquement acceptables à la préparation de préparations pharmaceutiques pour l'application au traitement des désordres de la circulation cardiaque.

**12.** Procédé de préparation d'un peptide qui présente comme séquence partielle d'un plus grand peptide ou exclusivement la séquence d'acides aminés de formule I

-AlaCysAsnThrAlaThrCysValThrHisArgLeuAlaGlyLeuLeuSer-
ArgSerGlyGlyMetValLysSerAsnPheValProThrAsnValGlySer- (I)
LysAlaPhe-

où les radicaux cystéine peuvent former des ponts disulfure de façon intra- ou intermoléculaire, ou un de ses dérivés avec groupe carboxyle terminal amidé et/ou groupe amino terminal acylé ou d'un sel d'un tel composé, caractérisé en ce que

a) on relie une liaison amide présente dans un tel composé par réaction d'un fragment de ce composé, qui présente un groupe carboxyle, ou d'un de ses dérivés d'acide carboxylique réactif avec le fragment complémentaire, qui présente un groupe amino, ou avec un de ses dérivés réactifs, où dans les fragments mentionnés les groupes fonctionnels libres se présentent à l'exception de deux groupes participant à la réaction, si nécessaire sous forme protégée, et on sépare les groupes protecteurs éventuellement présents, ou

b) pour préparer un des composés mentionnés ci-dessus, qui présente un pont disulfure intra- ou intermoléculaire, on oxyde avec un oxydant approprié l'un des composés mentionnés ci-dessus, où les groupes mercapto des radicaux cystéine se présentent sous forme libre ou bien où les groupes mercapto des radicaux cystéine sont protégés par des groupes protecteurs qui sont séparés dans les conditions de la réaction, ou

c) dans un tel peptide, peptidamide ou un de leurs dérivés N-acylés, où au moins un groupe fonctionnel est présent sous forme protégée, on sépare les groupes protecteurs présents, ou

d) pour préparer les peptides mentionnés ci-dessus, on cultive des cellules hôtes qui sont transformées avec un vecteur d'expression, lequel contient une séquence d'ADN réglée par une séquence de commande d'expression, codant pour la séquence d'acides aminés désirée, dans un milieu nutritif liquide qui contient des sources de carbone et d'azote assimilables, on libère si nécessaire le produit à partir des cellules hôtes et on l'isole et, si nécessaire, on fait réagir un di- ou polymère obtenu avec un réducteur approprié au clivage des liaisons disulfure et si nécessaire, on traite le produit réduit obtenu avec un oxydant approprié pour reconstituer les liaisons disulfure, ou

e) pour préparer un des peptidamides mentionnés ci-dessus, on traite un peptide qui présente outre la séquence d'acides aminés du peptidamide désiré, en position C-terminale un acide aminé $AS^{\Omega}$, qui peut être dégradé enzymatique en groupe $NH_2$ du groupe amide terminal du peptidamide désiré ou qui peut être substitué par de l'ammoniaque en présence d'une enzyme appropriée, à l'aide d'une enzyme appropriée, éventuellement en présence d'ammoniaque,

et, si on le désire, en ce que après réalisation d'une des étapes a-e) du procédé on transforme un sel en le composé libre ou un composé libre en son sel.

**13.** Séquence d'ADN qui contient comme séquence partielle d'une plus grande séquence ayant jusqu'à 290 désoxynucléotides dans un seul brin une séquence de formule IV

```
d(GCCETGYAAYACXGCEACXTGYGTXACXCAYNGKYTZGCEGGXYTZYTZQRSNGK
  AlaCysAsnThrAlaThrCysValThrHisArgLeuAlaGlyLeuLeuSerArg
                                                              (IV)
  QRSGGXGGXATGGTXAAMQRSAAYTTLGTXCCXACXAAYGTXGGXQRSAAMGCETTL)
  SerGlyGlyMetValLysSerAsnPheValProThrAsnValGlySerLysAlaPhe
```

dans laquelle seule la séquence désoxynucléotide du brin codant, commençant par l'extrémité 5' et à l'intérieur de celle-ci les acides aminés codés par les triplets correspondants sont indiqués et où

A = adénosyle,
T = thymidyle,
G = guanosyle,
C = cytidyle,
E = A, T, C ou G,
K = A, T, C ou G, lorsque N = C, ou K = A ou G, lorsque N = A,
L = T ou C,
M = A ou G,
N = A ou C,
Q = T ou A,
R,S: R = C et S = A, T, C ou G, lorsque Q = T ; ou R = G et S = T ou C, lorsque Q = A,
X = A, T, C ou G,

Y = T ou C et
Z = A, T, C ou G, lorsque Y = C, ou Z = A, ou G, lorsque Y = T,
où le "d" situé en avant des parenthèses signifie que les nucléosides mentionnés dans la parenthèse sont des 2-désoxyribo-nucléosides.

**14.** Séquence d'ADN selon la revendication 13 avec jusqu'à 150 désoxynucléotides dans le brin codant, qui contient la séquence d'ADN de formule IVb où seul le brin codant, commençant par l'extrémité 5' est indiqué, et aux fins de meilleure compréhension, également les acides aminés pour lesquels code chaque triplet sont mentionnés :

```
  AlaCysAsnThrAlaThrCysValThrHisArgLeuAlaGlyLeuLeuSerArg
d(GCTTGCAACACCGCTACCTGCGTTACCCACCGTCTGGCTGGTCTGCTGTCTCGT
                                                              (IVb)
  SerGlyGlyMetValLysSerAsnPheVaiProThrAsnValGlySerLysAlaPhe
  TCTGGTGGTATGGTTAAATCTAACTTCGTTCCGACCAACGTTGGTTCTAAAGCTTTC)
```

**15.** Microorganismes qui contiennent une séquence d'ADN selon la revendication 13 ou 14 et qui conviennent pour la production d'un peptide selon l'une des revendications 1-15.

**Revendications pour l'Etat contractant suivant : AT**

**1.** Procédé de préparation d'un peptide, qui contient comme séquence partielle d'un plus grand peptide ayant jusqu'à 90 esters d'acides aminés ou exclusivement la séquence d'acides aminés de formule I

```
-AlaCysAsnThrAlaThrCysValThrHisArgLeuAlaGlyLeuLeuSer-
ArgSerGlyGlyMetValLysSerAsnPheValProThrAsnValGlySer-        (I)
LysAlaPhe-
```

où les radicaux cystéine peuvent former des ponts disulfure de façon intra- ou intermoléculaire et leurs dérivés à acide carboxyle terminal amidé et/ou à groupe amino terminal acylé et leurs sels ou d'un sel d'un tel composé, caractérisé en ce que

a) on relie une liaison amide présente dans un tel composé par réaction d'un fragment de ce composé, qui présente un groupe carboxyle, ou d'un de ses dérivés d'acide carboxylique réactif avec le fragment complémentaire, qui présente un groupe amino, ou avec un de ses dérivés réactifs, où dans les fragments mentionnés les groupes fonctionnels libres se présentent à l'exception de deux groupes participant à la réaction, si nécessaire sous forme protégée, et on sépare les groupes protecteurs éventuellement présents, ou

b) pour préparer un des composés mentionnés ci-dessus, qui présente un pont disulfure intra- ou intermoléculaire, on oxyde avec un oxydant approprié l'un des composés mentionnés ci-dessus, où les groupes mercapto des radicaux cystéine se présentent sous forme libre ou bien où les groupes mercapto des radicaux cystéine sont protégés par des groupes protecteurs qui sont séparés dans les conditions de la réaction, ou

c) dans un tel peptide, peptidamide ou un de leurs dérivés N-acylés, où au moins un groupe fonctionnel est présent sous forme protégée, on sépare les groupes protecteurs présents, ou

d) pour préparer les peptides mentionnés ci-dessus, on cultive des cellules hôtes qui sont transformées avec un vecteur d'expression, lequel contient une séquence d'ADN réglée par une séquence de commande d'expression, codant pour la séquence d'acides aminés désirée, dans un milieu nutritif liquide qui contient des sources de carbone et d'azote assimilables, on libère si

nécessaire le produit à partir des cellules hôtes et on l'isole et, si nécessaire, on fait réagir un di- ou polymère obtenu avec un réducteur approprié au clivage des liaisons disulfure et si nécessaire, on traite le produit réduit obtenu avec un oxydant approprié pour reconstituer les liaisons disulfure, ou

e) pour préparer un des peptidamides mentionnés ci-dessus, on traite un peptide qui présente outre la séquence d'acides aminés du peptidamide désiré, en position C-terminale un acide aminé $AS^{\Omega}$, qui peut être dégradé enzymatique en groupe $NH_2$ du groupe amide terminal du peptidamide désiré ou qui peut être substitué par de l'ammoniaque en présence d'une enzyme appropriée, à l'aide d'une enzyme appropriée, éventuellement en présence d'ammoniaque,

et, si on le désire, en ce que après réalisation d'une des étapes a-e) du procédé on transforme un sel en le composé libre ou un composé libre en son sel.

2. Procédé selon la revendication 1, caractérisé en ce qu'on choisit les produits de départ de manière à préparer un peptide ayant jusqu'à 72 esters d'acides aminés ou un de ses dérivés avec groupe carbamoyle C-terminal et/ou groupe acétylamino N-terminal ou un de ses sels.

3. Procédé selon la revendication 2, caractérisé en ce qu'on choisit les produits de départ de manière à préparer le peptidamide de formule III ou un de ses sels.

S—————————————S

Ala-Cy-Asn-Thr-Ala-Thr-Cy-Val-Thr-His-Arg-Leu-Ala-Gly-Leu-Leu-Ser-Arg-Ser-Gly-Gly-Met-Val-Lys-Ser-Asn-Phe-Val-Pro- (III)

Thr-Asn-Val-Gly-Ser-Lys-Ala-Phe-$NH_2$

4. Procédé selon la revendication 2, caractérisé en ce qu'on choisit les produits de départ de manière à préparer le peptide de formule XV ou un de ses sels.

S—————————————S

Ala-Cy-Asn-Thr-Ala-Thr-Cy-Val-Thr-His-Arg-Leu-Ala-Gly-Leu-Leu-Ser-Arg-Ser-Gly-Gly-Met-Val-Lys-Ser-Asn-Phe-Val-Pro- (XV)

Thr-Asn-Val-Gly-Ser-Lys-Ala-Phe-Tyr-OH

5. Procédé selon la revendication 2, caractérisé en ce qu'on choisit les produits de départ de manière à préparer le peptide de formule XIX ou un de ses sels.

S—————————————S

Ala-Cy-Asn-Thr-Ala-Thr-Cy-Val-Thr-His-Arg-Leu-Ala-Gly-Leu-Leu-Ser-Arg-Ser-Gly-Gly-Met-Val-Lys-Ser-Asn-Phe-Val-Pro- (XIX)

Thr-Asn-Val-Gly-Ser-Lys-Ala-Phe-Gly-OH

6. Procédé selon l'une des revendications 1-5, caractérisé en ce qu'on choisit les produits de départ de manière à préparer un sel pharmaceutiquement acceptable.

7. Procédé selon l'une des revendications 1-6, caractérisé en ce que la séquence d'ADN mentionnée dans la variante d) du procédé contient une séquence de formule IV,

d(GCETGYAAYACXGCEACXTGYGTXACXCAYNGKYTZGCEGGXYTZYTZQRSNGK
AlaCysAsnThrAlaThrCysValThrHisArgLeuAlaGlyLeuLeuSerArg

(IV)

QRSGGXGGXATGGTXAAMQRSAAYTTLGTXCCXACXAAYGTXGGXQRSAAMGCETTL)
SerGlyGlyMetValLysSerAsnPheValProThrAsnValGlySerLysAlaPhe

dans laquelle seule la séquence désoxynucléotide du brin codant, commençant par l'extrémité 5' et à l'intérieur de celle-ci, les acides aminés codés par les triplets correspondants sont indiqués et où
A = adénosyle,
T = thymidyle,
G = guanosyle,
C = cytidyle,
E = A, T, C ou G,
K = A, T, C ou G, lorsque N = C, ou K = A ou G, lorsque N = A,
L = T ou C,
M = A ou G,
N = A ou C,
Q = T ou A,
R,S: R = C et S = A, T, C ou G, lorsque Q = T ; ou R = G et S = T ou C, lorsque Q = A,
X = A, T, C ou G,
Y = T ou C et
Z = A, T, C ou G, lorsque Y = C, ou Z = A, ou G, lorsque Y = T,
où le "d" situé en avant des parenthèses signifie que les nucléosides mentionnés dans la parenthèse sont des 2-désoxyribo-nucléosides.

**8.** Procédé selon la revendication 7, caractérisé en ce que la séquence d'ADN mentionnée dans la variante d) du procédé contient la séquence d'ADN de formule IVb, où seul le brin codant commençant par l'extrémité 5' est indiqué et, aux fins de meilleure compréhension, sont également mentionnés les acides aminés pour lesquels code chaque triplet.

AlaCysAsnThrAlaThrCysValThrHisArgLeuAlaGlyLeuLeuSerArg
d(GCTTGCAACACCGCTACCTGCGTTACCCACCGTCTGGCTGGTCTGCTGTCTCGT

(IVb)

SerGlyGlyMetValLysSerAsnPheValProThrAsnValGlySerLysAlaPhe
TCTGGTGGTATGGTTAAATCTAACTTCGTTCCGACCAACGTTGGTTCTAAAGCTTTC)